(19) Europäisches Patentamt
European Patent Office
Office européen des brevets

(11) EP 3 050 970 B1

(12) EUROPEAN PATENT SPECIFICATION

(45) Date of publication and mention of the grant of the patent:
**18.09.2019 Bulletin 2019/38**

(51) Int Cl.:
***C12P 7/16*** *(2006.01)* *C07K 14/00* *(2006.01)*
*C12N 5/00* *(2006.01)*

(21) Application number: **15305096.8**

(22) Date of filing: **28.01.2015**

# (54) Modified microorganism for optimized production of 1,4-butanediol

Modifizierter Mikroorganismus zur optimierten Herstellung von 1,4-Butandiol

Micro-organisme modifié pour optimiser la production de 1,4-butanediol

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.08.2016 Bulletin 2016/31**

(73) Proprietor: **Metabolic Explorer**
**63360 Saint Beauzire (FR)**

(72) Inventors:
- **Vasseur, Perrine**
  **63270 Martres sur Morges (FR)**
- **Dischert, Wanda**
  **63270 Vic-le-Comte (FR)**
- **Soucaille, Philippe**
  **31450 Deyme (FR)**

(74) Representative: **Regimbeau**
**87 rue de Sèze**
**69477 Lyon Cedex 06 (FR)**

(56) References cited:
- **XIAO-JUN JI ET AL: "Elimination of carbon catabolite repression infor efficient 2,3-butanediol production from glucose-xylose mixtures", APPLIED MICROBIOLOGY AND BIOTECHNOLOGY, SPRINGER, BERLIN, DE, vol. 89, no. 4, 19 October 2010 (2010-10-19), pages 1119-1125, XP019880533, ISSN: 1432-0614, DOI: 10.1007/S00253-010-2940-5**
- **HARRY YIM ET AL: "Metabolic engineering of Escherichia coli for direct production of 1,4-butanediol", NATURE CHEMICAL BIOLOGY, NATURE PUB. GROUP , vol. 7, no. 7 1 July 2011 (2011-07-01), pages 445-452, XP002706396, ISSN: 1552-4450, DOI: 10.1038/NCHEMBIO.580 Retrieved from the Internet: URL:http://www.nature.com/nchembio/journal/v7/n7/full/nchembio.580.html [retrieved on 2011-05-22]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## INTRODUCTION

A genetically modified microorganism for the optimized production of 1,4-butanediol (1,4-BDO), where the microorganism metabolically transforms xylose into 1,4-BDO is described herein. A method for the optimized production of 1,4-butanediol by culturing said genetically modified microorganism in a fermentation medium and recovering the 1,4-BDO from said medium is further described herein.

1,4-Butanediol, also known as 1,4-BDO or BDO, is the organic compound with the formula $HOCH_2CH_2CH_2CH_2OH$. This colourless viscous liquid is derived from butane by adding alcohol groups at each end of the chain. 1,4-butanediol is one of four stable isomers of butanediol, and is used industrially as a solvent and in the manufacture of some types of plastics, elastic fibers and polyurethanes. In organic chemistry, 1,4-butanediol is more particularly used for the synthesis of γ-butyrolactone (GBL). Worldwide production of 1,4-butanediol is reported to be of about one million metric tons per year, and was, as a matter of fact, billions of lbs in 2013.

Conventional processes for the synthesis of BDO use petrochemical feedstocks for their starting materials. For example, acetylene is reacted with 2 molecules of formaldehyde in the Reppe synthesis reaction (Sampat, 2011), followed by catalytic hydrogenation to form 1,4-butanediol. It has been estimated that 90% of the acetylene produced in the U.S.A. is consumed for butanediol production. Alternatively, it can be formed by esterification and catalytic hydrogenation of maleic anhydride, which is derived from butane. It is therefore desirable to develop a method for the production of these chemicals by alternative means that not only substitute renewable for petroleum-based feedstocks, but also use less energy and that are capital-intensive processes.

Methods for the preparation of 1,4-butanediol by fermentation exist and have been described among others by the company Genomatica: those methods are based on the culture of non-naturally occurring microbial organisms containing a 1,4-butanediol (BDO) pathway expressed in a sufficient amount to produce BDO from raw material (WO2012177943A1).

Alternative methods of production of 1,4-BDO where the microorganism metabolically transforms xylose into 1,4-BDO have also been described in the art. Methods have notably been developed in which a microorganism comprises a specific metabolic pathway for the conversion of xylose into 1,4-BDO, for example through 1,2,4-butanetriol as an intermediate metabolite (See US2013/0203141 and WO2008/091288). Those methods, based on the conversion of xylose into 1,4-BDO, aim to improve this particular metabolic pathway to favour the production of 1,2,4-butanetriol and then 1,4-butanediol by overexpressing the genes encoding the enzymes involved in this pathway.

US2013/0203141 notably describes recombinant microorganisms having a metabolic pathway for the conversion of xylose, as the sole carbon and energy source, into 1,4-BDO (in other words capable of exclusive conversion of xylose into 1,4 BDO). However, the 1,4-BDO yields obtained by this method are very low and decrease over time, and are therefore not compatible with large scale fermentation production of 1,4-BDO.

The inventors have surprisingly discovered that the overall production of 1,4-BDO can be greatly improved by further genetically modifying a microorganism capable of exclusively converting xylose for 1,4-BDO. In particular, the inventors have discovered that 1,4-BDO production can be optimized thanks to additional genetic modifications which provide the microorganism with reducing power (NAD(P)H) and energy (ATP) for 1,4-BDO production and growth from a source of carbon other than xylose, and which inhibit catabolite repression. Thus, xylose can be used by the microorganism for the sole production of 1,4-BDO, and the other source of carbon can be used for solely providing the energy and cofactors necessary for the growth of the microorganism and for the production of 1,4-BDO, while in the meantime avoiding any repression of the conversion of xylose into 1,4-BDO.

In this regard, it must be noted, that even though WO2008/091288 discloses the use of a mix of carbon source for the production of 1,2,3-butanetriol, this mix was not co-utilized by the microorganism in the same way as proposed herein: indeed, the carbon sources other than D-xylose used in WO2008/091288 are preliminary converted into D-xylose prior to their use in the fermentation process. Thus, by contrast to the microorganism and method described herein, the microorganism disclosed by WO2008/091288 solely uses D-xylose (originating from xylose and from the other carbon source) for both the production of 1,2,3-butanetriol and the growth of the microorganism.

A microorganism genetically modified for an optimized production of 1,4-butanediol is provided herein for the first time. This microorganism, which is genetically modified in order to exclusively convert xylose into 1,4-butanediol, comprises further genetic modifications in order to:

- provide the reducing power and/or energy necessary for 1,4-butanediol production and growth of the microorganism, from a source of carbon other than xylose, and
- inhibit catabolite repression.

A method for an optimized production of 1,4-butanediol by fermentation comprising culturing the microorganism of the invention in a culture medium comprising xylose and a source of carbon other than xylose, and recovering the produced 1,4-BDO from the culture medium is also described herein.

## SUMMARY OF THE INVENTION

The present invention is defined in claims 1 to 10.

## DETAILED DESCRIPTION OF THE INVENTION

It shall be understood that the following detailed description is not limitative and that various modifications, substitutions, omissions, and changes may be made without departing from the scope of the invention. It shall also be understood that the terminology used herein is for the purpose of describing particular embodiments of the invention, and is not intended to be limiting.

Furthermore, unless otherwise stated, all technical and scientific terms used herein have the same meanings as commonly understood by one of ordinary skill in the art. Conventional microbiological and molecular biological techniques are also those well-known and commonly used in the art. Such techniques are fully explained in the literature.

Nevertheless, with respect to the use of different terms throughout the current specification, the following definitions more particularly apply.

The singular forms "a," "an," and "the" include herein plural reference unless the context clearly dictates otherwise. Thus, for example, a reference to "a microorganism" includes a plurality of such microorganisms, and a reference to "an endogenous gene" is a reference to one or more endogenous genes, and so forth.

The terms "comprise," "contain," "involve," or "include" or variations such as "comprises," "comprising," "containing," "involved," "includes," "including" are used herein in an inclusive sense, i.e. to specify the presence of the stated features but not to preclude the presence or addition of further features in various embodiments of the invention.

The term "microorganism," as used herein, refers to a living microscopic organism, which may be a single cell or a multicellular organism and which can generally be found in nature. In the present context, the microorganism is preferably a bacterium, yeast or fungus. More preferably, the microorganism is selected among *Enterobacteriaceae, Bacillaceae, Clostridiaceae, Streptomycetaceae* and yeast. Even more preferably, the microorganism is a species of *Escherichia, Klebsiella, Thermoanaerobacterium, Clostridium or Saccharomyces*. Yet even more preferably, the microorganism is selected from *Escherichia coli, Klebsiella pneumoniae, Thermoanaerobacterium thermosaccharolyticum, Clostridium acetobutylicum* and *Saccharomyces cerevisiae.* Most preferably, the microorganism of the invention is *Escherichia coli.*

The term "recombinant microorganism" or "genetically modified microorganism," as used herein, refers to a microorganism as defined above that is not found in nature and therefore genetically differs from its natural counterpart. In other words, it refers to a microorganism that is modified by introduction and/or by deletion and/or by modification of its genetic elements. Such modification can be performed for example by genetic engineering, or by forcing the development and evolution of new metabolic pathways by combining directed mutagenesis and evolution under specific selection pressure (see, for example, WO2005/073364 or WO2008/116852).

A microorganism genetically modified for the production of 1,4-BDO as described herein therefore means that said microorganism is a recombinant microorganism as defined above that is capable of producing 1,4-BDO. In other words, said microorganism has been genetically modified to allow production of 1,4-BDO.

The term "source of carbon," "carbon source," or "carbon substrate" as used herein refers to any carbon source capable of being metabolized by a microorganism and which contains at least one carbon atom. A carbon source may be derived from renewable feed-stock. Renewable feed-stock is defined as raw material required for certain industrial processes that can be regenerated within a brief delay and in sufficient amount to permit its transformation into the desired product. Vegetal biomass, treated or not, is an interesting renewable carbon source. In the present context, xylose is a carbon source, and a source of carbon other than xylose is advantageously a carbohydrate.

The term "carbohydrate" refers herein to any carbon source capable of being metabolized by a microorganism and containing at least one carbon atom, two atoms of hydrogen and one atom of oxygen. The carbohydrate is preferably selected from the group consisting of monosaccharides such as glucose, fructose, mannose, galactose and the like, disaccharides such as sucrose, cellobiose, maltose, lactose and the like, oligosaccharides such as raffinose, stacchyose, maltodextrins and the like, polysaccharides such as cellulose, hemicellulose, starch and the like, methanol, formaldehyde and glycerol. According to a preferred embodiment of the invention, the source of carbon other than xylose is advantageously a carbohydrate comprising 3, 6 or 12 carbon atoms, or any combination thereof. In a more preferred embodiment of the invention, the source of carbon other than xylose is selected from glycerol, glucose, galactose, fructose, lactose, maltose, sucrose, and any combination thereof. More preferably, the carbon source other than xylose is selected from glycerol, glucose, sucrose, and any combination thereof, and most preferably is sucrose.

In the present context, a microorganism can be genetically modified by modulating the expression level of one or more endogenous genes. By "modulating," it is meant herein that the expression level of said gene is up-regulated, downregulated, or even completely abolished in comparison to its natural expression level. Such modulation can therefore result in an enhancement of the activity of the gene product, or alternatively, in a lower or null activity of the endogenous gene product.

The term "endogenous gene" refers herein to a gene that is naturally present in the microorganism.

An endogenous gene can be overexpressed by introducing heterologous sequences which favour upregulation in addition to endogenous regulatory elements or by substituting those endogenous regulatory elements with such heterologous sequences, or by introducing one or more supplementary copies of the endogenous gene into the chromosome or a plasmid within the microorganism. Endogenous gene activity and/or expression level can also be modified by introducing mutations into their coding sequence to modify the gene product. A deletion of an endogenous gene can also be performed to totally inhibit its expression within the microorganism. Another way to modulate the expression of an endogenous gene is to exchange its promoter (i.e. wild type promoter) with a stronger or weaker promoter to up or down regulate the expression level of this gene. Promoters suitable for such purpose can be homologous or heterologous and are well-known in the art. It is within the skill of the person in the art to select appropriate promoters for modulating the expression of an endogenous gene.

In addition, or alternatively, a microorganism can be genetically modified to express one or more exogenous genes, provided that said genes are introduced into the microorganism with all the regulatory elements necessary for their expression in the host microorganism. The modification or "transformation" of microorganisms with exogenous DNA is a routine task for those skilled in the art. In the present context, the term "overexpression" or "overexpressing" is also used herein in relation to the expression of exogenous genes in the microorganism.

By "exogenous gene," it is meant herein that said gene is not naturally occurring in the microorganism. In order to express an exogenous gene in a microorganism, such a gene can be directly integrated into the microorganism chromosome, or be expressed extra-chromosomally by plasmids or vectors within the microorganism. A variety of plasmids, which differ in respect to their origin of replication and their copy number in a cell, are well-known in the art and can be easily selected by the skilled practitioner for such a purpose. Exogenous genes are advantageously homologous genes.

In the present context, the term "homologous gene" or "homolog" not only refers to a gene inherited by two species (i.e. microorganism species) by a theoretical common genetic ancestor, but also includes genes which may be genetically unrelated that have, nonetheless, evolved to encode proteins which perform similar functions and/or have similar structure (i.e. functional homolog). Therefore, the term "functional homolog" refers herein to a gene that encodes a functionally homologous protein.

Using the information available in databases such as Uniprot (for proteins), Genbank (for genes), or NCBI (for proteins or genes), those skilled in the art can easily determine the sequence of a specific protein and/or gene of a microorganism, and identify, based on this sequence, the one of equivalent genes, or homologs, in another microorganism. This routine work can be performed by a sequence alignment of a specific gene sequence of a microorganism with gene sequences or the genome of other microorganisms, which can be found in the above-mentioned databases. Such sequence alignment can advantageously be performed using the BLAST algorithm developed by Altschul and colleagues (Altschul et al., 1990). Once a sequence homology has been established between those sequences, a consensus sequence can be derived and used to design degenerate probes in order to clone the corresponding homolog gene of the related microorganism. These routine methods of molecular biology are well-known to those skilled in the art.

The microorganism can also be genetically modified to increase or decrease the activity of one or more proteins.

Increasing such activity can be obtained by improving the protein catalytic efficiency, by decreasing protein turnover, by decreasing messenger RNA (mRNA) turnover, by increasing transcription of the gene, or by increasing translation of the mRNA.

Improving the protein catalytic efficiency means increasing the kcat and/or decreasing the Km for a given substrate and/or a given cofactor, and/or increasing the Ki for a given inhibitor. kcat, Km and Ki are Michaelis-Menten constants that the man skilled in the art is able to determine (Segel, 1993). Decreasing protein turnover means stabilizing the protein. Methods to improve protein catalytic efficiency and/or decrease protein turnover are well-known by the man skilled in the art. These include rational engineering with sequence and/or structural analysis and directed mutagenesis, as well as random mutagenesis and screening. Mutations can be introduced by site-directed mutagenesis by conventional methods such as Polymerase Chain Reaction (PCR), by random mutagenesis techniques, for example via mutagenic agents (Ultra-Violet rays or chemical agents like nitrosoguanidine (NTG) or ethylmethanesulfonate (EMS)) or DNA shuffling or error-prone PCR. Stabilizing the protein can also be achieved by adding a "tag" peptide sequence either at the N-terminus or the C-terminus of the protein. Such tags are well-known in the art, and include, among others, the Glutathione-S-Transferase (GST).

Decreasing mRNA turnover can be achieved by modifying the gene sequence of the 5'-untranslated region (5'-UTR) and/or the coding region, and/or the 3'-UTR (Carrier and Keasling, 1999).

Increasing the transcription of a gene, whether endogenous or exogenous, can be achieved by increasing the number of its copies within the microorganism and/or by using a promoter leading to a higher level of expression of the gene. In the present context, the term "overexpression" or "overexpressing" is also used to designate an increase in transcription of a gene in a microorganism.

As indicated above, to increase the number of copies of a gene in the microorganism, said gene can be encoded chromosomally or extra-chromosomally. When the gene of interest is to be encoded on the chromosome, several copies of the gene can be introduced on the chromosome by methods of genetic recombination, which are well-known to in the art (e.g. gene replacement). When the gene is to be encoded extra-chromosomally in the microorganism, it can be carried by different types of plasmid that differ in respect to their origin of replication depending on the microorganism in which they can replicate, and by their copy number in the cell. The microorganism transformed by said plasmid can contain 1 to 5 copies of the plasmid, or about 20 copies of it, or even up to 500 copies of it, depending on the nature of the plasmid. Examples of low copy number plasmids which can replicate in *E. coli* include, without limitation, the pSC101 plasmid (tight replication), the RK2 plasmid (tight replication), as well as the pACYC and pRSF1010 plasmids, while an example of high copy number plasmid which can replicate in *E. coli* is pSK bluescript II.

Promoters which can increase the expression level of a gene are also well-known to the skilled person in the art, and can be homologous (originating from same species) or heterologous (originating from a different species). Examples of such promoters include, without limitation, the promoters Ptrc, Ptac, Plac, and the lambda promoter cl. These promoters can also be induced ("inducible promoters") by a particular compound or by specific external condition like temperature or light.

Increasing translation of the mRNA can be achieved by modifying the Ribosome Binding Site (RBS). A RBS is a sequence on mRNA that is bound by the ribosome when initiating protein translation. It can be either the 5' cap of a mRNA in eukaryotes, a region 6-7 nucleotides upstream of the start codon AUG in prokaryotes (called the Shine-Dalgarno sequence), or an internal ribosome entry site (IRES) in viruses. By modifying this sequence, it is possible to change the protein translation initiation rate, to proportionally alter its production rate, and control its activity inside the cell. It is also possible to optimize the strength of a RBS sequence to achieve a targeted translation initiation rate by using the software RBS CALCULATOR (Salis, 2011). It is within the skill of the person in the art to select the RBS sequence based on the nature of the mRNA.

Decreasing the activity of a protein can mean either decreasing its specific catalytic activity by mutating the gene encoding said protein so as to change the corresponding amino acid sequence and/or decreasing concentrations of the protein in the cell by mutating the nucleotide sequence or by deleting the coding region of said gene.

In a first aspect, a microorganism genetically modified for the production of 1,4-butanediol by exclusively converting xylose into 1,4-butanediol is described herein, wherein said microorganism is further genetically modified for:

i) providing reducing power and/or energy for 1,4-butanediol production and microorganism growth, from a carbon source other than xylose, and
ii) at least partially, preferably totally, inhibiting carbon catabolite repression,

thereby optimizing the production of 1,4-butanediol by said microorganism.

Microorganisms genetically modified for the conversion of xylose into 1,4-BDO, in particular for its exclusive conversion into 1,4-BDO, are well-known in the art, and have notably been described by US2013/0203141 (see also WO2008/091288 for the production of the intermediate metabolite 1,2,3-butanetriol).

Accordingly, in a preferred embodiment, the metabolic pathway for the exclusive conversion of xylose into 1,4-BDO comprises at least the steps:

- elimination of one carbon from the xylose backbone, thereby obtaining an intermediate metabolite having an aliphatic backbone of 4 carbons, and
- elimination of one hydroxyl group from said intermediate metabolite.

The metabolic pathway for said conversion is preferably the pathway as represented in Figure 1.

More preferably, the genetic modification for exclusively converting xylose into 1,4-BDO is an overexpression of at least one of the following genes:

- a gene encoding a xylose dehydrogenase,
- a gene encoding a xylolactone hydrolase,
- a gene encoding a 3-deoxy-D-glycero-pentulosonate decarboxylase,
- a gene encoding a 1,2,4-butanetriol dehydratase,
- a gene encoding a D-xylonate dehydratase,
- a gene encoding a NADPH dependant 1, 2, 4-butanetriol dehydrogenase
- a gene encoding a NADPH dependant 1,4-butanediol dehydrogenase, and

- any combination thereof.

When endogenous genes coding for the above activities are present in the microorganism, they are advantageously overexpressed. When the microorganism does not comprise genes coding for one of these enzymes, it is advantageously transformed with one or more exogenous genes coding for said enzyme(s).

In an example, notably when the genetically modified microorganism is *E. coli*:

α) genes encoding xylose dehydrogenase, xylolactone hydrolase, 3-deoxy-D-glycero-pentulosonate decarboxylase, and 1,2,4-butanetriol dehydratase are heterologous genes introduced into the microorganism, and

β) genes encoding D-xylonate dehydratase, NADPH-dependant 1,2,4-butanetriol dehydrogenase and NADPH-dependant 1,4-butanediol dehydrogenase are endogenous and are therefore advantageously overexpressed.

Genes coding for the above described enzymes are well-known in the art:

- genes encoding a xylose dehydrogenase are disclosed in US2013/0203141, and include, without limitation, the *Xdh* encoding genes from *Caulobacter crescentus, Haloarcula marismortui, Burkholderia fugorum* LB400 and *Haloferax volcanii* DS2,
- genes encoding a xylolactone hydrolase include, without limitation, the *xylC* gene from *Caulobacter crescentus,*
- genes encoding a 3-deoxy-D-glycero-pentulosonate decarboxylase are disclosed in US2013/0203141 and WO2008/091288, and include, without limitation, the *mdlC* gene from *Pseudomonas putida,*
- genes encoding a 1,2,4-butanetriol dehydratase are disclosed in US2013/0203141, and include, without limitation, the *gldABC* and *dhaB123* genes from *Klebsiella pneumoniae,* the *pddABC* gene from *Klebsiella oxytoca,* and the *dhaBCE* gene from *Clostridium pasteurianum,*
- genes encoding a D-xylonate dehydratase are disclosed in US2013/0203141 and WO 2008/091288, and include, without limitation, the *yjhG* and *yagF* genes from *E. coli,* and
- genes encoding both a NADPH-dependant 1,2,4-butanetriol dehydrogenase and a NADPH-dependant 1,4-butanediol dehydrogenase are disclosed in US2013/0203141 and WO2011137192, and include, without limitation, the *yqhD* and *adhP* genes from *E. coli.*

The nucleotide of the above-mentioned genes, or the amino acid sequence encoded by said genes, are described in Table 1 below, according to their accession number and version in a database and/or according to their sequence identification.

The microorganism as described above is further genetically modified for providing reducing power and/or energy necessary for 1,4-butanediol production and microorganism growth, from a carbon source other than xylose. This feature is particularly advantageous as it avoids using xylose for both providing this reducing power and/or energy, and therefore the xylose is exclusively used for producing 1,4-BDO.

In order to do so, the microorganism described herein is genetically modified by deleting and/or attenuating genes encoding enzymes using xylose or other metabolites of the selected pathway converting xylose as substrate into 1,4-BDO.

Thus, the genetic modification i) is an attenuation and/or deletion of at least one of the following genes:

- a gene encoding a xylose isomerase (e.g. *xylA* gene from *E. coli*),
- a gene encoding a xylulose kinase (e.g. *xylB* gene from *E. coli*),
- a gene encoding a 3-deoxy-D-glycero-pentulosonate aldolases (e.g. *yjhH* and/or *yagE* genes from *E. coli*),
- a gene encoding a keto-acid dehydrogenase (e.g. *yiaE* and/or *ycdW* genes from *E. coli*), and
- any combination thereof.

These genes and methods to delete or attenuate them are disclosed in US2013/0203141.

The nucleotide of the above-mentioned genes, or the amino acid sequence encoded by said genes, are described in Table 1 below, according to their accession number and version in a database and/or according to their sequence identification.

As indicated above, the microorganism described herein is also genetically modified so that it can use a carbon source other than xylose for providing the reducing power and/or energy which are necessary for 1,4-butanediol production and for the growth of the microorganism.

This feature is critical in the context of the present invention. Indeed, in microorganisms, some carbon sources are preferred to others. Most naturally occurring microorganisms, including *Escherichia coli,* prefer glucose to other sugars even if they are able to metabolize an array of monosaccharides (Kim et al., 2010), and are unable for instance to co-utilize glucose in addition to xylose in an effective manner. Accordingly, a diauxic fermentation pattern occurs generally in microorganisms when two sugars are present in the culture medium: this regulatory mechanism is known as catabolite repression. The same phenomenon has been observed by Monod in the 1940s with glucose and lactose.

It is notably recognized in *E. coli* that the glucose enters with the aid of glucose specific permease EIICBglc (encoded by *ptsG*) in the phosphoenolpyruvate (PEP): carbohydrate phosphotransferase system (PTS). During glucose transport, the level of cyclic AMP (cAMP) is lowered by dephosphorylated EIIAglc (encoded by *crr*), which in turn limits the availability of the catabolite repressor protein (CRP) and cAMP complex (cAMP-CRP). Then, all the genes involved in the catabolism of sugars other than glucose which are generally regulated by cAMP-CRP are repressed.

However, even though this catabolite repression mechanism has been extensively studied in *Enterobacteriaceae,* catabolite repression by carbohydrates that are not transported by PTS system exist and are not well understood (Hogema et al., 1997).

Accordingly, the genetic modification ii) of the microorganism described herein is selected from at least one of the following:

- deletion of a gene encoding a glucose phophotransferase,
- deletion of a gene encoding a phosphocarrier Hpr protein,
- expression, preferably from a constitutive or inducible promoter not regulated by cAMP-CRP, of a gene and/or operon involved in a sugar importer system wherein said sugar is a carbon source other than xylose,
- expression of a gene encoding an ABC xylose transporter, preferably from a constitutive or inducible promoter not regulated by cAMP-CRP,
- overexpression of a gene encoding a glucose permease,
- overexpression of a gene encoding a glucose facilitator,
- overexpression of a gene encoding a glucokinase,
- modulation of the expression of a gene involved in cAMP levels, preferably of a gene encoding adenylate cyclase,
- modulation of the expression a gene encoding a CRP and/or a CRP-like protein,
- expression of a gene encoding a cAMP-independent CRP protein, preferably from a constitutive or inducible promoter not regulated by cAMP-CRP, and
- any combination thereof.

More preferably, for the co-utilisation of xylose and glucose, a deletion of a gene encoding a phophotransferase and/or a phosphocarrier Hpr protein is advantageously combined with an overexpression of a gene encoding a glucose permease or a glucose facilitator, along with an overexpression of a gene encoding a glucokinase.

Genes coding for the above described proteins are well-known in the art:

- genes encoding a glucose phophotransferase include, without limitation, the pstG gene from *E. coli,*
- genes encoding a phosphocarrier Hpr protein, which is one of two sugar-non-specific protein constituents of the phosphoenolpyruvate:sugar phosphotransferase system (PTSsugar), include, without limitation, the *ptsH* gene from *E. coli,*
- genes/operon involved in a sugar importer system (PTS or other system) include, without limitation, the *lacY* gene from *E.coli* for the assimilation of lactose, the *malFG* genes from *E. coli* for the assimilation of maltose, and the scrKYABR or cscBKAR operons respectively from *Salmonella typhimurium* and *E. coli* for the assimilation of sucrose,
- genes encoding an ABC xylose transporter include, without limitation, the *xylFGH* operon from *E. coli,*
- genes encoding a glucose permease include, without limitation, the *galP* gene from *E. coli,*
- genes encoding a glucose facilitator include, without limitation, the *glf* gene from *Zymomonas mobilis,*
- genes encoding a glucokinase include, without limitation, the *glk* gene from *E. coli,*
- genes encoding an adenylate cyclase, include, without limitation the *cyaA* gene from *E. coli,*
- genes encoding a CRP and/or a CRP-like protein include, without limitation, the CRP genes from *E.coli* and the *ccpA* gene from *Bacillus subtilis* and other firmicutes,
- genes encoding a cAMP-independent CRP protein have been described by Hogema et al. (1997), and include, without limitation, genes encoding CRP proteins mutated in two regions: from residues 53 to 62 corresponding of the cAMP-binding domain of the CRP protein and from residues 141 to 148 corresponding to a proximal boundary of the DNA-binding domain (Helix-Turn-Helix). Examples of such protein mutants include CRP mutants Asp to His residue 53, Ser to Phe residue 62, Gly to Asp residue 141, Arg to Asp residue 142, Leu to Arg residue 148, and any combination thereof.

More preferably, for the co-utilisation of xylose and glucose in *E.coli,* the *ptsG* or *ptsH* gene deletion can be combined with an overexpression of the *galP* or *glf* gene along with an overexpression of the *glk gene.*

The nucleotide sequence of the above mentioned genes, or the amino acid sequence encoded by said genes, are described in Table 1 below, according to their accession number and version in a database and/or according to their sequence identification.

In a further example, the microorganism described herein comprises further genetic modification(s) to use sucrose as source of carbon other than xylose, as described in WO2012/004247.

To do so, the microorganism preferably comprises functional genes coding for a PTS sucrose utilization system and/or for a non-PTS sucrose utilization system.

A PTS sucrose utilization system is a system for sucrose utilization based on the transport of sucrose by a phosphoenolpyruvate (PEP)-dependent sucrose phosphotransferase system (Sucrose-PTS). A phosphotransferase system couples the transport of a sugar (e.g. sucrose or glucose) with the phosphorylation of the sugar using PEP as phosphate donor. After transport into the cell, the sucrose-phosphate is cleaved into glucose-6-phosphate and fructose by an invertase.

Fructose is then phosphorylated into fructose-6-phosphate by a fructokinase. The genes coding for this PTS sucrose utilization system can be controlled by a regulatory protein.

A non-PTS sucrose utilization system is a system for sucrose utilization based on transport of sucrose by a system independent of phosphoenolpyruvate. After transport into the cell, the sucrose is cleaved into glucose and fructose by an invertase. Fructose is then phosphorylated into fructose-6-phosphate by a fructokinase and glucose is phosphorylated into glucose-6-phosphate by a glucokinase. The genes coding for this non-PTS sucrose utilization system can be controlled by a regulatory protein.

In a particular example, the microorganism expresses naturally or has been genetically modified to express the genes of the operon scrKYABR from *Salmonella,* i.e. the *scr*K gene encoding a fructokinase, the *scr*Y gene encoding a porin, the *scr*A gene encoding the IIBC protein, the *scrB* gene encoding a sucrose-6-P invertase, and the *scr*R gene encoding a repressor, and any combination thereof. A conjugative plasmid pUR400 bearing scrKYABR might be used to transform the microorganism. These genes can be expressed in the microorganism alone, in any combination comprising at least two of these genes, or all together in a combination. The gene scrR can preferably be omitted.

In particular example, the microorganism expresses naturally or has been genetically modified to express the genes from the *E. coli* strain EC3132, i.e. the operon *csc*BKAR encoding a sucrose:proton symport transport system (*csc*B gene), a fructokinase (*csc*K gene), an invertase (cscA gene) and a sucrose-specific repressor (*csc*R gene). These genes can be expressed in the microorganism alone, in any combination comprising at least two of these genes, or all together in a combination. The gene cscR can preferably be omitted. Homologous genes from other organisms can also be used.

More specifically, the present invention is directed to a genetically modified *Escherichia coli* for the production of 1,4-butanediol by exclusively converting xylose into 1,4-butanediol, wherein said microorganism is further genetically modified for:

i) providing reducing power and/or energy for 1,4-butanediol production and microorganism growth from a carbon source other than xylose, wherein said genetic modification is the deletion of the following endogenous genes:

- the *xylA* gene encoding a xylose isomerase,
- the *xylB* gene encoding a xylulose kinase,
- the *yjhH* and *yagE* gene encoding a 3-deoxy-D-glycero-pentulosonate aldolase,
- the *yiaE* and *ycdW* gene encoding a keto-acid dehydrogenase, and

ii) at least partially inhibiting carbon catabolite repression, thereby optimizing the production of 1,4-butanediol by said microorganism wherein said genetic modification is selected from at least one of the following:

- deletion of the endogenous *ptsG* gene encoding a glucose phophotransferase,
- deletion of the endogenous *ptsH* gene encoding a phosphocarrier Hpr protein,
- expression of the endogenous *lacY* gene and/or the endogenous *malFG* and/or *cscBKAR* operon or the heterologous *scrKYABR* operon from *Salmonella typhimurium,* involved in a sugar importer system wherein said sugar is a carbon source other than xylose,
- expression of the endogenous *xylFGH* operon encoding an ABC xylose transporter,
- overexpression of the endogenous galP gene encoding a glucose permease,
- overexpression of the heterologous glf gene encoding a glucose facilitator from *Zymomonas mobilis,*
- overexpression of the endogenous *glk* gene encoding a glucokinase,
- expression of a gene encoding a cAMP-independent CRP protein, wherein said CRP protein is coded by SEQ ID NO: 28, 29, 30, 31, or 32, and
- any combination thereof.

According to a preferred embodiment, the microorganism of the invention comprises as a further genetic modification an overexpression of at least one gene involved in vitamin B12 transport.

Such modification can notably increase the intracellular vitamin B12 concentration, and has been described in US2011/207189: it includes among others the overexpression of the *btuBCDF* genes in order to increase the binding of vitamin B12 to membranes and its rate of uptake into cells, as well as the removal of regulatory controls.

In the present context, the genes that can be overexpressed for improving vitamin B12 transport are preferably selected from:

- a gene encoding a vitamin B12 outer membrane porin (e.g. *btuB* gene from *E.coli*),
- a gene encoding a membrane subunit of the vitamin B12 ABC transporter (e.g. *btuC* gene from *E.coli*),
- a gene encoding an ATG- or GTP-binding protein of the vitamin B12 transporter (e.g. *btuD* gene from *E.coli*), and
- any combination thereof.

Such genes are known in the art and are notably disclosed in US2011/207189.

The nucleotide sequence of the above-mentioned genes, or the amino acid sequence encoded by said genes, are described in Table 1 below, according to their accession number and version in a database and/or according to their sequence identification.

According to a further preferred embodiment, the microorganism of the invention comprises as a further genetic modification an overexpression of at least one gene involved in coenzyme B12 utilisation and/or 1,2,4-butanetriol dehydratase reactivation.

Coenzyme B12 is indeed a cofactor for 1,2,4-butanetriol dehydratase, which is inactivated by oxidation of coenzyme B12 and needs reactivation for restoring its activity.

Genes involved in coenzyme B12 utilisation and/or 1,2,4-butanetriol dehydratase reactivation are described in US2008/176302, and include without limitation genes encoding a cobalamine adenosyl transferase (e.g. the *btuR* gene from *E. coli),* genes encoding a 1,2,4-butanetriol dehydratase reactivase (e.g. the *dhaB4* and *orf2b* genes from *Klebsiella pneumoniae;* the *ddrA* and *ddrB* genes from *Klebisella oxytoca;* the *orfZ* and *orfX* genes from *Clostridium pasteurianum*), and any combination thereof. Moreover, it may be advantageous to overexpress a gene which improves the reactivation system, such as the genes *orf2a* from *Klebsiella pneumoniae* and/or *orfY* from *Clostridium pasteurianum.*

In a more preferred particular example, particularly when the microorganism comprises an endogenous gene encoding a cobalamine adenosyl transferase and no or low expression of genes encoding 1,2,4-butanetriol dehydratase reactivase, coenzyme B12 production/regeneration and 1,2,4-butanetriol dehydratase reactivation are improved by overexpression of an endogenous gene encoding a cobalamine adenosyl transferase (e.g. the *btuR* gene from *E.coli*) and overexpression of at least one heterologous gene encoding a 1,2,4-butanetriol dehydratase reactivase (e.g. the *dhaB4, orf2b,* and *orf2a* genes from *Klebsiella pneumonia,* and any combination thereof).

The nucleotide sequence of the above-mentioned genes, or the amino acid sequence encoded by said genes, are described in Table 1 below, according to their accession number and version in a database and/or according to their sequence identification.

Yet, in a preferred embodiment, the microorganism of the invention comprises a further genetic modification of at least one gene involved in the production of NADPH as a source of reducing power. Indeed, reducing enzymes such as dehydrogenases are in need of reducing power available in the microorganism, particularly in the form of NADPH. Strategies for increasing NADPH availability in the cell are well-known in the art, and have notably been reviewed by Lee et al. (2013) and also described by US8088620, WO2012/055798 and EP14305691.9.

The genetic modification for improving the production of NADPH, and therefore its availability in the microorganism, is preferably selected from:

- overexpression of a gene or operon encoding a membrane-bound transhydrogenase,
- deletion or attenuation of a gene encoding a soluble transhydrogenase,
- overexpression of a gene encoding a NADPH generating glyceraldehyde 3-phosphate dehydrogenase,
- deletion or attenuation of a gene encoding a phosphoglucose isomerase,
- deletion or attenuation of a gene encoding a phosphofructokinase,
- overexpression of a gene encoding a glucose-6-phosphate dehydrogenase,
- overexpression of a mutant gene encoding a lipoamide dehydrogenase capable of generating NADPH,
- overexpression of a gene encoding a bi-functional NAD(P)H-hydrate repair enzyme, and
- any combination thereof.

The deletion or attenuation of a gene encoding a phosphofructokinase is more preferably combined with an overexpression of a gene encoding a glucose-6-phosphate dehydrogenase, in order to increase the flux of NADPH through the pentose phosphate pathway.

More preferably, the genetic modification for improving the production of NADPH is selected from:

- overexpression of a gene encoding a membrane-bound transhydrogenase,
- deletion or attenuation of a gene encoding a phosphoglucose isomerase and/or a soluble transhydrogenase, and
- overexpression of a gene encoding a NADPH generating glyceraldehyde 3-phosphate dehydrogenase.

Genes coding for the above described proteins are well-known in the art:

- genes or operons encoding a membrane-bound transhydrogenase include, without limitation the *pntAB* operon from *E. coli,* as notably described by WO2012/055798A1,
- genes encoding a soluble transhydrogenase include, without limitation, the *udhA* gene from *E. coli,*
- genes encoding a NADPH generating glyceraldehyde 3-phosphate dehydrogenase include, without limitation, the *gapN* from *Streptococcus mutans* (as described by Centeno-Leija et al. (2013) which can be used for example to substitute the endogenous *gapA* gene from *E. coli,*
- genes encoding a phosphoglucose isomerase include, without limitation the *pgi* gene from *E. coli,*
- genes encoding a phosphofructokinase include, without limitation, the *pfkA* gene from *E. coli* as notably described by WO2005/047498,
- genes encoding a glucose-6-phosphate dehydrogenase include, without limitation, the *zwf* gene from *E. coli* as notably described by Lim et al. (2002),
- mutant genes encoding a lipoamide dehydrogenase capable of generating NADPH include, without limitation, the mutant *lpd* gene (*lpd**) from *E. coli* as notably described by Bocanegra et al. (1993), and
- genes encoding a bi-functional NAD(P)H-hydrate repair enzyme include, without limitation, the *yjeF* gene from *E. coli* as notably described by Marbaix et al. (2011).

In *E. coli,* should the *pfkA* gene be deleted or attenuated, such genetic modification is preferably combined with an overexpression of the *zwf* gene.

More preferably, the genetic modification for improving the production of NADPH is selected from:

- overexpression of a *pntAB* gene from *E. coli,*

- deletion of the *pfkA* gene and/or *udhA* gene from *E. coli,* and

- substitution of a *gapA* from *E. coli* by a *gapN* gene from *Streptococcus mutans.*

The nucleotide sequence of the above-mentioned genes, or the amino acid sequence encoded by said genes, are described in Table 1 below, according to their accession number and version in a database and/or according to their sequence identification.

**Table 1: Genes and proteins described herein** (ND = non disclosed)

| Enzyme full name(s) | Gene name | Origin (Genus species) | SEQ ID NO | Database | Accession number in the database | Version number in the database |
|---|---|---|---|---|---|---|
| Xylose dehydrogenase | *xdh* | *Caulobacter crescentus* | 1 | ND | ND | ND |
| | *xdh* | *Haloarcula marismortui* | ND | Genbank | AAW78223 | AAW78223.1 GI:58429660 |
| | *not available* | *Burkholderia fugorum LB400* | ND | Genbank | GN088955 | GN088955.1 GI: 226882916 |
| | *gfo2* | *Haloferax volcanii DS2* | ND | NCBI | YP_ 003533786 | YP_ 003533786.1 GI: 292653888 |
| Xylonolactonase (Xylolactone hydrolase) | *xylC* | *Caulobacter crescentus* | 2 | ND | ND | ND |

(continued)

| Enzyme full name(s) | Gene name | Origin (Genus species) | SEQ ID NO | Database | Accession number in the database | Version number in the database |
|---|---|---|---|---|---|---|
| 3-deoxy-D-glycero-pentulosonic acid decarboxylase (3-deoxy-D-glycero-pentulosonate decarboxylase; benzoylformate decarboxylase; 2-keto acid decarboxylase) | *mdlC* | *Pseudomonas putida* | 3 | ND | ND | ND |
| 1,2,4-butanetriol dehydratase (diol dehydratase) | *dhaB123* | *Klebsiella pneumoniae* | 4 | ND | ND | ND |
| | *gldABC* | *Klebsiella pneumoniae* | ND | Genbank | U60992 | U60992.1 GI: 1778021 |
| | *pddABC* | *Klebsiella oxytoca* | ND | Genbank | D45071 | D45071.1 GI: 868005 |
| | *dhaBCE* | *Clostridium pasteurianum* | ND | Genbank | AF051373 | AF051373.1 GI:3360388 |
| D-Xylonate dehydratase | *yjhG* | *Escherichia coli* | 5 | ND | ND | ND |
| | *yagF* | *Escherichia coli* | 6 | ND | ND | ND |
| Alcohol dehydrogenase (NADPH dependant 1,2,4-butanetriol dehydrogenase; NADPH dependant 1,4-butanediol dehydrogenase) | *adhP* | *Escherichia coli* | 7 | ND | ND | ND |
| | *yqhD* | *Escherichia coli* | 8 | ND | ND | ND |
| D-xylose isomerase | *xylA* | *Escherichia coli* | 9 | ND | ND | ND |
| D-xylulose kinase | *xylB* | *Escherichia coli* | 10 | ND | ND | ND |
| 3-deoxy-D-glero-pentulosonic acid aldolase | *yjhH* | *Escherichia coli* | 11 | ND | ND | ND |
| | *yagE* | *Escherichia coli* | 12 | ND | ND | ND |
| keto-acid dehydrogenase | *yiaE* | *Escherichia coli* | 53 | ND | ND | ND |
| | *ycdW* | *Escherichia coli* | 54 | ND | ND | ND |
| Glucose phophotransferase Enzyme IIBC(Glc) (glucose permease) | *ptsG* | *Escherichia coli* | 13 | ND | ND | ND |

(continued)

| Enzyme full name(s) | Gene name | Origin (Genus species) | SEQ ID NO | Database | Accession number in the database | Version number in the database |
|---|---|---|---|---|---|---|
| EIIA(Glc), phosphocarrier for glucose PTS transport (Carbohydrate repression resistance) | *crr* | *Escherichia coli* | 14 | ND | ND | ND |
| Histine protein (PTS system histidine phosphocarrier protein HPr, (phosphohistidinopro tein-hexose phosphotransferase) | *ptsH (hpr)* | *Escherichia coli* | 15 | ND | ND | ND |
| lactose permease | *lacY* | *Escherichia coli* | 16 | ND | ND | ND |
| membrane subunit of the maltose ABC transporter | *malF* | *Escherichia coli* | 17 | ND | ND | ND |
| | *malG* | *Escherichia coli* | 18 | ND | ND | ND |
| importer of sucrose | *scrKYABR* | *Salmonella typhimurium* | 19 | ND | ND | ND |
| sucrose:proton symport transport system | *cscBKAR* | *Escherichia coli* | 20 | ND | ND | ND |
| importer of xylose | *xylFGH* | *Escherichia coli* | 21 | ND | ND | ND |
| glucose permease (galactose:H+ symporter) | *galP* | *Escherichia coli* | 22 | ND | ND | ND |
| glucose facilitator | *glf* | *Zymomonas mobilis* | 23 | ND | ND | ND |
| glucokinase | *glk* | *Zymomonas mobilis* | 24 | ND | ND | ND |
| | *glk* | *Escherichia coli* | 25 | ND | ND | ND |
| Adenylate cyclase | *cyaA* | *Escherichia coli* | 26 | ND | ND | ND |
| CRP (cAMP receptor protein ; cAMP-activated global transcription factor) | *crp* | *Escherichia coli* | 27 | ND | ND | ND |
| | *crp** | *Escherichia coli* | 28 | ND | ND | ND |
| | | | 29 | ND | ND | ND |
| | | | 30 | ND | ND | ND |
| | | | 31 | ND | ND | ND |
| | | | 32 | ND | ND | ND |
| CRP-like protein (catabolite control protein A) | *ccpA* | *Bacillus subtilis* | ND | NCBI | NC_000964 (entire genome) | NC_000964.3 GI: 255767013 |

(continued)

| Enzyme full name(s) | Gene name | Origin (Genus species) | SEQ ID NO | Database | Accession number in the database | Version number in the database |
|---|---|---|---|---|---|---|
| vitamin B12 outer membrane porin (vitamin B12 receptor precursor protein) | *btuB* | *Escherichia coli* | 33 | ND | ND | ND |
| membrane subunit of the vitamin B12 ABC transporter | *btuC* | *Escherichia coli* | 34 | ND | ND | ND |
| ATP-or GTP-binding protein of the vitamin BI2 transporter | *btuD* | *Escherichia coli* | 35 | ND | ND | ND |
| periplasmic binding protein of the vitamin BI2 transporter | *btuF* | *Escherichia coli* | 36 | ND | ND | ND |
| cobalamin adenosyltransferase (Cobinamide adenosyltransferase) | *btuR* | *Escherichia coli* | 37 | ND | ND | ND |
| 1,2,4-butanetriol dehydratase reactivase (α subunit) | *dhaB4* | *Klebsiella pneumoniae* | 38 | ND | ND | ND |
| | *ddrA* | *Klebsiella oxytoca* | ND | Genbank | AF017781 | AF017781.1 GI:3115375 |
| | *orfZ* | *Clostridium pasteurianum* | ND | Genbank | AF051373 | AF051373.1 GI:3360388 |
| 1,2,4-butanetriol dehydratase reactivase (β subunit) | *orf2b* | *Klebsiella pneumoniae* | 39 | ND | ND | ND |
| | *ddrB* | *Klebsiella oxytoca* | ND | Genbank | AF017781 | AF017781.1 GI:3115375 |
| | *orfX* | *Clostridium pasteurianum* | ND | Genbank | AF006034 (operon) | AF006034.1 GI:2393883 |
| unknown function | *orf2a* | *Klebsiella pneumoniae* | 40 | ND | ND | ND |
| | *orfY* | *Clostridium pasteurianum* | ND | Genbank | AF006034 (operon) | AF006034.1 GI:2393883 |
| membrane-bound transhydrogenase (membrane bound proton translocating pyridine nucleotide transhydrogenase) | *pntAB* | *Escherichia coli* | 41 | ND | ND | ND |
| soluble pyridine nucleotide transhydrogenase | *sthA (udhA)* | *Escherichia coli* | 42 | ND | ND | ND |
| NADP-dependent glyceraldehyde-3-phosphate dehydrogenase | *gapN* | *Streptococcus mutans* | 43 | ND | ND | ND |

(continued)

| Enzyme full name(s) | Gene name | Origin (Genus species) | SEQ ID NO | Database | Accession number in the database | Version number in the database |
|---|---|---|---|---|---|---|
| NADH generating glyceraldehyde-3-phosphate (Glyceraldehyde 3-phosphate dehydrogenase A) | *gapA* | *Escherichia coli* | 44 | ND | ND | ND |
| Glucose-6-phosphate isomerase (phosphoglucose isomerase) | *pgi* | *Escherichia coli* | 45 | ND | ND | ND |
| Phosphofructokinase (6-phosphofructokinase-1) | *pfkA* | *Escherichia coli* | 46 | ND | ND | ND |
| Glucose-6-phosphate 1-dehydrogenase | *zwf* | *Escherichia coli* | 47 | ND | ND | ND |
| NADPH generating dihydrolipoamide dehydrogenase (lipoamide dehydrogenase) | *lpd* | *Escherichia coli* | 48 | ND | ND | ND |
| | *lpd** | *Escherichia coli* | 49 | ND | ND | ND |
| Bifunctional NAD(P)H-hydrate repair enzyme (NAD(P)HX epimerase / NAD(P)HX dehydratase) | *yjeF (nrr)* | *Escherichia coli* | 50 | ND | ND | ND |

In another aspect, a method for an optimized production of 1,4-butanediol by fermentation is described herein comprising:

a) culturing the genetically modified microorganism as described above in a culture medium comprising xylose and a carbon source other than xylose, and

b) recovering the 1,4-butanediol from said culture medium.

Fermentation mediums and sources of carbon are well known in the art. The terms "fermentative process," "fermentation" or "culture" as used herein are used interchangeably to refer to the experimental conditions allowing the growth of a given microorganism. The growth of a microorganism is generally performed in fermenters with an appropriate growth medium adapted to the microorganism being used.

An "appropriate culture medium" means herein a medium (e.g., a sterile, liquid media) comprising nutrients essential or beneficial to the maintenance and/or growth of the microorganism such as carbon sources or carbon substrates; nitrogen sources, for example peptone, yeast extracts, meat extracts, malt extracts, urea, ammonium sulfate, ammonium chloride, ammonium nitrate and ammonium phosphate; phosphorus sources, for example monopotassium phosphate or dipotassium phosphate; trace elements (e.g., metal salts) for example magnesium salts, cobalt salts and/or manganese salts; as well as growth factors such as amino acids and vitamins.

The source of carbon other than xylose is preferably a carbohydrate.

Said carbohydrate can be selected from the group consisting of monosaccharides such as glucose, fructose, mannose, arabinose, galactose and the like, disaccharides such as sucrose, cellobiose, maltose, lactose and the like, oligosaccharides such as raffinose, stacchyose, maltodextrins and the like, polysaccharides such as cellulose, hemicellulose, starch and the like, methanol, formaldehyde and glycerol. Particularly preferred carbohydrates according to the invention comprise 3, 6 or 12 carbon atoms, and are more preferably glycerol, glucose, fructose, galactose, lactose, maltose, sucrose and any combination thereof. Most preferably, the carbohydrate is sucrose.

In a particular example, the carbon source, preferably the carbohydrate, is derived from renewable feed-stock, such as vegetable biomass.

The person skilled in the art can easily determine the culture conditions necessary for growing the microorganism described herein. In particular, it is well-known that bacteria can be fermented at a temperature comprised between 20°C and 55°C, preferentially between 25°C and 40°C. *E. coli* can more particularly be cultured at a temperature comprised between about 30°C and about 37°C.

The method described herein can be performed either in a batch process, in a fed-batch process or in a continuous process, and under aerobic, micro-aerobic or anaerobic conditions.

A fermentation "under aerobic conditions" means that oxygen is provided to the culture by dissolving gas into the liquid phase of the culture. This can be achieved by (1) sparging oxygen containing gas (e.g. air) into the liquid phase, or (2) shaking the vessel containing the culture medium in order to transfer the oxygen contained in the head space into the liquid phase. The main advantage of fermentation under aerobic conditions is that the presence of oxygen as an electron acceptor improves the capacity of the strain to produce more energy in the form of ATP for cellular processes, thereby improving the general metabolism of the strain.

Micro-aerobic conditions can be used herein and are defined as culture conditions wherein low percentages of oxygen (e.g. using a mixture of gas containing between 0.1 and 10% of oxygen, completed to 100% with nitrogen) are dissolved into the liquid phase.

By contrast, "anaerobic conditions" are defined as culture conditions wherein no oxygen is provided to the culture medium. Strictly anaerobic conditions can be obtained by sparging an inert gas like nitrogen into the culture medium to remove traces of other gas. Nitrate can be used as an electron acceptor to improve ATP production by the strain and improve its metabolism.

According to a particular example, the method further comprises a step c) of purifying the 1,4-BDO of step b). Methods for purifying 1,4-BDO are well-known in the art, and have notably been described in WO 2010/141780, US 2011/0003355, WO 2013/183592 and WO 2014/152665.

## DRAWINGS

Figure 1 represents the metabolic pathway for the conversion of D-xylose into 1,4-BDO.

## EXAMPLES

The present invention is further defined in the following examples. It should be understood that these examples, while indicating preferred embodiments of the invention, are given by way of illustration only. From above disclosure and these examples, the man skilled in the art can make various changes to the invention to adapt it to various uses and conditions without modifying the essentials means of the invention.

Exemplary genes and enzymes required for constructing microorganisms with these capabilities are described as well as methods for cloning and transformation, monitoring product formation and using the engineered microorganisms for production.

In particular, examples show modified *Escherichia coli* (*E. coli*) strains, but these modifications can easily be performed in other microorganisms of the same family.

*Escherichia coli* belongs to the *Enterobacteriaceae* family, which comprises members that are Gram-negative, rod-shaped, non-spore forming and are typically 1-5 μm in length. Most members have flagella used to move about, but a few genera are non-motile. Many members of this family are a normal part of the gut flora found in the intestines of humans and other animals, while others are found in water or soil, or are parasites on a variety of different animals and plants. *E. coli* is one of the most important model organism, but other important members of the *Enterobacteriaceae* family include *Klebsiella,* in particular *Klebsiella terrigena, Klebsiella planticola* or *Klebsiella oxytoca, Pantoea* and *Salmonella.*

## PROTOCOLS

Several protocols have been used to construct 1,4-butanediol producing strains described in the following examples.

**Protocol 1** (Chromosomal modifications by homologous recombination, selection of recombinants and antibiotic cassette excision) and **protocol 2** (Transduction of phage P1) used in this invention have been fully described in the patent application WO2013/001055.

**Protocol 3:** Construction of recombinant plasmids

Recombinant DNA technology is well-described and known by the man skilled in the art.

Briefly, the DNA fragments are PCR amplified using oligonucleotides (the person skilled in the art is able to design) and MG1655 or other microorganism genomic DNA as matrix (according to the targeted gene). The DNA fragments and selected plasmid are digested with compatible restriction enzymes, ligated and then transformed in competent cells. Transformants are analysed and recombinant plasmids of interest are verified by DNA sequencing.

**Example 1: Overproduction of the enzymes of the metabolic pathway for the conversion of xylose into 1,4-butanediol and deletion of enzymes using xylose or 1,4-butanediol biosynthesis intermediates in an Escherichia coli strain - Construction of strains 1 to 8.**

*Escherichia coli* strain MG1655 was modified to produce 1,4-butanediol (1,4-BDO) from D-xylose using the pathway illustrated in Figure 1. The work seeks to maximize the carbon flux toward the production of 1,4-BDO and so to remove all the enzymes involved in other xylose consuming pathways or involved in conversion of 1,4-BDO-intermediate-compounds, which represents a loss of product.

In addition to the genes naturally expressed by *E. coli* (*yjhG* gene of sequence SEQ ID NO:5 and *yagF* gene of sequence SEQ ID NO:6, encoding for xylonate dehydratases, and *adhP* gene of sequence SEQ ID NO:7 and *yqhD* gene of sequence SEQ ID NO:8 encoding alcohol dehydrogenase - NADPH-dependant 1,2,4-butanetriol or 1,4-butanediol dehydrogenase), the genes coding for the following enzymes; the xylose dehydrogenase and the xylonolactonase of *Caulobacter crescentus* (*xdh* [CC0821 on CauloCyc, SEQ ID NO:1] and *xylC* [CC0820 on CauloCyc, SEQ ID NO:2], respectively), the 3-deoxy-D-glycero-pentulosonate decarboxylase of *Pseudomonas putida* (*mdlC* gene of sequence SEQ ID NO:3) and the 1,2,4-butanetriol dehydratase from *Klebsiella pneumoniae* (*dhaB123* gene of sequence SEQ ID NO:4) genes were heterologously and separately expressed with a Ptrc artificial promoter (sequence given in patent WO 2007/0770441) and their own ribosome binding site, using a pCL1920 plasmid (Lerner & Inouye, 1990). In fact, genes *xdh, xylC, mdlC* and the *dhaB123* operon were sequentially cloned on the pCL1920 giving the plasmid pBDO0001.

Moreover, in order to block the native xylose catabolic pathway, the genes encoding for the D-xylose isomerase (*xylA* gene of sequence SEQ ID NO:9) and the D-xylulose kinase (*xylB* gene of sequence SEQ ID NO:10) were deleted from the *E. coli* MG1655 chromosome using the homologous recombination strategy described by Datsenko & Wanner, 2000, and according to Protocol 1. More precisely, to delete *xylAB* operon, a PCR product carrying the antibiotic resistance gene together with FRT sites surrounded by sequences homologous to upstream and downstream regions of *xylAB* operon (SEQ ID NO:51 and SEQ ID NO:52), was generated and introduced into *E. coli* MG1655 in which the pKD46 vector was previously transformed. The antibiotic resistant transformants were verified with the appropriate oligonucleotides and the retained strain was named strain 1.

To avoid the degradation of the 3-deoxy-D-glycose pentulosonic acid (DGP), the genes encoding the keto acid dehydrogenases (*yiaE* gene of sequence SEQ ID NO:53 ; and *ycdW* gene of sequence SEQ ID NO:54) and the DGP aldolases (*yjhH* gene of sequence SEQ ID NO:11; and *yagE* gene of sequence SEQ ID NO:12) were also deleted using the same homologous recombination strategy. More precisely, to delete the *yjhH* gene (SEQ ID NO:11), a PCR product carrying the antibiotic resistance gene together with FRT sites, surrounded by sequences homologous to upstream and downstream regions of the *yjhH* gene (SEQ ID NO:55 and SEQ ID NO:56), was generated and introduced into strain 1 in which the pKD46 vector was previously transformed. The antibiotic resistant transformants were verified with the appropriate oligonucleotides and the retained strain was named strain 2. Then, to delete the *yagE* gene (SEQ ID NO:12), a PCR product carrying the antibiotic resistance gene together with FRT sites, surrounded by sequences homologous to upstream and downstream regions of the *yagE* gene (SEQ ID NO:57 and SEQ ID NO:58), was generated and introduced into strain 2 in which the pKD46 vector was previously transformed. The antibiotic resistant transformants were verified with the appropriate oligonucleotides and the retained strain was named strain 3. Next, to delete the *yiaE* gene (SEQ ID NO:53), a PCR product carrying the antibiotic resistance gene together with FRT sites, surrounded by sequences homologous to upstream and downstream regions of the *yiaE* gene (SEQ ID NO:59 and SEQ ID NO:60), was generated and introduced into strain 3 in which the pKD46 vector was previously transformed. The antibiotic resistant transformants were verified with the appropriate oligonucleotides and the retained strain was named strain 4. Finally, to delete the *ycdW* gene (SEQ ID NO:54), a PCR product carrying the antibiotic resistance gene together with FRT sites, surrounded by sequences homologous to upstream and downstream regions of the *ycdW* gene (SEQ ID NO:61 and SEQ ID NO:62), was generated and introduced into strain 4 in which the pKD46 vector was previously transformed. The antibiotic resistant transformants were verified with the appropriate oligonucleotides and the retained strain was named strain 5.

As the *yjhH* gene belongs together with *yjhG* (SEQ ID NO:5) and *yjhI* to the *yjhIHG* operon, the sequences homologous to upstream and downstream regions of *yjhH* must be chosen as to not alter the expression of surrounding genes. It was the same for the *yagE* gene (SED ID NO:12) which belongs to the *yagEF* operon.

Finally, to suppress the catabolite repression, the glucose phophotransferase enzyme IIBC(Glc) encoded by the *pts G* gene (SEQ ID NO:13), was deleted by using the homologous recombination strategy described by Datsenko & Wanner, 2000 (according to Protocol 1) as described in patent application EP 14305691.9, in particular in Example 2 of said document (referred to herein as SEQ ID NO:63 and SEQ ID NO:64). The appropriate PCR product was introduced into strain 5 in which the pKD46 vector was previously transformed. The antibiotic resistant transformants were verified with the appropriate oligonucleotides and the retained strain was named strain 6.

Each time, a different antibiotic resistance gene was used among kanamycin, chloramphenicol, gentamycin, tetracycline, blasticidin or spectinomycin.

Before using strain 6, the antibiotic cassettes were removed from Δ*xylAB,* Δ*yjhH,* Δ*yagE,* Δ*yiaE,* Δ*ycdW* modifications using the Flp recombinase as described by Datsenko & Wanner, 2000 (according to Protocol 1), giving rise to strain 7.

Then, the plasmid pBDO0001 described above was introduced into strain 7, giving rise to strain 8.

**Example 2: Enhancing of coenzyme B12 production/regeneration capabilities of the 1,4-butanediol producing *E. coli* recombinant strain - Construction of strains 9 to 11.**

The 1,2,4-butanetriol dehydratase DhaB123 from *Klebsiella pneumoniae* uses coenzyme B12 to function, so the genes involved in coenzyme B12 production/regeneration were overexpressed.

First, the genes encoding the 1,2,4-butanetriol dehydratase reactivase of *Klebsiella pneumoniae, dhaB4* (SEQ ID NO:38) as well as *orf2a* (SEQ ID NO:40) and *orf2b* (SEQ ID NO:39) from *Klebsiella pneumoniae,* were overexpressed with a Ptrc artificial promoter. More precisely, the *dhaB4* gene was cloned with its own ribosome binding site on plasmid pBDO0001 downstream of the *dhaB123* genes by keeping the operon structure, and the *orf2a* and *orf2b* genes were cloned afterwards together with a Ptrc artificial promoter downstream of *dhaB1234* operon, also on the pBDO0001 plasmid, giving rise to plasmid pBDO0002.

Secondly, the cobalamin adenosyltransferase encoded by the *btuR* gene (SEQ ID NO:37) was overproduced by replacing the natural promoter of the *yciK-btuR* operon with an artificial promoter.

To replace the natural promoter of the *yciK-btuR* operon (*yciK:* SEQ ID NO: 65) on the chromosome, the homologous recombination strategy described by Datsenko & Wanner, 2000 (according to Protocol 1) was used. More precisely, a PCR product carrying the Ptrc artificial promoter (SEQ ID NO: 66) and the kanamycin resistance gene together with FRT sites, surrounded by sequences homologous to *yciK* gene and to the upstream region of the *yciK* gene on the chromosome (SEQ ID NO: 67 and SEQ ID NO: 68), was generated and introduced into strain 7 in which the pKD46 vector was previously transformed. The kanamycin resistant transformants were verified with the appropriate oligonucleotides and the retained strain was named strain 9.

Before using strain 9, the antibiotic cassette was removed from *yciK-btuR* locus using the Flp recombinase according to Protocol 1, giving rise to strain 10.

Finally, the plasmid pBDO0002 was introduced into strain 10, giving rise to strain 11.

**Example 3: Improving 1,4-butanediol production by increasing NADPH availability of the 1,4-butanediol producing *E. coli* recombinant strain - Construction of strains 12 to 17.**

The alcohol dehydrogenases, the NADPH-dependant 1,2,4-butanetriol dehydrogenase and the 1,4-butanediol dehydrogenase, encoded by *adhP* and *yqhD,* are in need of reducing power available in the organism, particularly in the form of NADPH, so the genes involved in NADPH production were overexpressed.

The membrane bound proton translocating pyridine nucleotide transhydrogenase encoded by the *pntAB* operon (SEQ ID NO:41) was overproduced by replacing the endogenous promoter and ribosome binding site of the *pntA* gene of *Escherichia coli* MG1655 by the inducible Ptrc promoter (from the plasmid pTRC99A, Amersham Pharmacia) and the define ribosome binding site RBS120 (from RBS Calculator software), as described in patent application EP 14305691.9, in particular in Example 4 of said document (referred herein as SEQ ID NO:69). The appropriate PCR product described in patent application EP 14305691.9 was introduced into strain 10 in which the pKD46 vector was previously transformed. The antibiotic resistant transformants were verified with the appropriate oligonucleotides and the retained strain was named strain 12.

The soluble pyridine nucleotide transhydrogenase encoded by the *sthA* gene (previously known as *udhA,* and of sequence SEQ ID NO: 42) was deleted by using the homologous recombination strategy described by Datsenko & Wanner, 2000 (according to Protocol 1) and as described in patent application WO 2012/055798, in particular in Example 2 of said application (referred to herein as SEQ ID NO:70 and SEQ ID NO:71). The appropriate PCR product was introduced into strain 12 in which the pKD46 vector was previously transformed. The antibiotic resistant transformants were verified with the appropriate oligonucleotides and the retained strain was named strain 13.

The phosphofructokinase encoded by the *pfkA* gene (SEQ ID NO:46) was deleted by using the homologous recombination strategy described by Datsenko & Wanner, 2000 (according to Protocol 1), and as described in patent application EP 14305691.9, in particular in Example 5 of said document (referred to herein as SEQ ID NO:72 and SEQ ID NO:73). The appropriate PCR product was introduced into strain 13 in which the pKD46 vector was previously transformed. The antibiotic resistant transformants were verified with the appropriate oligonucleotides and the retained strain was named strain 14.

The NAD-dependent glyceraldehyde phosphate dehydrogenase encoded by the *gapA* gene from *Escherichia coli* (SEQ ID NO: 44) was replaced by the *gapN* gene from *Streptococcus mutans* (SEQ ID NO: 43) coding for a NADP-dependent glyceraldehyde-3-phosphate dehydrogenase (Centeno-Leija et al., 2013) by using the homologous recombination strategy described by Datsenko & Wanner, 2000 (according to Protocol 1). The substitution of the *gapA* gene by the *gapN* gene was done by simultaneous substitution of the *gapA* promoter and ribosome binding site by the thermoinducible PR01 promoter (SEQ ID NO:74) and its cognate thermolabile repressor CI857 (SEQ ID NO:75) as described in Example 1 of patent application EP 2532751, and the define ribosome binding site RBS150 (from RBS Calculator software, SEQ ID NO: 76). More precisely, a PCR product carrying the CI857 gene, the PR01 promoter, the RBS150 ribosome binding site, the *gapN* gene from *Streptococcus mutans* and the antibiotic resistance gene together with FRT sites, surrounded by sequences homologous to the upstream and downstream regions of *gapA* gene on the chromosome (SEQ ID NO:77 and SEQ ID NO:78), was generated and introduced into strain 14 in which the pKD46 vector was previously transformed. The antibiotic resistant transformants were verified with the appropriate oligonucleotides and the retained strain was named strain 15.

Before using strain 15, the antibiotic cassettes were removed from the *pntAB, udhA, pfkA* and *gapA* loci using the Flp recombinase according to Protocol 1, giving rise to strain 16.

Finally, the plasmid pBDO0002 described above was introduced into strain 16, giving rise to strain 17.

**Example 4: Overproducing the enzyme involved in sucrose metabolism in the 1,4-butanediol producing *E. coli* recombinant strain - Construction of strains 18 to 20.**

To avoid catabolite repression when the strain is grown on a mix of sucrose and xylose, the natural promoter of the operon *xylFGH* coding for the importer of xylose (XylF the periplasmic protein, XylG the ATP binding subunit, XylH the membrane subunit) was replaced by an artificial one by using the homologous recombination strategy described by Datsenko & Wanner, 2000 (according to Protocol 1). More precisely, a PCR product carrying the Ptrc artificial promoter (SEQ ID NO: 66) and the kanamycin resistance gene together with FRT sites, surrounded by sequences homologous to the *xylF* gene and to the upstream region of the *xylF* gene on the chromosome (SEQ ID NO:79 and SEQ ID NO:80), was generated and introduced into strain 16 in which the pKD46 vector was previously transformed. The kanamycin resistant transformants were verified with the appropriate oligonucleotides and the retained strain was named strain 18. Before using strain 18, the antibiotic cassette was removed from the *xylFGH* operon using the Flp recombinase according to Protocol 1, giving rise to strain 19.

To allow growth of *E. coli* on sucrose, the genes encoding the fructokinase, *scrK,* the porine, *scrY,* the protein IIBC, *scrA,* the sucrose-6-phosphate invertase, *scrB* and the repressor, *scrR* from the plasmid pUR400 of *Salmonella typhimurium* (Schmidt et al., 1982, SEQ ID NO:19) were cloned under their natural promoters on a pBBR1MCS plasmid (Kovach et al., 1995), giving the plasmid pBDO0003.

Finally, the plasmids pBDO0002 and pBDO0003 were introduced into strain 19, giving rise to strain 20.

**Example 5: Microbial production of 1,4-butanediol.**

Production strains were evaluated in 500 ml baffled Erlenmeyer flasks using modified M9 medium (Anderson, 1946) that was supplemented with 0.5 mg/L of coenzyme B12, 30 g/l MOPS, 20g/L D-xylose and 10 g/l glucose and adjusted to pH 6.8. Spectinomycin was added at a concentration of 50 $mg.L^{-1}$ and tetracycline at 5 $mg.L^{-1}$ when it was necessary in preculture and culture. A preculture was grown at 37°C in LB medium (Sigma). After 24 hours of growth, it was used to inoculate a 50 mL culture of modified M9 medium to an OD600 of about 0.2, at 30°C and 200 rpm. When sugars in the culture medium were exhausted, the culture was centrifuged and the broth analysed for 1,4-butanediol by GC-MS.

The 1,4-butanediol titer was expressed as follows:

$$Titer_{BDO} = \frac{1,4-bu\tan ediol\ (mg)}{volume\ (L)}$$

**Table 2: 1,4-butanediol (BDO) titer for each strain evaluated in Erlenmeyer flasks.**

| The sign (-) means a production of 1,4-BDO inferior to 5 mg/L, while the sign (+) corresponds to a production of 1,4-BDO comprised between 5 and 15 mg/L, and the sign (++) corresponds to a production of 1,4-BDO above 15 mg/L. | |
|---|---|
| Strain | BDO Yield |
| MG1655 | - |
| Strain 8 | + |
| Strain 11 | + |
| Strain 17 | ++ |

Production of 1,4-butanediol was observed for all recombinant strains. The production was enhanced by the genetic modifications performed to increase the NADPH pool in the cell.

Strain 20 (not presented in table 2) was tested on a mix of xylose and sucrose in the same conditions as described above in example 5, and its 1,4-BDO production was similar to strain 17.

**REFERENCES**

- Altschul S, Gish W, Miller W, Myers E, Lipman DJ, J. Mol. Biol, 1990, 215 (3): 403-410
- Anderson, Proc. Natl. Acad. Sci. USA., 1946, 32:120-128
- Bocanegra J, Scrutton N, Perham R, Biochemistry, 1993, 32 (11): 2737-2740
- Carrier T & Keasling J, Biotechnol Prog., 1999, 15 (1): 58-64
- Centeno-Leija S, Utrilla J, Flores N, Rodriguez A, Gosset G, Martinez A, Antonie Van Leeuwenhoek., 2013, 104 (6), 913-924.
- Datsenko KA & Wanner BL, Proc Natl Acad Sci U S A., 2000, 97: 6640-6645
- Hogema et al., Molecular microbiology, 1997, 24-857-867
- Kim, J. H. et al, Appl. Microbiol. Biotechnol., 2010, 88, 1077-1085
- Kovach ME, Elzer PH, Hill DS, Robertson GT, Farris MA, Roop RM, Peterson KM., Gene, 1995, 166(1): 175-6
- Lee S, McCormick M, Lippard S, Cho U, Nature, 2013, 494: 380-384
- Lerner C.G. and Inouye M., Nucleic Acids Research, 1990, 18(15):4631
- Lim S, Jung Y, Shin H, Lee Y, J Biosci Bioeng., 2002, 93 (6):543-549
- Marbaix A, Noel G, Detroux A, Vertommen D, Schaftingen E, Linster C, J Biol Chem., 2011, 286 (48):, 41246-41252
- Salis H, Methods Enzymol., 2011, 498:19-42
- Sampat, B.G., Chemical Weekly Special Report, 2011, January 18, pp. 205-211
- Schmid K, Schupfner M, Schmitt R, J. Bacteriol., 1982, 151: 68-76
- Segel IH, Enzyme kinetics, (1993), John Wiley & Sons, pp. 44-54 and 100-112


## Claims

**1.** A genetically modified *Escherichia coli* for the production of 1,4-butanediol by exclusively converting xylose into 1,4-butanediol, wherein said microorganism further comprises:

i) the deletion of the following endogenous genes:

- the *xylA* gene encoding a xylose isomerase,
- the *xylB* gene encoding a xylulose kinase,
- the *yjhH* and *yagE* genes encoding a 3-deoxy-D-glycero-pentulosonate aldolase,
- the *yiaE* and *ycdW* genes encoding a keto-acid dehydrogenase, thereby providing reducing power and/or energy for 1,4-butanediol production and microorganism growth from a carbon source other than xylose, and

ii) at least one of the following genetic modifications at least partially inhibiting carbon catabolite repression:

- deletion of the endogenous ptsG gene encoding a glucose phophotransferase,
- deletion of the endogenous *ptsH* gene encoding a phosphocarrier Hpr protein,
- expression of the endogenous *lacY* gene and/or the endogenous *malFG* and/or *cscBKAR* operon or the heterologous *scrKYABR* operon from *Salmonella typhimurium,* involved in a sugar importer system wherein said sugar is a carbon source other than xylose,
- expression of the endogenous *xylFGH* operon encoding an ABC xylose transporter,
- overexpression of the endogenous *galP* gene encoding a glucose permease,
- overexpression of the heterologous *glf* gene encoding a glucose facilitator from *Zymomonas mobilis,*
- overexpression of the endogenous *glk* gene encoding a glucokinase,
- expression of a gene encoding a cAMP-independent CRP protein, wherein said CRP protein is coded by SEQ ID NO: 28, 29, 30, 31, or 32, and
- any combination thereof,

thereby optimizing the production of 1,4-butanediol by said microorganism.

**2.** The microorganism according to claim 1, wherein said source of carbon other than xylose is selected from at least one carbohydrate comprising 3, 6 or 12 carbon atoms and any combination thereof.

**3.** The microorganism according to claim 1 or 2, wherein the genetic modification for exclusively converting xylose into 1,4-butanediol is an overexpression of at least one the following genes:

- a heterologous *xdh* or gfo2 gene encoding a xylose dehydrogenase,

- a heterologous *xylC* gene encoding a xylolactone hydrolase,
- a heterologous *mdlC* gene encoding a 3-deoxy-D-glycero-pentulosonate decarboxylase,
- a heterologous *dhaB123, gldABC, pddABC,* or dhaBCE gene encoding a 1,2,4-butanetriol dehydratase,
- an endogenous *yhjG* or *yagF* gene encoding a D-xylonate dehydratase,
- an endogenous *adhP* or *yqhD* gene encoding a NADPH dependant 1,2,4-butanetriol dehydrogenase
- an endogenous *adhP* or *yqhD* gene encoding a NADPH dependant 1,4-butanediol dehydrogenase, and
- any combination thereof.

**4.** The microorganism according to any one of claims 1 to 3, comprising as a further genetic modification an overexpression of at least one gene involved in vitamin B12 transport, preferably selected from:

- an endogenous *btuB* gene encoding a vitamin B12 outer membrane porin,
- an endogenous *btuC* gene encoding a membrane subunit of the vitamin B12 ABC transporter,
- an endogenous *btuD* gene encoding an ATP- or GTP-binding protein of the vitamin B12 transporter, and
- any combination thereof.

**5.** The microorganism according to any one of claims 1 to 4, comprising as a further genetic modification an overexpression of at least one gene involved in coenzyme B12 utilisation and/or 1,2,4-butanetriol dehydratase reactivation, preferably selected from:

- an endogenous *btuR* gene encoding a cobalamine adenosyl transferase,
- at least one heterologous gene selected from *dhaB4, orf2b, ddrA, ddrB, orfZ,* and *orfX* encoding a 1,2,4-butanetriol dehydratase reactivase, and
- any combination thereof.

**6.** The microorganism according to any one of claims 1 to 5, comprising a further genetic modification of at least one gene involved in the production of NADPH as a source of reducing power, said modification being preferably selected from:

- overexpression of an endogenous *pntAB* gene or operon encoding a membrane-bound transhydrogenase,
- deletion of an endogenous *udhA* gene encoding a soluble transhydrogenase,
- overexpression of an endogenous *gapA* gene or a heterologous gapN gene encoding a NADPH generating glyceraldehyde 3-phosphate dehydrogenase,
- deletion of an endogenous *pgi* gene encoding a phosphoglucose isomerase,
- deletion of an endogenous *pfkA* gene encoding a phosphofructokinase,
- overexpression of an endogenous zwf gene encoding a glucose-6-phosphate dehydrogenase,
- overexpression of an endogenous mutant *lpd* gene encoding a lipoamide dehydrogenase capable of generating NADPH,
- overexpression of an endogenous *yjeF* gene encoding a bi-functional NAD(P)H-hydrate repair enzyme, and
- any combination thereof.

**7.** The microorganism according to any one of claims 1 to 6, wherein the metabolic pathway of said microorganism for the exclusive conversion of xylose into 1,4-butanediol comprises at least the steps:

- elimination of one carbon from the xylose backbone, thereby obtaining an intermediate metabolite having an aliphatic backbone of 4 carbons, and
- elimination of one hydroxyl from said intermediate metabolite.

**8.** A method for an optimized production of 1,4-butanediol by fermentation comprising:

a) culturing a genetically modified microorganism as defined in any one of claims 1 to 7 in a culture medium comprising xylose and a carbon source other than xylose, under fermentation conditions allowing conversion of xylose into 1,4-butanediol, and
b) recovering the 1,4-butanediol from said culture medium.

**9.** The method of claim 8, wherein said carbon source is as defined in claim 2.

**10.** The method of claim 9, wherein said carbon source is selected from the group consisting of glycerol, glucose, galactose, fructose, lactose, maltose, sucrose, and any combination thereof.

## Patentansprüche

1. Genetisch modifizierte Escherichia coli zur Herstellung von 1,4-Butandiol durch ausschließliches Konvertieren von Xylose in 1,4-Butandiol, wobei der Mikroorganismus ferner umfasst:

   i) die Entfernung der folgenden endogenen Gene:

      das xylA-Gen, das eine Xylose-Isomerase codiert,
      das xylB-Gen, das eine Xylulose-Kinase codiert,
      die yjhH- und yagE-Gene, die eine 3-Deoxy-D-glycero-pentulosonat-aldolase codieren,
      die yiaE and ycdW-Gene, die eine Ketosäuredehydrogenase codieren, wodurch sie Reduktionskraft bereitstellen und/oder Energie für die 1,4-Butandiol-Herstellung und das Wachstum von Mikroorganismen aus einer Kohlenstoffquelle, die keine Xylose ist, und

   ii) mindestens eine der folgenden genetischen Modifikationen, die zumindest teilweise die Kohlenstoff-Katabolit-Repression hemmen:

      Löschen des endogenen ptsG-Gens, das eine Glukose-Phosphotransferase codiert,
      Löschen des endogenen ptsH-Gens, das ein Phosphoträger-Hpr-Protein codiert,
      Expression des endogenen lacY-Gens und/oder des endogenen malFG- und/oder cscBKAR-Operons oder des heterologen scrKYABR-Operons aus Salmonella typhimurium, eingebunden in ein Zucker-Importer-System, wobei der Zucker eine Kohlenstoffquelle ist, die keine Xylose ist,
      Expression des endogenen xylFGH-Operons, das einen ABC-Xylose-Transporter codiert,
      Überexpression des endogenen galP-Gens, das eine Glukose-Permease codiert,
      Überexpression des heterologen glf-Gens, das einen Glukose-Facilitator aus Zymomonas mobilis codiert,
      Überexpression des endogenen glk-Gens, das eine Glucokinase codiert,
      Expression eines Gens, das ein cAMP-unabhängiges CRP-Protein codiert, wobei das CRP-Protein durch SEQ ID Nr. 28, 29, 30, 31 oder 32,
      eine Kombination derselben,
      wodurch die Herstellung von 1,4-Butandiol durch den Mikroorganismus optimiert wird.

2. Mikroorganismus nach Anspruch 1, wobei die Quelle von Kohlenstoff, die keine Xylose ist, ausgewählt wird unter mindestens einem Kohlenwasserstoff, der 3, 6 oder 12 Kohlenstoffatome hat, und eine Kombination derselben.

3. Mikroorganismus nach Anspruch 1 oder 2, wobei die genetische Modifikation zur ausschließlichen Konvertierung von Xylose in 1,4-Butandiol eine Überexpression von mindestens einem der folgenden Gene ist:

   - ein heterologes xdh- oder gfo2-Gen, das eine Xylose-Dehydrogenase codiert,
   - ein heterologes xylC-Gen, das eine Xylolacton-Hydrolase codiert,
   - ein heterologes mdlC-gehen, das eine 3-Deoxy-D-glycero-pentulosonatdecarboxylase codiert,
   - ein heterologes dhaB123-, gldABC-, pddABC- oder dhaBCE-Gen, das eine 1,2,4-Butantrioldehydratase codiert,
   - ein endogenes yhjG- oder yagF-Gen, das eine D-Xylonatdehydrogenase codiert,
   - ein endogenes adhP- oder yqhD-Gen, das eine NADPH-abhängige 1,2,4-Butantrioldehydrogenase codiert,
   - ein endogenes adhP- oder yqhD-Gen, das eine NADPH-abhängige 1,4-Butandioldehydrogenase codiert, und
   - eine Kombination derselben.

4. Mikroorganismus nach einem der Ansprüche 1 bis 3, der als weitere genetische Modifikation eine Überexpression von mindestens einem Gen enthält, das am Vitamin B12-Transport beteiligt ist, vorzugsweise ausgewählt aus:

   - einem endogenen btuB-Gen, das ein Vitamin B12-Außenmembran-Porin codiert,
   - ein endogenes btuC-Gen, das eine Membran-Untereinheit des Vitamin B12-ABC-Transporters codiert,
   - ein endogenes btuD-Gen, das ein ATP- oder GTP-bindendes Protein des Vitamin B12-Transporters codiert, und
   - eine Kombination derselben.

5. Mikroorganismus nach einem der Ansprüche 1 bis 4, der als weitere genetische Modifikation eine Überexpression von mindestens einem Gen umfasst, das an der Nutzung des Coenzyms B12 und/oder an der Reaktivierung von 1,2,4-Butantriol-Dehydratase beteiligt ist, vorzugsweise ausgewählt aus:

   - einem endogenen btuR-Gen, das eine Cobalamin-Adenosyltransferase codiert,
   - mindestens einem heterologen Gen, das ausgewählt wird aus dhaB4, orf2b, ddrA, ddrB, orfZ und orfX, die eine 1,2,4-Butantriol-Dehydratase-Reaktivase codieren,
   - einer Kombination derselben.

6. Mikroorganismus nach einem der Ansprüche 1 bis 5, der eine weitere genetische Modifikation von mindestens einem Gen enthält, das an der Bildung von NADPH als Quelle von Reduktionskraft beteiligt ist, wobei die Modifikation vorzugsweise ausgewählt wird aus:

   - Überexpression eines endogenen pntAB-Gens oder -Operons, das eine membrangebundene Transhydrogenase codiert,
   - Deletion eines endogenen udhA-Gens, das eine lösliche Transhydrogenase codiert,
   - Überexpression eines endogenen gapA-Gens oder eines heterologen gapN-Gens, das eine NADPH erzeugende Glyceraldehyd-3-Phosphatdehydrogenase codiert,
   - Deletion eines endogenen pgi-Gens, das eine Phosphoglucose-Isomerase codiert,
   - Deletion eines endogenen pfkA-Gens, das eine Phosphofructokinase codiert,
   - Überexpression eines endogenen zwf-Gens, das eine Glucose-6-Phosphatdehydrogenase codiert,
   - Überexpression eines endogenen mutierten lpd-Gens, das eine Lipoamiddehydrogenase codiert, die in der Lage ist, NADPH zu erzeugen,
   - Überexpression eines endogenen yjeF-Gens, das ein bifunktionales NAD(P)H-Hydrat-Reparaturenzym codiert, und
   - einer Kombination derselben.

7. Mikroorganismus nach einem der Ansprüche 1 bis 6, wobei der Stoffwechselweg des Mikroorganismus für die ausschließliche Konversion von Xylose in 1,4-Butandiol zumindest die folgenden Schritte umfasst:

   - Beseitigung von einem Kohlenstoffatom aus der Xylose-Hauptkette, dadurch Gewinnen eines Zwischenmetaboliten, der eine aliphatische Hauptkette von 4 Kohlenstoffatomen hat, und
   - Beseitigung von einem Hydroxyl aus dem Zwischenmetaboliten.

8. Verfahren für eine optimierte Herstellung von 1,4-Butandiol durch Fermentierung, umfassend:

   a) Züchten eines genetisch modifizierten Mikroorganismus, wie in einem der Ansprüche 1 bis 7 definiert, in einem Kulturmedium, das Xylose und eine andere Kohlenstoffquelle als Xylose enthält, unter Fermentierungsbedingungen, die eine Konversion von Xylose in 1,4-Butandiol ermöglichen, und
   b) Gewinnen des 1,4-Butandiol aus dem Kulturmedium.

9. Verfahren nach Anspruch 8, wobei die Kohlenstoffquelle so wie in Anspruch 2 definiert ist.

10. Verfahren nach Anspruch 9, wobei die Kohlenstoffquelle ausgewählt wird aus der Gruppe, die aus Glycerol, Glukose, Galaktose, Fructose, Laktose, Maltose, Saccharose und einer Kombination derselben besteht.

## Revendications

1. *Escherichia coli* génétiquement modifié pour la production de 1,4-butanediol par la conversion exclusive de xylose en 1,4-butanediol, dans lequel ledit microorganisme comprend en outre :

   i) la délétion des gènes endogènes suivants :

      - le gène *xylA* codant une xylose isomérase,
      - le gène *xylB* codant une xylulose kinase,
      - les gènes *yjhH* et *yagE* codant une 3-désoxy-D-glycéro-pentulosonate aldolase,
      - les gènes *yiaE* et *ycdW* codant une cétoacide déshydrogénase,

ce qui confère ainsi un pouvoir réducteur et/ou de l'énergie pour la production de 1,4-butanediol et la croissance du micro-organisme à partir d'une source de carbone autre que le xylose, et
ii) au moins l'une des modifications génétiques suivants inhibant au moins partiellement la répression catabolique du carbone :

- la délétion du gène *ptsG* endogène codant une glucose phosphotransférase ;
- la délétion du gène *ptsH* endogène codant une protéine de phosphotransport Hpr,
- l'expression du gène *lacY* endogène et/ou de l'opéron *malFG et/ou cscBKAR* endogène ou de l'opéron *scrKYABR* hétérologue provenant de *Salmonella typhimurium,* impliqués dans un système importateur de sucre dans lequel ledit sucre est une source de carbone autre que le xylose,
- l'expression de l'opéron *xylFGH* endogène codant un transporteur de xylose ABC,
- la surexpression du gène *galP* endogène codant une glucose perméase,
- la surexpression du gène *glf* hétérologue codant un facilitateur de glucose provenant de *Zymomonas mobilis,*
- la surexpression du gène *glk* endogène codant une glucokinase,
- l'expression d'un gène codant une protéine CRP indépendante de cAMP, laquelle protéine CRP est codée par la SEQ ID NO : 28, 29, 30, 31 ou 32, et
- l'une quelconque de leurs combinaisons,

ce qui optimise ainsi la production de 1,4-butanediol par ledit microorganisme.

**2.** Microorganisme selon la revendication 1, dans lequel ladite source de carbone autre que le xylose est sélectionnée parmi au moins un hydrate de carbone comprenant 3, 6 ou 12 atomes de carbone et l'une quelconque de leurs combinaisons.

**3.** Microorganisme selon la revendication 1 ou 2, dans lequel la modification génétique pour la conversion exclusive du xylose en 1,4-butanediol est une surexpression d'au moins l'un des gènes suivants :

- un gène *xdh* ou *gfo2* hétérologue codant une xylose déshydrogénase,
- un gène *xylC* hétérologue codant une xylolactone hydrolase,
- un gène *mdlC* hétérologue codant une 3-désoxy-D-glycéro-pentulosonate décarboxylase,
- un gène *dhaB123, gldABC, pddABC* ou *dhaBCE* hétérologue codant une 1,2,4-butanetriol déshydratase,
- un gène *yhjG* ou *yagF* endogène codant une D-xylonate déshydratase,
- un gène *adhP* ou *yqhD* endogène codant une 1,2,4-butanetriol déshydrogénase dépendante du NADPH,
- un gène *adhP* ou *yqhD* endogène codant une 1,4-butanediol déshydrogénase dépendante du NADPH, et
- l'une quelconque de leurs combinaisons.

**4.** Microorganisme selon l'une quelconque des revendications 1 à 3, comprenant, en tant que modification génétique supplémentaire, une surexpression d'au moins un gène impliqué dans le transport de la vitamine B12, de préférence choisi parmi :

- un gène *btuB* endogène codant une porine de membrane extérieure de vitamine B12,
- un gène *btuC* endogène codant une sous-unité de membrane du transporteur ABC de vitamine B12,
- un gène *btuD* endogène codant une protéine se liant à l'ATP au la GTP du transporteur de vitamine B12, et
- l'une quelconque de leurs combinaisons.

**5.** Microorganisme selon l'une quelconque des revendications 1 à 4, comprenant, en tant que modification génétique supplémentaire, une surexpression d'au moins un gène impliqué dans l'utilisation de la coenzyme B12 et/ou dans la réactivation de la 1,2,4-butanetriol déshydratase, de préférence choisi parmi :

- un gène *btuR* endogène codant une adénosyle transférase de cobalamine,
- au moins un gène hétérologue choisi parmi *dhaB4, orf2b, ddrA, ddrB, orfZ* et *orfX* codant une 1,2,4-butanetriol déshydratase réactivase, et
- l'une quelconque de leurs combinaisons.

**6.** Microorganisme selon l'une quelconque des revendications 1 à 5, comprenant une modification génétique supplémentaire d'au moins un gène impliqué dans la production de NADPH en tant que source de pouvoir réducteur, ladite modification étant de préférence choisie parmi :

- la surexpression d'un gène ou opéron *pntAB* endogène codant une transhydrogénase liée à la membrane,
- la délétion d'un gène *udhA* endogène codant une transhydrogénase soluble,
- la surexpression d'un gène *gapA* endogène ou d'un gène *gapN* hétérologue codant une glycéraldéhyde 3-phosphate déshydrogénase générant du NADPH,
- la délétion d'un gène *pgi* endogène codant une phosphoglucose isomérase,
- la délétion d'un gène *pfkA* endogène codant une phosphofructokinase,
- la surexpression d'un gène *zwf* endogène codant une glucose-6-phosphate déshydrogénase,
- la surexpression d'un gène *lpd* mutant endogène codant une lipoamide déshydrogénase capable de générer du NADPH,
- la surexpression d'un gène *yjeF* endogène codant une enzyme de réparation d'hydrate de NAD(P)H bifonctionnelle, et
- l'une quelconque de leurs combinaisons.

**7.** Microorganisme selon l'une quelconque des revendications 1 à 6, dans lequel la voie métabolique dudit microorganisme pour la conversion exclusive de xylose en 1,4-butanediol comprend au moins les étapes de :

- élimination d'un carbone sur le squelette de xylose, ce qui donne ainsi un métabolite intermédiaire ayant un squelette aliphatique de 4 carbones, et
- élimination d'un hydroxyle sur ledit métabolite intermédiaire.

**8.** Procédé pour la production optimisée de 1,4-butanediol par fermentation, comprenant :

a) la culture d'un microorganisme génétiquement modifié tel que défini selon l'une quelconque des revendications 1 à 7 dans un milieu de culture comprenant du xylose et une source de carbone autre que le xylose, dans des conditions de fermentation permettant la conversion de xylose en 1,4-butanediol, et
b) la récupération du 1,4-butanediol à partir dudit milieu de culture.

**9.** Procédé selon la revendication 8, dans lequel ladite source de carbone est telle que définie dans la revendication 2.

**10.** Procédé selon la revendication 9, dans lequel ladite source de carbone est choisie dans le groupe constitué par le glycérol, le glucose, le galactose, le fructose, le lactose, le maltose, le saccharose, et l'une quelconque de leurs combinaisons.

FIGURE 1

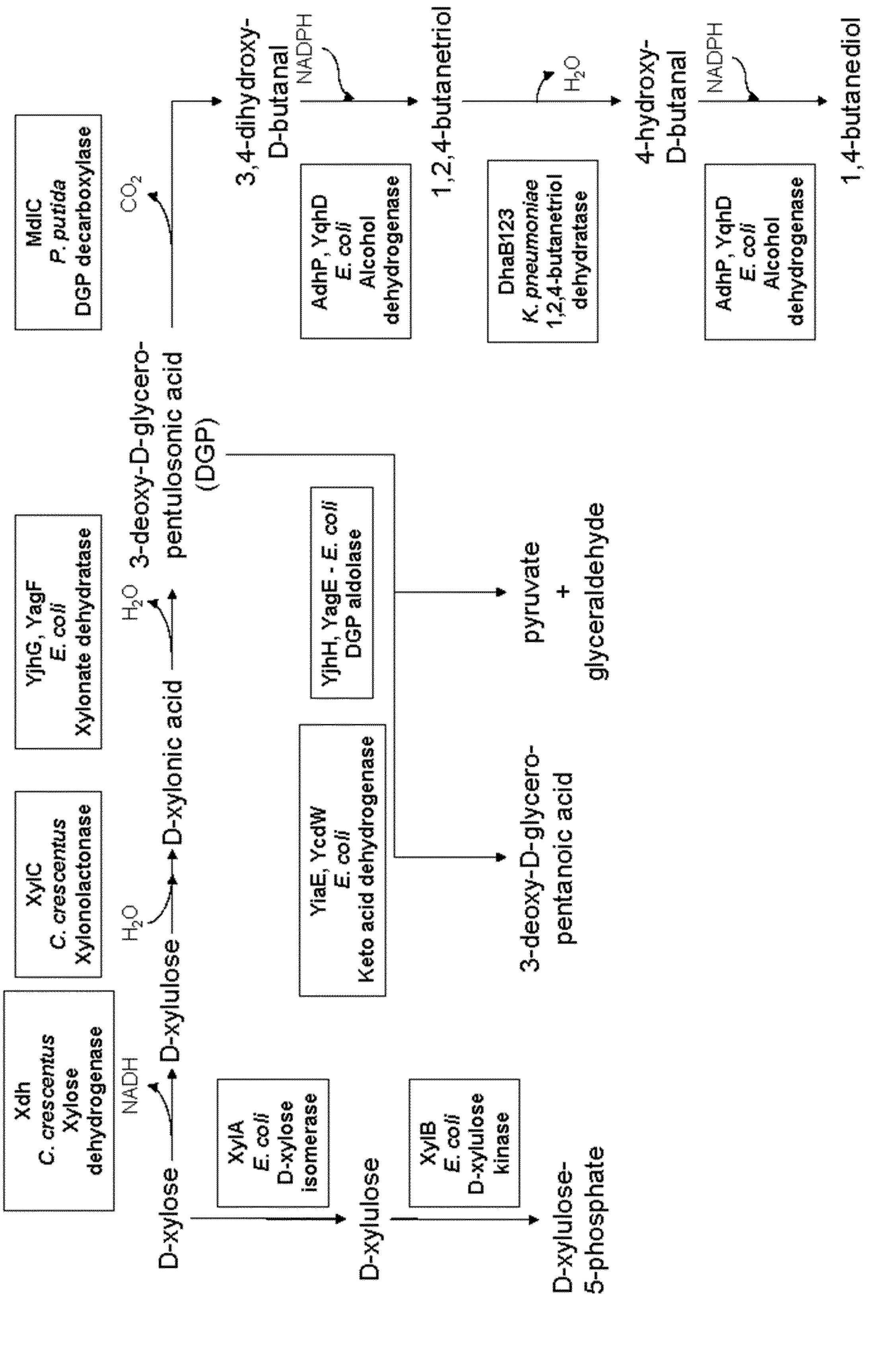
Xdh
C. crescentus
Xylose
dehydrogenase
XylC
C. crescentus
Xylonolactonase
YjhG, YagF
E. coli
Xylonate dehydratase
MdlC
P. putida
DGP decarboxylase
D-xylose
NADH
D-xylulose
$H_2O$
D-xylonic acid
$H_2O$
3-deoxy-D-glycero-
pentulosonic acid
(DGP)
$CO_2$
3,4-dihydroxy-
D-butanal
NADPH
1,2,4-butanetriol
$H_2O$
4-hydroxy-
D-butanal
NADPH
1,4-butanediol
XylA
E. coli
D-xylose
isomerase
YiaE, YcdW
E. coli
Keto acid dehydrogenase
YjhH, YagE - E. coli
DGP aldolase
AdhP, YqhD
E. coli
Alcohol
dehydrogenase
DhaB123
K. pneumoniae
1,2,4-butanetriol
dehydratase
AdhP, YqhD
E. coli
Alcohol
dehydrogenase
D-xylulose
XylB
E. coli
D-xylulose
kinase
D-xylulose-
5-phosphate
3-deoxy-D-glycero-
pentanoic acid
pyruvate
+
glyceraldehyde

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012177943 A1
- US 20130203141 A
- WO 2008091288 A
- WO 2005073364 A
- WO 2008116852 A
- WO 2011137192 A
- WO 2012004247 A
- US 2011207189 A
- US 2008176302 A
- US 8088620 B
- WO 2012055798 A
- EP 14305691 A
- WO 2012055798 A1
- WO 2005047498 A
- WO 2010141780 A
- US 20110003355 A
- WO 2013183592 A
- WO 2014152665 A
- WO 2013001055 A
- WO 20070770441 A
- EP 2532751 A


### Non-patent literature cited in the description


- **ALTSCHUL S ; GISH W ; MILLER W ; MYERS E ; LIPMAN DJ.** *J. Mol. Biol,* 1990, vol. 215 (3), 403-410
- **ANDERSON.** *Proc. Natl. Acad. Sci. USA.,* 1946, vol. 32, 120-128
- **BOCANEGRA J ; SCRUTTON N ; PERHAM R.** *Biochemistry,* 1993, vol. 32 (11), 2737-2740
- **CARRIER T ; KEASLING J.** *Biotechnol Prog.,* 1999, vol. 15 (1), 58-64
- **DATSENKO KA ; WANNER BL.** *Proc Natl Acad Sci U S A.,* 2000, vol. 97, 6640-6645
- **HOGEMA et al.** *Molecular microbiology,* 1997, 24-857, 867
- **KIM, J. H. et al.** *Appl. Microbiol. Biotechnol.,* 2010, vol. 88, 1077-1085
- **KOVACH ME ; ELZER PH ; HILL DS ; ROBERTSON GT ; FARRIS MA ; ROOP RM ; PETERSON KM.** *Gene,* 1995, vol. 166 (1), 175-6
- **LEE S ; MCCORMICK M ; LIPPARD S ; CHO U.** *Nature,* 2013, vol. 494, 380-384
- **LERNER C.G. ; INOUYE M.** *Nucleic Acids Research,* 1990, vol. 18 (15), 4631
- **LIM S ; JUNG Y ; SHIN H ; LEE Y.** *J Biosci Bioeng.,* 2002, vol. 93 (6), 543-549
- **MARBAIX A ; NOEL G ; DETROUX A ; VERTOMMEN D ; SCHAFTINGEN E ; LINSTER C.** *J Biol Chem.,* 2011, vol. 286 (48), 41246-41252
- **SALIS H.** *Methods Enzymol.,* 2011, vol. 498, 19-42
- **SAMPAT, B.G.** *Chemical Weekly Special Report,* 18 January 2011, 205-211
- **SCHMID K ; SCHUPFNER M ; SCHMITT R.** *J. Bacteriol.,* 1982, vol. 151, 68-76
- **SEGEL IH.** Enzyme kinetics. John Wiley & Sons, 1993, 44-54, 100-112

SEQUENCE LISTING

<110> METABOLIC EXPLORER

<120> Modified microorganism for optimized production of 1,4-butanediol

<130> B71817D34583

<160> 80

<170> PatentIn version 3.5

<210> 1
<211> 747

<212> DNA
<213> Caulobacter crescentus

<220>
<221> misc_feature
<223> xdh

<400> 1

```
atgtcctcag ccatctatcc cagcctgaag ggcaagcgcg tcgtcatcac cggcggcggc      60
tcgggcatcg gggccggcct caccgccggc ttcgcccgtc agggcgcgga ggtgatcttc     120
ctcgacatcg ccgacgagga ctccagggct cttgaggccg agctggccgg ctcgccgatc     180
ccgccggtct acaagcgctg cgacctgatg aacctcgagg cgatcaaggc ggtcttcgcc     240
gagatcggcg acgtcgacgt gctggtcaac aacgccggca atgacgaccg ccacaagctg     300
gccgacgtga ccggcgccta ttgggacgag cggatcaacg tcaacctgcg ccacatgctg     360
ttctgcaccc aggccgtcgc gccgggcatg aagaagcgtg gcggcggggc ggtgatcaac     420
ttcggttcga tcagctggca cctggggctt gaggacctcg tcctctacga aaccgccaag     480
gccggcatcg aaggcatgac ccgcgcgctg gcccgggagc tgggtcccga cgacatccgc     540
gtcacctgcg tggtgccggg caacgtcaag accaagcgcc aggagaagtg gtacacgccc     600
gaaggcgagg cccagatcgt ggcggcccaa tgcctgaagg gccgcatcgt cccggagaac     660
gtcgccgcgc tggtgctgtt cctggcctcg gatgacgcgt cgctctgcac cggccacgaa     720
tactggatcg acgccggctg gcgttga                                         747
```

<210> 2
<211> 870
<212> DNA
<213> Caulobacter crescentus

<220>
<221> misc_feature
<223> xylC

<400> 2

```
atgaccgctc aagtcacttg cgtatgggat ctgaaggcca cgttgggcga aggcccgatc      60
```

```
tggcatggcg acaccctgtg gttcgtcgac atcaagcagc gtaaaatcca caactaccac      120
cccgccaccg gcgagcgctt cagcttcgac gcgccggatc aggtgacctt cctcgcgccg      180
atcgtcggcg cgaccggctt tgtcgtcggt ctgaagaccg ggattcaccg cttccacccg      240
gccacgggct tcagcctgct gctcgaggtc gaggacgcgg cgctgaacaa ccgccccaac      300
gacgccacgg tcgacgcgca aggccgtctg tggttcggca ccatgcacga cggggaagag      360
aacaatagcg gctcgctcta tcggatggac ctcaccggcg tcgcccggat ggaccgcgac      420
atctgcatca ccaacggccc gtgcgtctcg cccgacggca agaccttcta ccacaccgac      480
accctggaaa agacgatcta cgccttcgac ctggccgagg acggcctgct gtcgaacaag      540
cgcgtcttcg tgcagttcgc cctgggcgac gatgtctatc cggacggttc ggtcgtcgat      600
tccgaaggct atctgtggac cgccctgtgg ggcggtttcg gcgcggtccg cttctcgccg      660
caaggcgacg ccgtgacgcg catcgaactg cccgccccca acgtcaccaa gccctgcttc      720
ggcgggcctg acctgaagac cctctatttc accaccgccc gcaagggcct gagcgacgag      780
accctggccc agtacccgct ggccggcggt gtgttcgccg ttccggtcga tgtggccggc      840
caaccccagc atgaggtccg ccttgtctaa                                        870
```

<210> 3
<211> 1587
<212> DNA
<213> Pseudomonas putida

<220>
<221> misc_feature
<223> mdlC

<400> 3

```
atggcttcgg tacacggcac cacatacgaa ctcttgcgac gtcaaggcat cgatacggtc      60
ttcggcaatc ctggctcgaa cgagctcccg tttttgaagg actttccaga ggactttcga     120
tacatcctgg ctttgcagga agcgtgtgtg gtgggcattg cagacggcta tgcgcaagcc     180
agtcggaagc cggctttcat taacctgcat tctgctgctg gtaccggcaa tgctatgggt     240
gcactcagta acgcctggaa ctcacattcc ccgctgatcg tcactgccgg ccagcagacc     300
agggcgatga ttggcgttga agctctgctg accaacgtcg atgccgccaa cctgccacga     360
ccacttgtca aatggagcta cgagcccgca agcgcagcag aagtccctca tgcgatgagc     420
agggctatcc atatggcaag catggcgcca caaggccctg tctatctttc ggtgccatat     480
gacgattggg ataaggatgc tgatcctcag tcccaccacc tttttgatcg ccatgtcagt     540
tcatcagtac gcctgaacga ccaggatctc gatattctgg tgaaagctct caacagcgca     600
tccaacccgg cgatcgtcct gggcccggac gtcgacgcag caaatgcgaa cgcagactgc     660
gtcatgttgg ccgaacgcct caaagctccg gtttgggttg cgccatccgc tccacgctgc     720
ccattcccta cccgtcatcc ttgcttccgt ggattgatgc cagctggcat cgcagcgatt     780
tctcagctgc tcgaaggtca cgatgtggtt ttggtaatcg gcgctccagt gttccgttac     840
caccaatacg acccaggtca atatctcaaa cctggcacgc gattgatttc ggtgacctgc     900
gacccgctcg aagctgcacg cgcgccaatg ggcgatgcga tcgtggcaga cattggtgcg     960
atggctagcg ctcttgccaa cttggttgaa gagagcagcc gccagctccc aactgcagct    1020
ccggaacccg cgaaggttga ccaagacgct ggccgacttc acccagagac agtgttcgac    1080
acactgaacg acatggcccc ggagaatgcg atttacctga acgagtcgac ttcaacgacc    1140
gcccaaatgt ggcagcgcct gaacatgcgc aaccctggta gctactactt ctgtgcagct    1200
ggcggactgg gcttcgccct gcctgcagca attggcgttc aactcgcaga acccgagcga    1260
caagtcatcg ccgtcattgg cgacggatcg gcgaactaca gcattagtgc gttgtggact    1320
gcagctcagt acaacatccc cactatcttc gtgatcatga acaacggcac ctacggtgcg    1380
ttgcgatggt ttgccggcgt tctcgaagca gaaaacgttc ctgggctgga tgtgccaggg    1440
atcgacttcc gcgcactcgc caagggctat ggtgtccaag cgctgaaagc cgacaacctt    1500
gagcagctca agggttcgct acaagaagcg ctttctgcca aaggcccggt acttatcgaa    1560
gtaagcaccg taagcccggt gaagtga                                        1587
```

<210> 4
<211> 2693
<212> DNA
<213> Klebsiella pneumoniae

<220>

<221> misc_feature
<223> dhaB123

<400> 4

```
atgaaaagat caaaacgatt tgcagtactg gcccagcgcc ccgtcaatca ggacgggctg      60
attggcgagt ggcctgaaga ggggctgatc gccatggaca gcccctttga cccggtctct     120
tcagtaaaag tggacaacgg tctgatcgtc gaactggacg gcaaacgccg ggaccagttt     180
gacatgatcg accgatttat cgccgattac gcgatcaacg ttgagcgcac agagcaggca     240
atgcgcctgg aggcggtgga aatagcccgt atgctggtgg atattcacgt cagccgggag     300
gagatcattg ccatcactac cgccatcacg ccggccaaag cggtcgaggt gatggcgcag     360
atgaacgtgg tggagatgat gatggcgctg cagaagatgc gtgcccgccg gaccccctcc     420
aaccagtgcc acgtcaccaa tctcaaagat aatccggtgc agattgccgc tgacgccgcc     480
gaggccggga tccgcggctt ctcagaacag gagaccacgg tcggtatcgc gcgctacgcg     540
ccgtttaacg ccctggcgct gttggtcggt tcgcagtgcg gccgccccgg cgtgttgacg     600
```

cagtgctcgg tggaagaggc caccgagctg gagctgggca tgcgtggctt aaccagctac 660

gccgagacgg tgtcggtcta cggcaccgaa gcggtattta ccgacggcga tgatacgccg 720

tggtcaaagg cgttcctcgc ctcggcctac gcctcccgcg ggttgaaaat gcgctacacc 780

tccggcaccg gatccgaagc gctgatgggc tattcggaga gcaagtcgat gctctacctc 840

gaatcgcgct gcatcttcat tactaaaggc gccggggttc agggactgca aaacggcgcg 900

gtgagctgta tcggcatgac cggcgctgtg ccgtcgggca ttcgggcggt gctggcggaa 960

aacctgatcg cctctatgct cgacctcgaa gtggcgtccg ccaacgacca gactttctcc 1020

cactcggata ttcgccgcac cgcgcgcacc ctgatgcaga tgctgccggg caccgacttt 1080

attttctccg gctacagcgc ggtgccgaac tacgacaaca tgttcgccgg ctcgaacttc 1140

gatgcggaag attttgatga ttacaacatc ctgcagcgtg acctgatggt tgacggcggc 1200

ctgcgtccgg tgaccgaggc ggaaaccatt gccattcgcc agaaagcggc gcgggcgatc 1260

caggcggttt tccgcgagct ggggctgccg ccaatcgccg acgaggaggt ggaggccgcc 1320

acctacgcgc acggcagcaa cgagatgccg ccgcgtaacg tggtggagga tctgagtgcg 1380

gtggaagaga tgatgaagcg caacatcacc ggcctcgata ttgtcggcgc gctgagccgc 1440

agcggctttg aggatatcgc cagcaatatt ctcaatatgc tgcgccagcg ggtcaccggc 1500

gattacctgc agacctcggc cattctcgat cggcagttcg aggtggtgag tgcggtcaac 1560

gacatcaatg actatcaggg gccgggcacc ggctatcgca tctctgccga acgctgggcg 1620

gagatcaaaa atattccggg cgtggttcag cccgacacca ttgaataagg cggtattcct 1680

gtgcaacaga caacccaaat tcagccctct tttaccctga aaacccgcga gggcggggta 1740

gcttctgccg atgaacgcgc cgatgaagtg gtgatcggcg tcggccctgc cttcgataaa 1800

caccagcatc acactctgat cgatatgccc catggcgcga tcctcaaaga gctgattgcc 1860

ggggtggaag aagaggggct tcacgcccgg gtggtgcgca ttctgcgcac gtccgacgtc 1920

tcctttatgg cctgggatgc ggccaacctg agcggctcgg ggatcggcat cggtatccag 1980

tcgaagggga ccacggtcat ccatcagcgc gatctgctgc cgctcagcaa cctggagctg 2040

ttctcccagg cgccgctgct gacgctggag acctaccggc agattggcaa aaacgctgcg 2100

cgctatgcgc gcaaagagtc accttcgccg gtgccggtgg tgaacgatca gatggtgcgg 2160

ccgaaattta tggccaaagc cgcgctattt catatcaaag agaccaaaca tgtggtgcag 2220

gacgccgagc ccgtcaccct gcacatcgac ttagtaaggg agtgaccatg agcgagaaaa 2280

ccatgcgcgt gcaggattat ccgttagcca cccgctgccc ggagcatatc ctgacgccta 2340

ccggcaaacc attgaccgat attaccctcg agaaggtgct ctctggcgag gtgggcccgc 2400

aggatgtgcg gatctcccgc cagacccttg agtaccaggc gcagattgcc gagcagatgc 2460

agcgccatgc ggtggcgcgc aatttccgcc gcgcggcgga gcttatcgcc attcctgacg 2520

agcgcattct ggctatctat aacgcgctgc gcccgttccg ctcctcgcag gcggagctgc 2580
tggcgatcgc cgacgagctg gagcacacct ggcatgcgac agtgaatgcc gcctttgtcc 2640
gggagtcggc ggaagtgtat cagcagcggc ataagctgcg taaaggaagc taa 2693

<210> 5
<211> 1968
<212> DNA
<213> Escherichia coli

<220>
<221> misc_feature
<223> yjhG

<400> 5

atgtctgttc gcaatatttt tgctgacgag agccacgata tttacaccgt cagaacgcac 60
gccgatggcc cggacggcga actcccatta accgcagaga tgcttatcaa ccgcccgagc 120
ggggatctgt tcggtatgac catgaatgcc ggaatgggtt ggtctccgga cgagctggat 180
cgggacggta ttttactgct cagtacactc ggtggcttac gcggcgcaga cggtaaaccc 240
gtggcgctgg cgttgcacca ggggcattac gaactggaca tccagatgaa agcggcggcc 300
gaggttatta aagccaacca tgccctgccc tatgccgtgt acgtctccga tccttgtgac 360
gggcgtactc agggtacaac ggggatgttt gattcgctac cataccgaaa tgacgcatcg 420
atggtaatgc gccgccttat tcgctctctg cccgacgcga aagcagttat tggtgtggcg 480
agttgcgata aggggcttcc ggccaccatg atggcactcg ccgcgcagca caacatcgca 540
accgtgctgg tccccggcgg cgcgacgctg cccgcaaagg atggagaaga caacggcaag 600
gtgcaaacca ttggcgcacg cttcgccaat ggcgaattat ctctacagga cgcacgccgt 660
gcgggctgta aagcctgtgc ctcttccggc ggcggctgtc aatttttggg cactgccggg 720
acatctcagg tggtggccga aggattggga ctggcaatcc cacattcagc cctggcccct 780
tccggtgagc ctgtgtggcg ggagatcgcc agagcttccg cgcgagctgc gctgaacctg 840
agtcaaaaag gcatcaccac ccgggaaatt ctcaccgata aagcgataga gaatgcgatg 900
acggtccatg ccgcgttcgg tggttcaaca aacctgctgt tacacatccc ggcaattgct 960
caccaggcag gttgccatat cccgaccgtt gatgactgga tccgcatcaa caagcgcgtg 1020
ccccgactgg tgagcgtact gcctaatggc ccggtttatc atccaacggt caatgccttt 1080
atggcaggtg gtgtgccgga agtcatgttg catctgcgca gcctcggatt gttgcatgaa 1140
gacgttatga cggttaccgg cagcacgctg aaagaaaacc tcgactggtg ggagcactcc 1200
gaacggcgtc agcggttcaa gcaactcctg ctcgatcagg aacaaatcaa cgctgacgaa 1260
gtgatcatgt ctccgcagca agcaaaagcg cgcggattaa cctcaactat caccttcccg 1320

```
gtgggcaata ttgcgccaga aggttcggtg atcaaatcca ccgccattga cccctcgatg     1380
attgatgagc aaggtatcta ttaccataaa ggtgtggcga aggtttatct gtccgagaaa     1440
agtgcgattt acgatatcaa acatgacaag atcaaggcgg gcgatattct ggtcattatt     1500
ggcgttggac cttcaggtac agggatggaa gaaacctacc aggttaccag tgccctgaag     1560
catctgtcat acggtaagca tgtttcgtta atcaccgatg cacgtttctc gggcgtttct     1620
actggcgcgt gcatcggcca tgtggggcca gaagcgctgg ccggaggccc catcggtaaa     1680
ttacgcaccg gggatttaat tgaaattaaa attgattgtc gcgagcttca cggcgaagtc     1740
aatttcctcg gaacccgtag cgatgaacaa ttaccttcac aggaggaggc aactgcaata     1800
ttaaatgcca gacccagcca tcaggattta cttcccgatc ctgaattgcc agatgatacc     1860
cggctatggg caatgcttca ggccgtgagt ggtgggacat ggaccggttg tatttatgat     1920
gtaaacaaaa ttggcgcggc tttgcgcgat tttatgaata aaaactga                   1968
```

<210> 6
<211> 1383
<212> DNA
<213> Escherichia coli

<220>
<221> misc_feature
<223> yagF

<400> 6

```
atgacgcaat taaccatgaa agacaaaatt ggctacgggc tgggagacac cgcctgcggc      60
ttcgtctggc aggccacgat gttcctgctg gcctatttct acaccgacgt cttcggcctg     120
tcggcgggga ttatgggcac gctgtttttg gtctcccgcg tgctcgacgc cgtcaccgac     180
ccgctgatgg ggctgctggt agaccgcacc cgcacgcggc acggccagtt ccgcccgttc     240
ctgctgtggg gggccatccc gttcggcatc gtctgcgtgc tgaccttcta cacgccggac     300
ttctccgcac agggcaagat catctacgcc tgcgtgacct acattctcct gaccctggtc     360
tacaccttcg ttaacgtgcc gtactgcgcc atgccgggcg tcatcaccgc cgacccgaaa     420
gagcgtcacg ccctgcagtc ctggcgcttc ttcctggcgg cggcgggctc gctcgctatc     480
agcggcatcg cgctgccgct ggtgagcatc atcggcaaag gggacgagca ggtgggctac     540
ttcggcgcca tgtgcgtgct ggggctgagc ggcgtggtgc tgctctacgt ctgcttcttc     600
acgaccaaag agcgctacac ctttgaggtg cagccgggct cgtcggtggc gaaagacctt     660
aagctgctgc tgggcaacag ccagtggcgc atcatgtgcg cgttcaagat gatggcgacc     720
tgctccaacg tggtgcgcgg cggggcgacg ctctacttcg tgaaatacgt gatggatcac     780
ccggagttgg cgacccagtt tttactttac ggcagcctcg ccaccatgtt cggctcgctt     840
tgctcctcac gcctgctggg ccgcttcgac cgcgtcaccg ccttcaagtg gatcatcgtc     900

gcctactcgc tgatcagcct gctgattttc gtcaccccgg cggagcacat cgcgctcatt     960
tttgccctca acatcctgtt cctgttcgtc tttaatacca ccacgccgct gcagtggctg    1020
atggcttctg acgtggtgga ctacgaggag agccgcagcg gtcgccgcct cgacgggctg    1080
gtgttctcca cctacctgtt cagcctgaag attggcctgg cgattggcgg ggcggtggtg    1140
ggctggatcc tggcgtacgt caactattcc gccagcagca gcgtgcagcc ggttgaggtg    1200
ctcaccacca tcaaaattct gttctgcgtg gtgccggtgg tgctctacgc gggcatgttc    1260
atcatgctgt cgctctacaa gctcaccgat gcccgcgtgg aggccatcag ccggcagctg    1320
attaagcacc gcgcggcgca gggcgaggcc gttcccgacg ccgcgacagc cgcatcccat    1380
taa                                                                  1383
```

<210> 7
<211> 1041
<212> DNA
<213> Escherichia coli

<220>
<221> misc_feature
<223> adhP

<400> 7

```
atgcagaaca tcatccgaaa aggaggaact atgaaggctg cagttgttac gaaggatcat     60
catgttgacg ttacgtataa aacactgcgc tcactgaaac atggcgaagc cctgctgaaa    120
atggagtgtt gtggtgtatg tcataccgat cttcatgtta agaatggcga ttttggtgac    180
aaaaccggcg taattctggg ccatgaaggc atcggtgtgg tggcagaagt gggtccaggt    240
gtcacctcat taaaaccagg cgatcgtgcc agcgtggcgt ggttctacga aggatgcggt    300
cattgcgaat actgtaacag tggtaacgaa acgctctgcc gttcagttaa aaatgccgga    360
tacagcgttg atggcgggat ggcggaagag tgcatcgtgg tcgccgatta cgcggtaaaa    420
gtgccagatg gtctggactc ggcggcggcc agcagcatta cctgtgcggg agtcaccacc    480
tacaaagccg ttaagctgtc aaaaattcgt ccagggcagt ggattgctat ctacggtctt    540
ggcggtctgg gtaacctcgc cctgcaatac gcgaagaatg tctttaacgc caaagtgatc    600
gccattgatg tcaatgatga gcagttaaaa ctggcaaccg aaatgggcgc agatttagcg    660
attaactcac acaccgaaga cgccgccaaa attgtgcagg agaaaactgg tggcgctcac    720
gctgcggtgg taacagcggt agctaaagct gcgtttaact cggcagttga tgctgtccgt    780
gcaggcggtc gtgttgtggc tgtcggtcta ccgccggagt ctatgagcct ggatatccca    840
cgtcttgtgc tggatggtat tgaagtggtc ggttcgctgg tcggcacgcg ccaggattta    900
actgaagcct tccagtttgc cgccgaaggt aaagtggtgc cgaaagtcgc cctgcgtccg    960

ttagcggaca tcaacaccat ctttactgag atggaagaag gcaaaatccg tggccgcatg   1020
gtgattgatt tccgtcacta a                                             1041
```

<210> 8
<211> 1164
<212> DNA
<213> Escherichia coli

<220>
<221> misc_feature
<223> yqhD

<400> 8

```
atgaacaact ttaatctgca caccccaacc cgcattctgt ttggtaaagg cgcaatcgct      60
ggtttacgcg aacaaattcc tcacgatgct cgcgtattga ttacctacgg cggcggcagc     120
gtgaaaaaaa ccggcgttct cgatcaagtt ctggatgccc tgaaaggcat ggacgtgctg     180
gaatttggcg gtattgagcc aaacccggct tatgaaacgc tgatgaacgc cgtgaaactg     240
gttcgcgaac agaaagtgac tttcctgctg gcggttggcg gcggttctgt actggacggc     300
accaaattta tcgccgcagc ggctaactat ccggaaaata tcgatccgtg gcacattctg     360
caaacgggcg gtaaagagat taaaagcgcc atcccgatgg gctgtgtgct gacgctgcca     420
gcaaccggtt cagaatccaa cgcaggcgcg gtgatctccc gtaaaaccac aggcgacaag     480
caggcgttcc attctgccca tgttcagccg gtatttgccg tgctcgatcc ggtttatacc     540
tacaccctgc cgccgcgtca ggtggctaac ggcgtagtgg acgcctttgt acacaccgtg     600
gaacagtatg ttaccaaacc ggttgatgcc aaaattcagg accgtttcgc agaaggcatt     660
ttgctgacgc taatcgaaga tggtccgaaa gccctgaaag agccagaaaa ctacgatgtg     720
cgcgccaacg tcatgtgggc ggcgactcag gcgctgaacg gtttgattgg cgctggcgta     780
ccgcaggact gggcaacgca tatgctgggc cacgaactga ctgcgatgca cggtctggat     840
cacgcgcaaa cactggctat cgtcctgcct gcactgtgga atgaaaaacg cgataccaag     900
cgcgctaagc tgctgcaata tgctgaacgc gtctggaaca tcactgaagg ttccgatgat     960
gagcgtattg acgccgcgat tgccgcaacc cgcaatttct ttgagcaatt aggcgtgccg    1020
acccacctct ccgactacgg tctggacggc agctccatcc cggctttgct gaaaaaactg    1080
gaagagcacg gcatgaccca actgggcgaa aatcatgaca ttacgttgga tgtcagccgc    1140
cgtatatacg aagccgcccg ctaa                                           1164
```

<210> 9
<211> 1323
<212> DNA
<213> Escherichia coli

<220>
<221> misc_feature
<223> xylA

<400> 9

```
atgcaagcct attttgacca gctcgatcgc gttcgttatg aaggctcaaa atcctcaaac      60
ccgttagcat tccgtcacta caatcccgac gaactggtgt tgggtaagcg tatggaagag     120
cacttgcgtt ttgccgcctg ctactggcac accttctgct ggaacggggc ggatatgttt     180
ggtgtggggg cgtttaatcg tccgtggcag cagcctggtg aggcactggc gttggcgaag     240
cgtaaagcag atgtcgcatt tgagtttttc cacaagttac atgtgccatt ttattgcttc     300
cacgatgtgg atgtttcccc tgagggcgcg tcgttaaaag agtacatcaa taattttgcg     360
caaatggttg atgtcctggc aggcaagcaa gaagagagcg gcgtgaagct gctgtgggga     420
acggccaact gctttacaaa ccctcgctac ggcgcgggtg cggcgacgaa cccagatcct     480
gaagtcttca gctgggcggc aacgcaagtt gttacagcga tggaagcaac ccataaattg     540
ggcggtgaaa actatgtcct gtggggcggt cgtgaaggtt acgaaacgct gttaaatacc     600
gacttgcgtc aggagcgtga acaactgggc cgctttatgc agatggtggt tgagcataaa     660
cataaaatcg gtttccaggg cacgttgctt atcgaaccga aaccgcaaga accgaccaaa     720
catcaatatg attacgatgc cgcgacggtc tatggcttcc tgaaacagtt tggtctggaa     780
aaagagatta aactgaacat tgaagctaac cacgcgacgc tggcaggtca ctctttccat     840
catgaaatag ccaccgccat tgcgcttggc ctgttcggtt ctgtcgacgc caaccgtggc     900
gatgcgcaac tgggctggga caccgaccag ttcccgaaca gtgtggaaga gaatgcgctg     960
gtgatgtatg aaattctcaa agcaggcggt ttcaccaccg gtggtctgaa cttcgatgcc    1020
aaagtacgtc gtcaaagtac tgataaatat gatctgtttt acggtcatat cggcgcgatg    1080
gatacgatgg cactggcgct gaaaattgca gcgcgcatga ttgaagatgg cgagctggat    1140
aaacgcatcg cgcagcgtta ttccggctgg aatagcgaat tgggccagca aatcctgaaa    1200
ggccaaatgt cactggcaga tttagccaaa tatgctcagg aacatcattt gtctccggtg    1260
catcagagtg gtcgccagga acaactggaa aatctggtaa accattatct gttcgacaaa    1320
taa                                                                  1323
```

<210> 10
<211> 1455
<212> DNA
<213> Escherichia coli

<220>
<221> misc_feature
<223> xylB

<400> 10

```
atgtatatcg ggatagatct tggcacctcg ggcgtaaaag ttattttgct caacgagcag      60
ggtgaggtgg ttgctgcgca aacggaaaag ctgaccgttt cgcgcccgca tccactctgg     120
tcggaacaag acccggaaca gtggtggcag gcaactgatc gcgcaatgaa agctctgggc     180
gatcagcatt ctctgcagga cgttaaagca ttgggtattg ccggccagat gcacggagca     240
accttgctgg atgctcagca acgggtgtta cgccctgcca ttttgtggaa cgacgggcgc     300
tgtgcgcaag agtgcacttt gctggaagcg cgagttccgc aatcgcgggt gattaccggc     360
aacctgatga tgcccggatt tactgcgcct aaattgctat gggttcagcg gcatgagccg     420
gagatattcc gtcaaatcga caaagtatta ttaccgaaag attacttgcg tctgcgtatg     480
acgggggagt ttgccagcga tatgtctgac gcagctggca ccatgtggct ggatgtcgca     540
aagcgtgact ggagtgacgt catgctgcag gcttgcgact tatctcgtga ccagatgccc     600
gcattatacg aaggcagcga aattactggt gctttgttac ctgaagttgc gaaagcgtgg     660
ggtatggcga cggtgccagt tgtcgcaggc ggtggcgaca atgcagctgg tgcagttggt     720
gtgggaatgg ttgatgctaa tcaggcaatg ttatcgctgg ggacgtcggg ggtctatttt     780
gctgtcagcg aagggttctt aagcaagcca gaaagcgccg tacatagctt ttgccatgcg     840
ctaccgcaac gttggcattt aatgtctgtg atgctgagtg cagcgtcgtg tctggattgg     900
gccgcgaaat taaccggcct gagcaatgtc ccagctttaa tcgctgcagc tcaacaggct     960
gatgaaagtg ccgagccagt ttggtttctg ccttatcttt ccggcgagcg tacgccacac    1020
aataatcccc aggcgaaggg ggttttcttt ggtttgactc atcaacatgg ccccaatgaa    1080
ctggcgcgag cagtgctgga aggcgtgggt tatgcgctgg cagatggcat ggatgtcgtg    1140
catgcctgcg gtattaaacc gcaaagtgtt acgttgattg gggcggggc gcgtagtgag     1200
tactggcgtc agatgctggc ggatatcagc ggtcagcagc tcgattaccg tacggggggg    1260
gatgtggggc cagcactggg cgcagcaagg ctggcgcaga tcgcggcgaa tccagagaaa    1320
tcgctcattg aattgttgcc gcaactaccg ttagaacagt cgcatctacc agatgcgcag    1380
cgttatgccg cttatcagcc acgacgagaa acgttccgtc gcctctatca gcaacttctg    1440
ccattaatgg cgtaa                                                     1455
```

<210> 11
<211> 906
<212> DNA
<213> Escherichia coli

<220>
<221> misc_feature
<223> yjhH

<400> 11

atgaaaaaat tcagcggcat tattccaccg gtatccagca cgtttcatcg tgacggaacc 60

cttgataaaa aggcaatgcg cgaagttgcc gacttcctga ttaataaagg ggtcgacggg 120

ctgttttatc tgggtaccgg tggtgaattt agccaaatga atacagccca gcgcatggca 180

ctcgccgaag aagctgtaac cattgtcgac gggcgagtgc cggtattgat tggcgtcggt 240

tccccttcca ctgacgaagc ggtcaaactg gcgcagcatg cgcaagccta cggcgctgat 300

ggtatcgtcg ccatcaaccc ctactactgg aaagtcgcac cacgaaatct tgacgactat 360

taccagcaga tcgcccgtag cgtcacccta ccggtgatcc tgtacaactt tccggatctg 420

acgggtcagg acttaacccc ggaaaccgtg acgcgtctgg ctctgcaaaa cgagaatatc 480

gttggcatca aagacaccat cgacagcgtt ggtcacttgc gtacgatgat caacacagtt 540

aagtcggtac gcccgtcgtt ttcggtattc tgcggttacg atgatcattt gctgaatacg 600

atgctgctgg gcggcgacgg tgcgataacc gccagcgcta actttgctcc ggaactctcc 660

gtcggcatct accgcgcctg gcgtgaaggc gatctggcga ccgctgcgac gctgaataaa 720

aaactactac aactgcccgc tatttacgcc ctcgaaacac cgtttgtctc actgatcaaa 780

tacagcatgc agtgtgtcgg gctgcctgta gagacatatt gcttaccacc gattcttgaa 840

gcatctgaag aagcaaaaga taaagtccac gtgctgctta ccgcgcaggg cattttacca 900

gtctga 906

<210> 12
<211> 909
<212> DNA
<213> Escherichia coli

<220>
<221> misc_feature
<223> yagE

<400> 12

atgccgcagt ccgcgttgtt cacgggaatc attccccctg tctccaccat ttttaccgcc 60
gacggccagc tcgataagcc gggcaccgcc gcgctgatcg acgatctgat caaagcaggc 120
gttgacggcc tgttcttcct gggcagcggt ggcgagttct cccagctcgg cgccgaagag 180
cgtaaagcca ttgcccgctt tgctatcgat catgtcgatc gtcgcgtgcc ggtgctgatc 240
ggcaccggcg gcaccaacgc ccgggaaacc atcgaactca gccagcacgc gcagcaggcg 300
ggcgcggacg gcatcgtggt gatcaacccc tactactgga aagtgtcgga agcgaacctg 360
atccgctatt tcgagcaggt ggccgacagc gtcacgctgc cggtgatgct ctataacttc 420
ccggcgctga ccgggcagga tctgactccg gcgctggtga aaaccctcgc cgactcgcgc 480
agcaatatta tcggcatcaa agacaccatc gactccgtcg cccacctgcg cagcatgatc 540
cataccgtca aaggtgccca tccgcacttc accgtgctct gcggctacga cgatcatctg 600
ttcaataccc tgctgctcgg cggcgacggg gcgatatcgg cgagcggcaa ctttgccccg 660
caggtgtcgg tgaatcttct gaaagcctgg cgcgacgggg acgtggcgaa agcggccggg 720
tatcatcaga ccttgctgca aattccgcag atgtatcagc tggatacgcc gtttgtgaac 780
gtgattaaag aggcgatcgt gctctgcggt cgtcctgtct ccacgcacgt gctgccgccc 840
gcctcgccgc tggacgagcc gcgcaaggcg cagctgaaaa ccctgctgca acagctcaag 900
ctttgctga 909

<210> 13
<211> 1434
<212> DNA
<213> Escherichia coli

<220>
<221> misc_feature
<223> ptsG

<400> 13

```
atgtttaaga atgcatttgc taacctgcaa aaggtcggta aatcgctgat gctgccggta      60
tccgtactgc ctatcgcagg tattctgctg ggcgtcggtt ccgcgaattt cagctggctg     120
cccgccgttg tatcgcatgt tatggcagaa gcaggcggtt ccgtctttgc aaacatgcca     180
ctgatttttg cgatcggtgt cgccctcggc tttaccaata acgatggcgt atccgcgctg     240
gccgcagttg ttgcctatgg catcatggtt aaaaccatgg ccgtggttgc gccactggta     300
ctgcatttac ctgctgaaga aatcgcctct aaacacctgg cggatactgg cgtactcgga     360
gggattatct ccggtgcgat cgcagcgtac atgtttaacc gtttctaccg tattaagctg     420
cctgagtatc ttggcttctt tgccggtaaa cgctttgtgc cgatcatttc tggcctggct     480
gccatcttta ctggcgttgt gctgtccttc atttggccgc cgattggttc tgcaatccag     540
accttctctc agtgggctgc ttaccagaac ccggtagttg cgtttggcat ttacggtttc     600
atcgaacgtt gcctggtacc gtttggtctg caccacatct ggaacgtacc tttccagatg     660
cagattggtg aatacaccaa cgcagcaggt caggttttcc acggcgacat tccgcgttat     720
atggcgggtg acccgactgc gggtaaactg tctggtggct tcctgttcaa aatgtacggt     780
ctgccagctg ccgcaattgc tatctggcac tctgctaaac cagaaaaccg cgcgaaagtg     840
ggcggtatta tgatctccgc ggcgctgacc tcgttcctga ccggtatcac cgagccgatc     900
gagttctcct tcatgttcgt tgcgccgatc ctgtacatca tccacgcgat tctggcaggc     960
ctggcattcc caatctgtat tcttctgggg atgcgtgacg gtacgtcgtt ctcgcacggt    1020
ctgatcgact tcatcgttct gtctggtaac agcagcaaac tgtggctgtt cccgatcgtc    1080
ggtatcggtt atgcgattgt ttactacacc atcttccgcg tgctgattaa agcactggat    1140
ctgaaaacgc cgggtcgtga agacgcgact gaagatgcaa aagcgacagg taccagcgaa    1200
atggcaccgg ctctggttgc tgcatttggt ggtaaagaaa acattactaa cctcgacgca    1260
tgtattaccc gtctgcgcgt cagcgttgct gatgtgtcta aagtggatca ggccggcctg    1320
aagaaactgg gcgcagcggg cgtagtggtt gctggttctg gtgttcaggc gattttcggt    1380
actaaatccg ataacctgaa aaccgagatg gatgagtaca tccgtaacca ctaa          1434
```

<210> 14
<211> 510
<212> DNA
<213> Escherichia coli

<220>
<221> misc_feature
<223> crr

<400> 14

```
atgggtttgt tcgataaact gaaatctctg gtttccgacg acaagaagga taccggaact      60
attgagatca ttgctccgct ctctggcgag atcgtcaata tcgaagacgt gccggatgtc     120
gtttttgcgg aaaaaatcgt tggtgatggt attgctatca aaccaacggg taacaaaatg     180
gtcgcgccag tagacggcac cattggtaaa atctttgaaa ccaaccacgc attctctatc     240
gaatctgata gcggcgttga actgttcgtc cacttcggta tcgacaccgt tgaactgaaa     300
ggcgaaggct tcaagcgtat tgctgaagaa ggtcagcgcg tgaaagttgg cgatactgtc     360
attgaatttg atctgccgct gctggaagag aaagccaagt ctaccctgac tccggttgtt     420
atctccaaca tggacgaaat caaagaactg atcaaactgt ccggtagcgt aaccgtgggt     480
gaaaccccgg ttatccgcat caagaagtaa                                      510
```

<210> 15
<211> 258
<212> DNA
<213> Escherichia coli

<220>
<221> misc_feature
<223> ptsH (hpr)

<400> 15

```
atgttccagc aagaagttac cattaccgct ccgaacggtc tgcacacccg ccctgctgcc      60
cagtttgtaa aagaagctaa gggcttcact tctgaaatta ctgtgacttc caacggcaaa     120
agcgccagcg cgaaaagcct gtttaaactg cagactctgg gcctgactca aggtaccgtt     180
gtgactatct ccgcagaagg cgaagacgag cagaaagcgg ttgaacatct ggttaaactg     240
atggcggaac tcgagtaa                                                   258
```

<210> 16
<211> 1254
<212> DNA
<213> Escherichia coli

<220>
<221> misc_feature
<223> lacY

<400> 16

```
atgtactatt taaaaaacac aaacttttgg atgttcggtt tattcttttt cttttacttt        60
tttatcatgg gagcctactt cccgtttttc ccgatttggc tacatgacat caaccatatc       120
agcaaaagtg atacgggtat tatttttgcc gctatttctc tgttctcgct attattccaa       180
ccgctgtttg gtctgctttc tgacaaactc gggctgcgca aatacctgct gtggattatt       240
accggcatgt tagtgatgtt tgcgccgttc tttattttta tcttcgggcc actgttacaa       300
tacaacattt tagtaggatc gattgttggt ggtatttatc taggcttttg ttttaacgcc       360
ggtgcgccag cagtagaggc atttattgag aaagtcagcc gtcgcagtaa tttcgaattt       420
ggtcgcgcgc ggatgtttgg ctgtgttggc tgggcgctgt gtgcctcgat tgtcggcatc       480
atgttcacca tcaataatca gtttgttttc tggctgggct ctggctgtgc actcatcctc       540
gccgttttac tctttttcgc caaaacggat gcgccctctt ctgccacggt tgccaatgcg       600
gtaggtgcca accattcggc atttagcctt aagctggcac tggaactgtt cagacagcca       660
aaactgtggt tttgtcact gtatgttatt ggcgtttcct gcacctacga tgtttttgac        720
caacagtttg ctaatttctt tacttcgttc tttgctaccg gtgaacaggg tacgcgggta       780
tttggctacg taacgacaat gggcgaatta cttaacgcct cgattatgtt ctttgcgcca       840
ctgatcatta atcgcatcgg tgggaaaaac gccctgctgc tggctggcac tattatgtct       900
gtacgtatta ttggctcatc gttcgccacc tcagcgctgg aagtggttat tctgaaaacg       960
ctgcatatgt ttgaagtacc gttcctgctg gtgggctgct ttaaatatat taccagccag      1020
tttgaagtgc gtttttcagc gacgatttat ctggtctgtt tctgcttctt taagcaactg      1080
gcgatgattt ttatgtctgt actggcgggc aatatgtatg aaagcatcgg tttccagggc      1140
gcttatctgg tgctgggtct ggtggcgctg ggcttcacct taatttccgt gttcacgctt      1200
agcggccccg gcccgctttc cctgctgcgt cgtcaggtga atgaagtcgc ttaa            1254
```

<210> 17
<211> 1545
<212> DNA
<213> Escherichia coli

<220>
<221> misc_feature
<223> malF

<400> 17

atggatgtca ttaaaaagaa acattggtgg caaagcgacg cgctgaaatg gtcagtgcta 60
ggtctgctcg gcctgctggt gggttacctt gttgttttaa tgtacgcaca aggggaatac 120
ctgttcgcca ttaccacgct gatattgagt tcagcggggc tgtatatttt cgccaatcgt 180
aaagcctacg cctggcgcta tgtttacccg ggaatggctg gaatgggatt attcgtcctc 240
ttccctctgg tctgcaccat cgccattgcc ttcaccaact acagcagcac taaccagctg 300
acttttgaac gtgcgcagga agtgttgtta gatcgctcct ggcaagcagg caaaacctat 360
aactttggtc tttacccggc gggcgatgag tggcaactgg cgctcagcga cggcgaaacc 420
ggcaaaaatt acctctccga cgcttttaaa tttggcggcg agcaaaaact gcaactgaaa 480
gaaacgaccg cccagcccga aggcgaacgc gcgaatctgc gcgtgattac ccagaatcgt 540
caggcgctga gtgacattac cgccattctg ccggatggca acaaagtgat gatgagctcc 600
ctgcgccagt ttctggcac gcagccgctc tacacactcg acggtgacgg cacgttgacg 660
aataatcaga gcggcgtgaa atatcgtccg aataaccaaa ttggctttta ccagtccatt 720
accgccgacg gcaactgggg tgatgaaaag ctaagccccg gttacaccgt gaccaccggc 780
tggaaaaact ttacccgcgt ctttaccgac gaaggcattc agaaaccgtt cctcgccatt 840
ttcgtctgga ccgtggtgtt ctcgctgatc actgtctttt taacggtggc ggtcggcatg 900
gttctggcgt gtctggtgca gtgggaagcg ttgcgcggca aagcggtcta tcgcgtcctg 960
ctgattctgc cctacgcggt gccatcgttc atttcaatct tgattttcaa agggttgttt 1020
aaccagagct tcggtgaaat caacatgatg ttgagcgcgc tgtttggcgt gaagcccgcc 1080
tggttcagcg atccgaccac cgcccgcacg atgctaatta tcgtcaatac ctggctgggt 1140
tatccgtaca tgatgatcct ctgcatgggc ttgctgaaag cgattccgga cgatttgtat 1200
gaagcctcag caatggatgg cgcaggtccg ttccagaact tctttaagat tacgctgccg 1260
ctgctgatta aaccgctgac gccgctgatg atcgccagct tcgcctttaa ctttaacaac 1320
ttcgtgctga ttcaactgtt aaccaacggc ggcccggatc gtcttggcac gaccacgcca 1380
gccggttata ccgacctgct tgttaactac acctaccgca tcgcttttga aggcggcggg 1440
ggtcaggact tcggtctggc ggcagcaatt gccacgctga tcttcctgct ggtgggtgcg 1500
ctggcgatag tgaacctgaa agccacgcga atgaagtttg attaa 1545

<210> 18
<211> 891
<212> DNA
<213> Escherichia coli

<220>
<221> misc_feature
<223> malG

<400> 18

```
atggcaatgg tccaaccgaa atcgcaaaaa gctcgtttat ttattactca cctgctactg      60
ctacttttta tcgcagcgat tatgttcccg ctgctgatgg tcgtcgctat ctcgctgcgt     120
cagggaaact ttgcgaccgg cagcctgatc ccggagcaaa tctcctggga tcactggaaa     180
ctggcgttag gttttagcgt tgaacaggct gatggtcgca ttacgccacc gccattcccg     240
gtactgctgt ggctgtggaa ctcggtaaag gtcgccggga tttccgcgat tggcattgtg     300
gcgctctcca ccacctgcgc ctacgctttc gcccgtatgc gctttccagg caaagcgacg     360
ctgctgaaag gaatgctgat tttccagatg ttcccggcag tactttcact ggtcgcgttg     420
tatgcgttgt ttgatcgtct gggtgagtac attccattca ttggcctgaa tactcacggc     480
ggcgtaattt tcgcgtatct gggtgggatt gcgctgcatg tctggaccat caaaggctat     540
ttcgaaacca tcgacagttc gctggaagaa gctgctgcgc tggatggtgc gacaccgtgg     600
caggccttcc gccttgtcct gttgccgctg tcagtaccga ttctggcggt ggtattcatc     660
ctgtcgttta tcgctgccat tactgaagtt ccggtcgcgt cgctgttact gcgtgacgta     720
aacagctaca ccctggccgt ggggatgcag caatacctca acccgcaaaa ctacctgtgg     780
ggtgactttg ccgccgctgc cgtgatgtct gcattaccga tcaccatcgt cttcttgctg     840
gctcaacgct ggctggtcaa cggcctgacg gcaggtggtg tgaaaggtta a              891
```

<210> 19
<211> 6546
<212> DNA
<213> Salmonella typhimurium

<220>
<221> misc_feature
<223> scrKYABR

<400> 19

atgaatgcaa aagtttgggt tctgggcgac gcggtggtgg acctgctgcc ggagagcgaa 60

gggcgcctgc tgcagtgccc tggaggcgcg ccggctaacg tggcggtagg ggttgcccgc 120

cttggcggca acagcggatt tatcggcgcc gtcggcggtg acccgtttgg ccgctacatg 180

cgtcataccc tgcaacagga gcaggtcgac gtcagccata tgtatctcga cgatcagcac 240

cgcacgtcca ctgtggtcgt cgaccttgac gaccaggggg aacgcacctt tacctttatg 300

gtacgcccca gcgcggacct gttcctggtt gaagaagacc tgccacagtt tgccgccgga 360

cagtggttgc acgtctgctc catcgcgctc agcgccgagc ccagccgtag cactaccttc 420

gcggcgatgg agagcatcag gtctgccggc ggtcgggtca gctttgaccc taatattcgt 480

cccgatctct ggcaggatca ggctttgctg ctagcctgcc tcgatcgcgc tttgcacatg 540

gccaacgtgg taaagctatc ggaagaggag ctggtcttca tcagcagcag taatgattta 600

gcatacggaa tcgccagcgt aacggagcgc tatcagccag aattgctact ggtgacccgg 660
ggcaaagcgg gggtgcttgc cgcgtttcag cagaagttta cccatttcaa cgcccggcct 720
gtggccagcg tggacaccac cggcgcggga gacgcatttg tcgccggact gctcgccagc 780
cttgcggcta acgggatgcc aacggacatg accgcactgg aaccgacact cacgcttgca 840
cagacctgcg gcgccctggc caccacagcc aaaggtgcga tgaccgcctt gccttatcag 900
cgcgatctca accgtcagtt ttaatcctta aagccgcttt gcgcggctca ctttgttgca 960
tgcatcacat ttattaaacc ggtttagcat atttgtttta agaaaaacaa aggtcgggct 1020
taacatagcg cctaaaccgg tttagcaaaa attataattt tccattttta cttttgggat 1080
gccaacagca tgtacagaaa aagcacactt gcgatgctta tcgctttgct aaccagcgct 1140
gcctcagccc atgcgcaaac ggatataagc accattgaag cccgactcaa cgcgctggaa 1200
aaacgcctgc aggaggcaga aaacagggcg caaacggcgg aaaaccgcgc cggggcggcg 1260
gagaaaaaag ttcagcaact caccgcgcag cagcaaaaaa accagaactc gactcaggaa 1320
gtggctcagc gtaccgccag acttgagaaa aaagccgatg acaaaagcgg atttgagttt 1380
cacggttacg cccgctccgg cgtgataatg aatgattccg gcgccagcac caaatccgga 1440
gcctacataa cgccggcagg tgaaaccggc ggagctatcg gccgtctggg aaaccaggcc 1500
gatacctatg ttgaaatgaa tcttgaacat aagcagaccc tggataatgg ggccacgacc 1560
cgctttaagg tgatggtcgc cgacgggcaa acctcttata acgactggac tgcaagcacc 1620
agcgatctga acgttcgtca ggcctttgtc gaattgggta acctgccgac gttcgctggg 1680
ccatttaagg gctccaccct gtgggccggg aaacgtttcg accgcgacaa tttcgatatt 1740
cactggattg actctgatgt cgtgttcctc gccggtaccg gtggtggtat ctatgacgtg 1800
aagtggaacg acggcctgcg gagtaatttc tccctgtacg ggcgtaactt cggcgacatt 1860
gatgattcca gcaacagcgt gcagaactat atcctcacca tgaatcactt cgcaggtccg 1920
ctgcagatga tggtcagcgg tctgcgggcg aaggataacg acgagcgtaa agatagcaac 1980
ggcaatctgg caaaaggcga tgcggcaaac accggcgtgc atgcgctgct cggcctgcat 2040
aacgacagtt tctacggcct gcgcgacggt agcagtaaaa ccgctctgct ttatggtcat 2100
ggtctgggcg cagaggttaa aggtatcgga tctgatggcg cacttcgtcc gggagccgac 2160
acatggcgca ttgccagtta cggcaccacg ccgctcagcg aaaactggtc tgttgccccg 2220
gcaatgctgg cgcaacgcag taaagaccgc tatgccgatg gcgacagcta tcagtgggca 2280
acattcaacc tgcgtctgat tcaggcaatc aatcagaatt tcgctctcgc ctacgaaggc 2340
agctaccagt acatggatct taaacccgaa ggttataacg atcgtcaggc ggtgaacggt 2400
agcttctaca agctcacctt cgccccgaca tttaaggtcg gcagtatcgg tgatttcttc 2460

agtcgcccgg agattcgttt ctatacctcc tggatggact ggagcaaaaa actgaataat 2520
tacgccagcg acgacgccct gggcagtgac ggttttaact cgggcggcga atggtctttc 2580
ggtgtgcaga tggaaacctg gttctgacgc ttacgcctga tgacaggaat agccgggggt 2640
cagagcatct ttgtcacccc ggactcaact aagacgcaga aaaagcgctc ccgtgaacgc 2700
gggacgacaa cataaaaatg tttaagcctt aagagggtac tatggatttt gaacagattt 2760
cctgctcgct gcttccgctt cttggaggca aagaaaatat cgccagcgcc gcgcactgcg 2820
ccacgcgcct gcgcctggtg ctggtcgatg attcgctggc cgaccagcag gccatcggca 2880
aagttgaagg ggtgaagggc tgttttcgta atgccggaca gatgcagatt attttcggca 2940
ccggggtggt aaataaggtc tacgctgcct ttactcaggc ggcgggtatt agcgaatcca 3000
gcaaatcgga agccgccgac atcgcggcaa aaaagctcaa tccgttccag cgcatcgccc 3060
gcctgctatc aaacatcttc gtgccgataa tccctgccat cgtcgcctct ggtctgctga 3120
tgggcctgct gggaatggtc aaaacatacg gctgggttga cccgggcaac gccatctaca 3180
tcatgctgga tatgtgcagc tcggcggcat ttatcattct gccgattctg attggcttta 3240
ccgccgcccg cgaattcggc ggtaatcctt atctcggcgc gacgcttggc ggcattctga 3300
ctcatccagc gctgactaac gcctggggcg tggccgcggg tttccacacc atgaactttt 3360
tcggcttcga aattgccatg atcggctatc agggtacggt gttcccggta ctgctggcag 3420
tatggtttat gagcatcgtt gagaagcagt tgcgtcgcgc aatccccgat gccctggatt 3480
tgatcctgac gccgttcctg acggtgatta tatccggttt tatcgccctg ttgattatcg 3540
gcccggccgg tcgcgcactg ggcgacggta tctcgtttgt cctcagcacc ctgattagcc 3600
acgccggctg gctcgccggg ttactgtttg gcggtctcta ttcagttatc gtcattaccg 3660
gtattcatca cagcttccat gcggttgaag ccgggttgct gggcaatccc tccatcggcg 3720
tcaacttcct gctgccgatt tgggcgatgg ccaacgtcgc tcagggcgga gcctgtctgg 3780
cggtgtggtt caaaaccaaa gatgcaaaaa ttaaagccat tactctgccc tcggcgtttt 3840
ccgccatgct gggcatcacc gaggcggcga tttttggtat taacctgcgc tttgtgaagc 3900
catttattgc ggcgctgatt ggtggtgcgg cgggcggcgc atgggtggta tctgtacacg 3960
tctacatgac cgcggtcggc ttgacagcga tccccggcat ggccatcgtg caggccagtt 4020
cgctgttgaa ctacattatc gggatggtta tcgcctttgg cgtcgccttt acggtctccc 4080
tggttttgaa atacaaaacg gacgctgaat aatgtctctt ccatcacgac tgcctgcgat 4140
tttgcaggcc gtaatgcagg gccagccgcg cgcgctggcc gatagccact atccgcgctg 4200
gcaccatgcg ccggtcaccg ggctgatgaa cgaccccaac ggctttatcg aatttgccgg 4260
acgctatcat ctgttttatc agtggaaccc gctcgcctgc gatcatacgt ttaagtgctg 4320
ggcgcactgg agttccatcg atctgctgca ctggcagcat gagcccattg cgctgatgcc 4380

ggacgaagag tatgaccgta acggctgcta ctccggcagc gcggtggata acaacggtac 4440
gcttaccctg tgctataccg gcaacgtgaa gtttgccgag ggagggcgaa ccgcctggca 4500
atgcctggca acggaaaacg ctgacggcac cttccgcaaa atcggtccgg tcctgccgct 4560
gccggagggc tacaccggcc acgtgcgcga cccaaaagtc tggcgacacg aagacctgtg 4620
gtacatggtg ctgggcgcgc aggatcggca aaagcgcggc aaggtgctgc tgttcagctc 4680
tgcggatctc catcagtgga cgagtatggg tgaaatcgcc ggccacggca tcaatggcct 4740
cgacgacgtc ggctatatgt gggagtgccc ggatcttttt ccactcggcg accagcatat 4800
tctaatctgc tgtccgcagg ggattgcccg tgaggaagag tgctacctga acacctaccc 4860
ggcagtatgg atggcgggcg agtttgatta cgctgctggc gctttcagac acggcgaact 4920
gcacgaactg gacgccgggt ttgagttcta cgccccgcaa accatgctta ccagtgatgg 4980
ccgtcgtctg ctggtcggct ggatgggcgt gccggagggc gaagagatgc ttcagccgac 5040
cctgaacaac ggctggatcc atcagatgac ctgcctgcgt gagctggagt ttatcaacgg 5100
tcagctctat cagcgtccgc tacgggaact gagcgccctg cgcggtgaag cgaacggctg 5160
gtcggggaac gccctgccgc tggcaccgat ggaaatcgat ttgcaaaccc gcgggggcga 5220
tatgttgagc ctcgattttg gcggcgtatt aacccttgag tgcgatgcca gcggactccg 5280
cctggcccga cgcagtctcg ccagtgacga gatgcattat cgttactggc gcggaaacgt 5340
ccgctcgctg cgtgttttca tcgaccagtc gagcgtggag attttcataa acggcggtga 5400
aggggtgatg agcagccgct acttcccggc ctgctccggt cagctaacat tctccggcat 5460
cacgccggac gcattctgct actggccgct gcgaacttgc atggtagaat aagcgttttg 5520
cttcaggctc atggcgtcgt aatgaaaacc aaacgcgtaa ccattaaaga tatagccgaa 5580
caggctggcg tctccaaagc gaccgccagc ttggtactga atggtcgtgg caaggagctg 5640
cgcgtggcgc aggaaacgcg tgagcgcgta ctgtcgattg cccgtaagca tcactatcag 5700
ccaagcattc atgcccgctc gctgcgcaac aaccgcagcc acaccatcgg gctggtggtg 5760
ccggagatca ccaaccacgg ctttgcggtc tttgcccatg agctggagat gctgtgccgc 5820
gaggcgggcg tccagctgtt gatctcttgt actgatgaaa accccggtca ggagagcgtg 5880
gtggtcaata atatgattgc ccgccaggtc gacgggatga tcgtcgcttc ctgtatgcac 5940
aacgatgccg actatctcaa actcagccaa cagctgccag tggtgctgtt tgaccggtgc 6000
cccaatgaaa gcgcgctgcc gctggtaatg accgattcga ttaccccaac ggcggaactg 6060
atttcccgca tcgcgcctca gcatagcgat gagttctggt ttttaggcgg tcaggcgcgt 6120
ctgtcgccct cccgcgatcg tctgaccggg ttcacgcagg gtttggctca ggcgggtatt 6180
gccctgcgcc cggaatgggt gatcaacggc aattaccacc ccagctccgg ctatgagatg 6240

```
tttgccgcac tctgcgcgcg ccttgggcgg ccgcctaagg cgctattcac cgccgcctgc      6300

gggctgctcg aaggggttct gcgctatatg agccagcacc atttactcga ttccgatatt      6360

catctgacga gctttgacga tcactatctt tatgattcgc tgtcgctgcg tatcgacact      6420

gtccagcagg ataatcgcca gctggcctgg cactgctacg atctgataag ccagctgatc      6480

gagggcgata cgcccgaaac gctacaacgc tacctgcccg caaccctgca gtttcggcat      6540

cagtaa                                                                 6546
```

<210> 20
<211> 4885
<212> DNA
<213> Escherichia coli

<220>
<221> misc_feature
<223> cscBKAR

<400> 20

```
ctatattgct gaaggtacag gcgtttccat aactatttgc tcgcgttttt tactcaggaa      60
gaaaatgcca aatagcaaca tcaggcagac aatacccgaa attgcgaaga aaactgtctg     120
gtagcctgcg tggtcaaaga gtatcccagt cggcgttgaa agcagcacaa tcccaagcga     180
actggcaatt tgaaaaccaa tcagaaagat cgtcgacgac aggcgcttat caaagtttgc     240
cacgctgtat ttgaagacgg atatgacaca aagtggaacc tcaatggcat gtaacagctt     300
cactaatgaa ataatccagg ggttaacgaa caacgcgcag gaaaggatac gcaacgccat     360
aatcacaaca ccgataagta atgcattttt tggccctacc cgattcacaa agaaaggaat     420
aatcgccatg cacagcgctt cgagtaccac ctggaatgag ttgagataac catacaggcg     480
cgttcctaca tcgtgtgatt cgaataaacc tgcataaaag acaggaaaga gttgttgatc     540
aaaaatgtta tagaaagacc acgtccccac aataaatatg acgaaaaccc agaagtttcg     600
atccttgaaa actgcgataa aatcctcttt ttttacccct cccgcatccg ccgctatgca     660
ctggtgatcc ttatctttaa aacgcatgtt gatcatcata aatacagcgc caaatagcga     720
gaccaaccag aagttgatat ggggactgat actaaaaaat ataccggcaa agaacgcgcc     780
aatagcatag ccaaaagatc cccaggcgcg cgctgttcca tattcgaaat gaaaatttcg     840
cgccattttt tcggtgaagc tgtcaagcaa accgcatccc gccagatacc ccaggccaaa     900
aaagagcgcc cccagaatta gacctacaga aaaattgctt tgcagtaacg gttcataaac     960
gtaaatcata aacggtccgg tcaagaccag aatgaaactc atacaccaga tgagcggttt    1020
cttcagaccg agtttatcct gaacgatgcc gtagaacatc ataaatagaa tgctggtaaa    1080
ctggttgacc gaataaagtg tacctaattc cgtccctgtt aatcctagat gtcctttcag    1140
ccaaatagcg tataacgacc accacagcga ccaggaaata aaaaagagaa atgagtaact    1200
```

ggatgcaaaa cgatagtacg catttctgaa tggaatattc agtgccataa ttacctgcct 1260
gtcgttaaaa aattcatgtc ctatttagag ataagagcgg cctcgccgtt tacttctcac 1320
tttccagttc ttgtcgacat ggcagcgctg tcattgcccc tttcgctgtt actgcaagcg 1380
ctccgcaacg ttgagcgaga tcgataattc gtcgcatttc tctctcatct gtagataatc 1440
ccgtagagga cagacctgtg agtaacccgg caacgaacgc atctcccgcc ccagtgctat 1500
cgacacaatt cacagacatt ccagcaaaat ggtggacttg tcctcgataa cagaccacca 1560
ccccttctgc acctttagtc accaacagca tggcgatctc atactctttt gccagggcgc 1620
atatatcccg atcgttctgt gtttttccac tgataagtcg ccattcttct tccgagagct 1680
tgacgacatc cgccagttgt agcgcctgcc gcaaacacaa gcggagcaaa tgctcgtctt 1740
gccatagatc ttcacgaata ttgggatcga agctgacaaa acctccggca tgccggatcg 1800
ccgtcatcgc agtaaatgcg ctggtacgcg aaggctcggc agacaacgca attgaacaga 1860
gatgtaacca ttcgccatgt cgccagcagg gcaagtctgt cgtctctaaa aaaagatcgg 1920
cactggggcg gaccataaac gtaaatgaac gttctccttg atcgttcaga tcgacaagca 1980
ccgtggatgt ccggtgccat tcatcttgct tcagatacgt gatatcgaca ccctcagtta 2040
gcagcgttct ttgcattaac gcaccaaaag gatcatcacc gacccgacct ataaacccac 2100
ttgttccgcc taatctggcg attcccaccg caacgttagc tggcgcgccg ccaggacaag 2160
gcagtagccg cccgtctgat tctggcaaga gatctacgac cgcatcccct aaaacccata 2220
ctttggctga cattttttttc ccttaaattc atctgactta cgcatagtga taaacctctt 2280
tttcgcaaaa tcgtcatgga tttactaaaa catgcatatt cgatcacaaa acgtcatagt 2340
taacgttaac atttgtgata ttcatcgcat ttatgaaagt aagggacttt atttttataa 2400
aagttaacgt taacaattca ccaaatttgc ttaaccagga tgattaaaat gacgcaatct 2460
cgattgcatg cggcgcaaaa cgcactagca aaacttcacg agcgccgagg taacactttc 2520
tatccccatt ttcacctcgc gcctcctgcc gggtggatga acgatccaaa cggcctgatc 2580
tggtttaacg atcgttatca cgcgttttat caacatcacc cgatgagcga acactggggg 2640
ccaatgcact ggggacatgc caccagcgac gatatgatcc actggcagca tgagcctatt 2700
gcgctagcgc caggagacga gaatgacaaa gacgggtgtt tttcaggtag tgctgtcgat 2760
gacaatggtg tcctctcact tatctacacc ggacacgtct ggctcgatgg tgcaggtaat 2820
gacgatgcaa ttcgcgaagt acaatgtctg gctaccagtc gggatggtat tcatttcgag 2880
aaacagggtg tgatcctcac tccaccagaa ggcatcatgc acttccgcga tcctaaagtg 2940
tggcgtgaag ccgacacatg gtggatggta gtcggggcga aagacccagg caacacgggg 3000
cagatcctgc tttatcgcgg cagttcattg cgtgaatgga ctttcgatcg cgtactggcc 3060

cacgctgatg cgggtgaaag ctatatgtgg gaatgtccgg actttttcag ccttggcgat 3120
cagcattatc tgatgttttc cccgcaggga atgaatgccg agggatacag ttatcgaaat 3180
cgctttcaaa gtggcgtaat acccggaatg tggtcgccag gacgactttt tgcacaatcc 3240
gggcatttta ctgaacttga taacgggcat gacttttatg caccacaaag ctttgtagcg 3300
aaggatggtc ggcgtattgt tatcggctgg atggatatgt gggaatcgcc aatgccctca 3360
aaacgtgaag gctgggcagg ctgcatgacg ctggcgcgcg agctatcaga gagcaatggc 3420
aaactcctac aacgcccggt acacgaagct gagtcgttac gccagcagca tcaatctatc 3480
tctccccgca caatcagcaa taaatatgtt ttgcaggaaa acgcgcaagc agttgagatt 3540
cagttgcagt gggcgctgaa gaacagtgat gccgaacatt acggattaca gctcggcgct 3600
ggaatgcggc tgtatattga taaccaatct gagcgacttg ttttgtggcg gtattaccca 3660
cacgagaatt tagatggcta ccgtagtatt cccctcccgc agggtgacat gctcgcccta 3720
aggatattta tcgatacatc atccgtggaa gtatttatta acgacgggga ggcggtgatg 3780
agtagccgaa tatatccgca gccagaagaa cgggaactgt cgctctatgc ctcccacgga 3840
gtggctgtgc tgcaacatgg agcactctgg caactgggtt aacataatat caggtggaac 3900
aacggatcaa cagcgggcaa gggatccgcg tcactcttcc cccttcacga ccttcaataa 3960
tatgcaatgc agcttcccgc ccgataatgt catgtggaag ctgaattgtg gtcagcggcg 4020
gtaaaaacag atgcccgacg ccaaccagat tatcaaagcc cattacggcg acatcctgcg 4080
ggatacgtac cccccttcgcc aaaagaacct gataagccac aaaggctgcg cgatcgttac 4140
cacatatcag aacatcaaaa tctggtttgc ccgatttgaa gtgggcattg agtaaacttg 4200
cgagatcggt gtagtgatca tcacctgttg ccatgtgaaa ttgtttcacc tcagccagat 4260
ctcgtccagc atcacgccag gcctgctcaa atccctgccg acgataccct gttgccaacg 4320
cactttccgg tagccagaag cataacggtt gacgatagcc cgccgcgagc aaatgctgtg 4380
ttgattcata ttgtgcagtg taatcatcag ggatataact gggtaacgct gggtcatccg 4440
ccacacagtt cgccaataca atattttcac catacagaga ctcaggcagc gtgatatgtc 4500
gcagccccat tgtagtatag ataatgccat ccggacggtg ggcaagcagc tgacgtgccg 4560
cgcgggcagc gtcatcttca gaaaaaatat tgattaaaaa actattccag ccgaactcgc 4620
tggcggtttg ctcaatggca agcagaatat caacagagaa aggagtggta gcagtgtcct 4680
gcgccagcac ggcgagagtc gacggcttac gtccttgagc gcgcatctta cgggcggaaa 4740
gatcaggaac ataattcagg gtctggattg cctgcaatac gcggtcacgc gttgcaggac 4800
gcacagattc tgcattatgc atcacccggg agactgtcat catcgacact cccgccaggc 4860
gtgcgacatc ctttaatgaa gccat 4885

<210> 21
<211> 3771
<212> DNA
<213> Escherichia coli

<220>
<221> misc_feature
<223> xylFGH

<400> 21

```
atgaaaataa agaacattct actcaccctt tgcacctcac tcctgcttac caacgttgct      60
gcacacgcca aagaagtcaa aataggtatg gcgattgatg atctccgtct tgaacgctgg     120
caaaaagatc gagatatctt tgtgaaaaag gcagaatctc tcggcgcgaa agtatttgta     180
cagtctgcaa atggcaatga agaaacacaa atgtcgcaga ttgaaaacat gataaaccgg     240
ggtgtcgatg ttcttgtcat tattccgtat aacggtcagg tattaagtaa cgttgtaaaa     300
gaagccaaac aagaaggcat taaagtatta gcttacgacc gtatgattaa cgatgcggat     360
atcgattttt atatttcttt cgataacgaa aaagtcggtg aactgcaggc aaaagccctg     420
gtcgatattg ttccgcaagg taattacttc ctgatgggcg gctcgccggt agataacaac     480
gccaagctgt tccgcgccgg acaaatgaaa gtgttaaaac cttacgttga ttccggaaaa     540
attaaagtcg ttggtgacca atgggttgat ggctggttac cggaaaacgc attgaaaatt     600
atggaaaacg cgctaaccgc caataataac aaaattgatg ctgtagttgc ctcaaacgat     660
gccaccgcag gtggggcaat tcaggcatta agcgcgcaag gtttatcagg gaaagtagca     720
atctccggcc aggatgcgga tctcgcaggt attaaacgta ttgctgccgg tacgcaaact     780
atgacggtgt ataaacctat tacgttgttg gcaaatactg ccgcagaaat tgccgttgag     840
ttgggcaatg gtcaggaacc aaaagcagat accacactga ataatggcct gaaagatgtc     900
ccctcccgcc tcctgacacc gatcgatgtg aataaaaaca acatcaaaga tacggtaatt     960
aaagacggat tccacaaaga gagcgagctg taagcgttac gccccagcgc ggagcggggg    1020
cgtgatttct ctccatgccg cgtgaatgaa ttggcttagg tggagtcgtt atgccttatc    1080
tacttgaaat gaagaacatt accaaaacct tcggcagtgt gaaggcgatt gataacgtct    1140
gcttgcggtt gaatgctggc gaaatcgtct cactttgtgg ggaaaatggg tctggtaaat    1200
caacgctgat gaaagtgctg tgtggtattt atccccatgg ctcctacgaa ggcgaaatta    1260
tttttgcggg agaagagatt caggcgagtc acatccgcga taccgaacgc aaaggtatcg    1320
ccatcattca tcaggaattg gccctggtga aagaattgac cgtgctggaa aatatcttcc    1380
tgggtaacga aataacccac aatggcatta tggattatga cctgatgacg ctacgctgtc    1440
agaagctgct cgcacaggtc agtttatcca tttcacctga tacccgcgtt ggcgatttag    1500
ggcttgggca acaacaactg gttgaaattg ccaaggcact taataaacag gtgcgcttgt    1560
```

taattctcga tgaaccgaca gcctcattaa ctgagcagga aacgtcgatt ttactggata 1620

ttattcgcga tctacaacag cacggtatcg cctgtattta tatttcgcac aaactcaacg 1680

aagtcaaagc gatttccgat acgatttgcg ttattcgcga cggacagcac attggtacgc 1740

gtgatgctgc cggaatgagt gaagacgata ttatcaccat gatggtcggg cgagagttaa 1800

ccgcgcttta ccctaatgaa ccacatacca ccggagatga aatattacgt attgaacatc 1860

tgacggcatg gcatccggtt aatcgtcata ttaaacgagt taatgatgtc tcgttttccc 1920

tgaaacgtgg cgaaatattg ggtattgccg gactcgttgg tgccggacgt accgagacca 1980

ttcagtgcct gtttggtgtg tggcccggac aatgggaagg aaaaatttat attgatggca 2040

aacaggtaga tattcgtaac tgtcagcaag ccatcgccca ggggattgcg atggtccccg 2100

aagacagaaa gcgcgacggc atcgttccgg taatggcggt tggtaaaaat attaccctcg 2160

ccgcactcaa taaatttacc ggtggcatta gccagcttga tgacgcggca gagcaaaaat 2220

gtattctgga atcaatccag caactcaaag ttaaaacgtc gtcccccgac cttgctattg 2280

gacgtttgag cggcggcaat cagcaaaaag cgatcctcgc tcgctgtctg ttacttaacc 2340

cgcgcattct cattcttgat gaacccacca ggggtatcga tattggcgcg aaatacgaga 2400

tctacaaatt aattaaccaa ctcgtccagc agggtattgc cgttattgtc atctcttccg 2460

aattacctga agtgctcggc cttagcgatc gtgtactggt gatgcatgaa gggaaactaa 2520

aagccaacct gataaatcat aacctgactc aggagcaggt gatggaagcc gcattgagga 2580

gcgaacatca tgtcgaaaag caatccgtct gaagtgaaat tggccgtacc gacatccggt 2640

ggcttctccg ggctgaaatc actgaatttg caggtcttcg tgatgattgc agctatcatc 2700

gcaatcatgc tgttctttac ctggaccacc gatggtgcct acttaagcgc ccgtaacgtc 2760

tccaacctgt tacgccagac cgcgattacc ggcatcctcg cggtaggaat ggtgttcgtc 2820

ataatttctg ctgaaatcga cctttccgtc ggctcaatga tggggctgtt aggtggcgtc 2880

gcggcgattt gtgacgtctg gttaggctgg cctttgccac ttaccatcat tgtgacgctg 2940

gttctgggac tgcttctcgg tgcctggaac ggatggtggg tcgcgtaccg taaagtccct 3000

tcatttattg tcaccctcgc gggcatgttg gcatttcgcg gcatactcat tggcatcacc 3060

aacggcacga ctgtatcccc caccagcgcc gcgatgtcac aaattgggca aagctatctc 3120

cccgccagta ccggcttcat cattggcgcg cttggcttaa tggcttttgt tggttggcaa 3180

tggcgcggaa gaatgcgccg tcaggctttg ggtttacagt ctccggcctc taccgcagta 3240

gtcggtcgcc aggctttaac cgctatcatc gtattaggcg caatctggct gttgaatgat 3300

taccgtggcg ttcccactcc tgttctgctg ctgacgttgc tgttactcgg cggaatgttt 3360

atggcaacgc ggacggcatt tggacgacgc atttatgcca tcggcggcaa tctggaagca 3420

gcacgtctct ccgggattaa cgttgaacgc accaaacttg ccgtgttcgc gattaacgga 3480

ttaatggtag ccatcgccgg attaatcctt agttctcgac ttggcgctgg ttcaccttct 3540

gcgggaaata tcgccgaact ggacgcaatt gcagcatgcg tgattggcgg caccagcctg 3600

gctggcggtg tgggaagcgt tgccggagca gtaatggggg catttatcat ggcttcactg 3660

gataacggca tgagtatgat ggatgtaccg accttctggc agtatatcgt taaaggtgcg 3720

attctgttgc tggcagtatg gatggactcc gcaaccaaac gccgttcttg a 3771

<210> 22
<211> 1395
<212> DNA
<213> Escherichia coli

<220>
<221> misc_feature
<223> galP

<400> 22

atgcctgacg ctaaaaaaca ggggcggtca aacaaggcaa tgacgttttt cgtctgcttc 60

cttgccgctc tggcgggatt actctttggc ctggatatcg gtgtaattgc tggcgcactg 120

ccgtttattg cagatgaatt ccagattact tcgcacacgc aagaatgggt cgtaagctcc 180

atgatgttcg gtgcggcagt cggtgcggtg ggcagcggct ggctctcctt taaactcggg 240

cgcaaaaaga gcctgatgat cggcgcaatt ttgtttgttg ccggttcgct gttctctgcg 300

gctgcgccaa acgttgaagt actgattctt tcccgcgttc tactggggct ggcggtgggt 360

gtggcctctt ataccgcacc gctgtacctc tctgaaattg cgccggaaaa aattcgtggc 420

agtatgatct cgatgtatca gttgatgatc actatcggga tcctcggtgc ttatctttct 480

gataccgcct tcagctacac cggtgcatgg cgctggatgc tgggtgtgat tatcatcccg 540

gcaattttgc tgctgattgg tgtcttcttc ctgccagaca gcccacgttg gtttgccgcc 600

aaacgccgtt ttgttgatgc cgaacgcgtg ctgctacgcc tgcgtgacac cagcgcggaa 660

gcgaaacgcg aactggatga aatccgtgaa agtttgcagg ttaaacagag tggctgggcg 720

ctgtttaaag agaacagcaa cttccgccgc gcggtgttcc ttggcgtact gttgcaggta 780

atgcagcaat tcaccgggat gaacgtcatc atgtattacg cgccgaaaat cttcgaactg 840

gcgggttata ccaacactac cgagcaaatg tgggggaccg tgattgtcgg cctgaccaac 900

gtacttgcca cctttatcgc aatcggcctt gttgaccgct ggggacgtaa accaacgcta 960

acgctgggct tcctggtgat ggctgctggc atgggcgtac tcggtacaat gatgcatatc 1020

ggtattcact ctccgtcggc gcagtatttc gccatcgcca tgctgctgat gtttattgtc 1080

ggttttgcca tgagtgccgg tccgctgatt tgggtactgt gctccgaaat tcagccgctg 1140

aaaggccgcg attttggcat cacctgctcc actgccacca actggattgc caacatgatc 1200

gttggcgcaa cgttcctgac catgctcaac acgctgggta acgccaacac cttctgggtg 1260

tatgcggctc tgaacgtact gtttatcctg ctgacattgt ggctggtacc ggaaaccaaa 1320

cacgtttcgc tggaacatat tgaacgtaat ctgatgaaag gtcgtaaact gcgcgaaata 1380

ggcgctcacg attaa 1395

<210> 23
<211> 1422
<212> DNA
<213> Zymomonas mobilis

<220>
<221> misc_feature
<223> glf

<400> 23

```
atgagttctg aaagtagtca gggtctagtc acgcgactag ccctaatcgc tgctataggc      60
ggcttgcttt tcggttacga ttcagcggtt atcgctgcaa tcggtacacc ggttgatatc     120
cattttattg cccctcgtca cctgtctgct acggctgcgg cttccctttc tgggatggtc     180
gttgttgctg ttttggtcgg ttgtgttacc ggttctttgc tgtctggctg gattggtatt     240
cgcttcggtc gtcgcggcgg attgttgatg agttccattt gtttcgtcgc cgccggtttt     300
ggtgctgcgt taaccgaaaa attatttgga accggtggtt cggctttaca aatttttttgc     360
tttttccggt ttcttgccgg tttaggtatc ggtgtcgttt caaccttgac cccaacctat     420
attgctgaaa ttcgtccgcc agacaaacgt ggtcagatgg tttctggtca gcagatggcc     480
attgtgacgg gtgctttaac cggttatatc tttacctggt tactggctca tttcggttct     540
atcgattggg ttaatgccag tggttggtgc tggtctccgg cttcagaagg cctgatcggt     600
attgccttct tattgctgct gttaaccgca ccggatacgc cgcattggtt ggtgatgaag     660
ggacgtcatt ccgaggctag caaaatcctt gctcgtctgg aaccgcaagc cgatcctaat     720
ctgacgattc aaaagattaa agctggcttt gataaagcca tggacaaaag cagcgcaggt     780
ttgtttgctt ttggtatcac cgttgttttt gccggtgtat ccgttgctgc cttccagcag     840
ttagtcggta ttaacgccgt gctgtattat gcaccgcaga tgttccagaa tttaggtttt     900
ggagctgata cggcattatt gcagaccatc tctatcggtg ttgtgaactt catcttcacc     960
atgattgctt cccgtgttgt tgaccgcttc ggccgtaaac ctctgcttat ttggggtgct    1020
ctcggtatgg ctgcaatgat ggctgtttta ggctgctgtt tctggttcaa agtcggtggt    1080
gttttgcctt tggcttctgt gcttctttat attgcagtct ttggtatgtc atggggccct    1140
gtctgctggg ttgttctgtc agaaatgttc ccgagttcca tcaagggcgc agctatgcct    1200
atcgctgtta ccggacaatg gttagctaat atcttggtta acttcctgtt taaggttgcc    1260
gatggttctc cagcattgaa tcagactttc aaccacggtt tctcctatct cgttttcgca    1320

gcattaagta tcttaggtgg cttgattgtt gctcgcttcg tgccggaaac caaaggtcgg    1380
agcctggatg aaatcgagga gatgtggcgc tcccagaagt ag                        1422
```

<210> 24
<211> 984
<212> DNA
<213> Zymomonas mobilis

<220>
<221> misc_feature
<223> glk

<400> 24

```
atggaaattg ttgcgattga catcggtgga acgcatgcgc gtttctctat tgcggaagta        60
agcaatggtc gggttctttc tcttggagaa gaaacaactt ttaaaacggc agaacatgct       120
agcttgcagt tagcttggga acgtttcggt gaaaaactgg gtcgtcctct gccacgtgcc       180
gcagctattg catgggctgg cccggttcat ggtgaagttt taaaacttac caataaccct       240
tgggtattaa gaccagctac tctgaatgaa aagctggaca tcgatacgca tgttctgatc       300
aatgacttcg gcgcggttgc ccacgcggtt gcgcatatgg attcttctta tctggatcat       360
atttgtggtc ctgatgaagc gcttcctagc gatggtgtta tcactattct tggtccggga       420
acgggcttgg gtgttgccca tctgttgcgg actgaaggcc gttatttcgt catcgaaact       480
gaaggcggtc atatcgactt tgctccgctt gacagacttg aagacaaaat tctggcacgt       540
ttacgtgaac gtttccgccg cgtttctatc gaacgcatta tttctggccc gggtcttggt       600
aatatctacg aagcactggc tgccattgaa ggcgttccgt tcagcttgct ggatgatatt       660
aaattatggc agatggcttt ggaaggtaaa gacaaccttg ctgaagccgc tttggatcgc       720
ttctgcttga gccttggcgc tatcgctggt gatcttgctt tggcacaggg tcgaaccagt       780
gttgttattg gcggtggtgt cggtcttcgt atcgcttccc atttgccaga atctggtttc       840
cgtcagcgct ttgtttcaaa aggacgcttt gaacgcgtca tgtccaagat tccggttaag       900
ttgattactt atccgcagcc tggactgttg ggtgcgcagc tgcctatgcc aacaaatatt       960
ctgaagttga ataatatttt ttaa                                              984
```

<210> 25
<211> 966
<212> DNA
<213> Escherichia coli

<220>
<221> misc_feature
<223> glk

<400> 25

```
atgacaaagt atgcattagt cggtgatgtg ggcggcacca acgcacgtct tgctctgtgt      60
gatattgcca gtggtgaaat ctcgcaggct aagacctatt cagggcttga ttaccccagc     120
ctcgaagcgg tcattcgcgt ttatcttgaa gaacataagg tcgaggtgaa agacggctgt     180
attgccatcg cttgcccaat taccggtgac tgggtggcga tgaccaacca tacctgggcg     240
ttctcaattg ccgaaatgaa aaagaatctc ggttttagcc atctggaaat tattaacgat     300
tttaccgctg tatcgatggc gatcccgatg ctgaaaaaag agcatctgat tcagtttggt     360
ggcgcagaac cggtcgaagg taagcctatt gcggtttacg gtgccggaac ggggcttggg     420
gttgcgcatc tggtccatgt cgataagcgt tgggtaagct tgccaggcga aggcggtcac     480
gttgattttg cgccgaatag tgaagaagag gccattatcc tcgaaatatt gcgtgcggaa     540
attggtcatg tttcggcgga gcgcgtgctt tctggccctg ggctggtgaa tttgtatcgc     600
gcaattgtga aagctgacaa ccgcctgcca gaaaatctca agccaaaaga tattaccgaa     660
cgcgcgctgg ctgacagctg caccgattgc cgccgcgcat tgtcgctgtt ttgcgtcatt     720
atgggccgtt ttggcggcaa tctggcgctc aatctcggga catttggcgg cgtgtttatt     780
gcgggcggta tcgtgccgcg cttccttgag ttcttcaaag cctccggttt ccgtgccgca     840
tttgaagata aagggcgctt taaagaatat gtccatgata ttccggtgta tctcatcgtc     900
catgacaatc cgggccttct cggttccggt gcacatttac gccagacctt aggtcacatt     960
ctgtaa                                                                966
```

<210> 26
<211> 2547
<212> DNA
<213> Escherichia coli

<220>
<221> misc_feature
<223> cyaA

<400> 26

```
ttgtacctct atattgagac tctgaaacag agactggatg ccataaatca attgcgtgtg        60
gatcgcgcgc ttgctgctat ggggcctgca ttccaacagg tctacagtct actgccgaca       120
ttgttgcact atcaccatcc gctaatgccg ggttaccttg atggtaacgt tcccaaaggc       180
atttgccttt acacgcctga tgaaactcaa cgccactacc tgaacgagct tgaactgtat       240
cgtggaatgt cagtacagga tccgccgaaa ggtgagcttc caattactgg tgtatacacc       300
atgggcagca cctcgtccgt agggcaaagt tgttcctctg acctggatat ctgggtctgt       360
catcaatcct ggctcgatag cgaagagcgc caattgctac aacgtaaatg tagcctgctg       420
gaaaactggg ccgcctcgct gggtgtggaa gtcagcttct tcctgattga tgaaaaccgc       480
ttccgtcata atgaaagcgg cagcctgggg ggcgaagatt gtggctccac ccagcatata       540
```

```
ctgctgcttg acgaatttta tcgtaccgcc gtgcgtctcg ccggtaagcg tattctgtgg 600
aatatggtgc cgtgcgacga agaagagcat tacgacgact atgtgatgac gctttacgcg 660
cagggcgtgc tgacgccaaa tgaatggctg gatctcggtg gcttaagctc gctttctgct 720
gaagagtact ttggtgccag cctttggcag ctctacaaga gtatcgattc cccatacaaa 780
gcggtactga aaacactgct gctggaagcc tattcctggg aatacccgaa cccacgtctg 840
ctggcgaaag atatcaaaca gcgtttgcac gacggcgaga ttgtatcgtt tggtctcgat 900
ccatactgca tgatgctgga gcgtgttact gaatacctga cggcgattga agattttacc 960
cgtctggatt tagtacgtcg ctgcttctat ttaaaagtgt gcgaaaagct cagccgtgaa 1020
cgcgcctgcg taggctggcg tcgcgcagtg ttgagccagt tagtgagcga gtggggttgg 1080
gacgaagctc gtctggcaat gctcgataac cgcgctaact ggaagattga tcaggtgcgt 1140
gaggcgcaca acgagttgct cgacgcgatg atgcagagct accgtaatct gatccgcttt 1200
gcgcgtcgca ataaccttag cgtctccgcc agtccgcagg atatcggcgt gctgacgcgt 1260
aagctgtatg ccgcgtttga agcattacca ggtaaagtga cgctggtaaa cccgcagatt 1320
tcacccgatc tctcggaacc gaatctgacc tttatttatg tgccgccggg ccgggctaac 1380
cgttcaggtt ggtatctgta taaccgcgcg ccaaatattg agtcgatcat cagccatcag 1440
ccgctggaat ataaccgtta cctgaataaa ctggtggcgt gggcatggtt taacggcctg 1500
ctgacctcgc gcacccgttt gtatattaaa ggtaacggca ttgtcgattt gcctaagttg 1560
caggagatgg tcgccgacgt gtcgcaccat ttcccgctgc gcttacctgc accgacaccg 1620
aaggcgctct acagcccgtg tgagatccgc catctggcga ttatcgttaa cctggaatat 1680
gacccgacag cggcgttccg caatcaggtg gtgcatttcg atttccgtaa gctggatgtc 1740
ttcagctttg gcgagaatca aaattgcctg gtaggtagcg ttgacctgct gtaccgcaac 1800
tcgtggaacg aagtgcgtac gctgcacttc aacggcgagc aatcgatgat cgaagccctg 1860
aaaactattc tcggcaaaat gcatcaggac gccgcaccgc cagatagcgt ggaagtcttc 1920
tgttatagcc agcatctgcg cggcttaatt cgtactcgcg tgcagcaact ggtttctgag 1980
tgtattgaat tgcgtctttc cagcacccgc caggaaaccg ggcgtttcaa ggcgctgcgc 2040
gtttctggtc aaacctgggg gttgttcttc gaacgcctga atgtatcggt acagaaactg 2100
gaaaacgcca tcgagtttta tggcgcgatt tcgcataaca aactgcacgg cctgtcagtg 2160
caggttgaaa ccaatcacgt caaattaccg gcggtggtgg acggctttgc cagcgaaggg 2220
atcatccagt tctttttcga agaaacgcaa gacgagaatg gctttaatat ctacattctc 2280
gacgaaagca accgggttga ggtatatcac cactgcgaag gcagcaaaga ggagctggta 2340
cgtgacgtca gtcgcttcta ctcgtcatcg catgaccgtt ttacctacgg ctcaagcttc 2400
```

```
atcaacttca acctgccgca gttctatcag attgtgaagg ttgatggtcg tgaacaggtg     2460

attccgttcc gcacaaaatc tatcggtaac atgccgcctg ccaatcagga tcacgatacg     2520

ccgctattac agcaatattt ttcgtga                                         2547
```

<210> 27
<211> 210
<212> PRT
<213> Escherichia coli

<220>
<221> misc_feature
<223> crp

<400> 27

```
Met Val Leu Gly Lys Pro Gln Thr Asp Pro Thr Leu Glu Trp Phe Leu
1               5                   10                  15

Ser His Cys His Ile His Lys Tyr Pro Ser Lys Ser Thr Leu Ile His
            20                  25                  30

Gln Gly Glu Lys Ala Glu Thr Leu Tyr Tyr Ile Val Lys Gly Ser Val
        35                  40                  45

Ala Val Leu Ile Lys Asp Glu Glu Gly Lys Glu Met Ile Leu Ser Tyr
    50                  55                  60

Leu Asn Gln Gly Asp Phe Ile Gly Glu Leu Gly Leu Phe Glu Glu Gly
65                  70                  75                  80

Gln Glu Arg Ser Ala Trp Val Arg Ala Lys Thr Ala Cys Glu Val Ala
                85                  90                  95

Glu Ile Ser Tyr Lys Lys Phe Arg Gln Leu Ile Gln Val Asn Pro Asp
            100                 105                 110

Ile Leu Met Arg Leu Ser Ala Gln Met Ala Arg Arg Leu Gln Val Thr
        115                 120                 125

Ser Glu Lys Val Gly Asn Leu Ala Phe Leu Asp Val Thr Gly Arg Ile
    130                 135                 140

Ala Gln Thr Leu Leu Asn Leu Ala Lys Gln Pro Asp Ala Met Thr His
145                 150                 155                 160

Pro Asp Gly Met Gln Ile Lys Ile Thr Arg Gln Glu Ile Gly Gln Ile
                165                 170                 175
```

Val Gly Cys Ser Arg Glu Thr Val Gly Arg Ile Leu Lys Met Leu Glu
180 185 190

Asp Gln Asn Leu Ile Ser Ala His Gly Lys Thr Ile Val Val Tyr Gly
195 200 205

Thr Arg
210

<210> 28
<211> 210
<212> PRT
<213> Escherichia coli

<220>
<221> misc_feature
<223> mutated crp

<400> 28

Met Val Leu Gly Lys Pro Gln Thr Asp Pro Thr Leu Glu Trp Phe Leu
1 5 10 15

Ser His Cys His Ile His Lys Tyr Pro Ser Lys Ser Thr Leu Ile His
20 25 30

Gln Gly Glu Lys Ala Glu Thr Leu Tyr Tyr Ile Val Lys Gly Ser Val
35 40 45

Ala Val Leu Ile Lys His Glu Glu Gly Lys Glu Met Ile Leu Ser Tyr
50 55 60

Leu Asn Gln Gly Asp Phe Ile Gly Glu Leu Gly Leu Phe Glu Glu Gly
65 70 75 80

Gln Glu Arg Ser Ala Trp Val Arg Ala Lys Thr Ala Cys Glu Val Ala
85 90 95

Glu Ile Ser Tyr Lys Lys Phe Arg Gln Leu Ile Gln Val Asn Pro Asp
100 105 110

Ile Leu Met Arg Leu Ser Ala Gln Met Ala Arg Arg Leu Gln Val Thr
115 120 125

Ser Glu Lys Val Gly Asn Leu Ala Phe Leu Asp Val Thr Gly Arg Ile
130 135 140

Ala Gln Thr Leu Leu Asn Leu Ala Lys Gln Pro Asp Ala Met Thr His
145 150 155 160

```
Pro Asp Gly Met Gln Ile Lys Ile Thr Arg Gln Glu Ile Gly Gln Ile
                165                 170                 175

Val Gly Cys Ser Arg Glu Thr Val Gly Arg Ile Leu Lys Met Leu Glu
            180                 185                 190

Asp Gln Asn Leu Ile Ser Ala His Gly Lys Thr Ile Val Val Tyr Gly
        195                 200                 205

Thr Arg
    210
```

<210> 29
<211> 210
<212> PRT
<213> Escherichia coli

<220>
<221> misc_feature
<223> mutated crp

<400> 29

```
Met Val Leu Gly Lys Pro Gln Thr Asp Pro Thr Leu Glu Trp Phe Leu
1               5                   10                  15

Ser His Cys His Ile His Lys Tyr Pro Ser Lys Ser Thr Leu Ile His
            20                  25                  30

Gln Gly Glu Lys Ala Glu Thr Leu Tyr Tyr Ile Val Lys Gly Ser Val
        35                  40                  45

Ala Val Leu Ile Lys Asp Glu Glu Gly Lys Glu Met Ile Leu Phe Tyr
    50                  55                  60

Leu Asn Gln Gly Asp Phe Ile Gly Glu Leu Gly Leu Phe Glu Glu Gly
65                  70                  75                  80

Gln Glu Arg Ser Ala Trp Val Arg Ala Lys Thr Ala Cys Glu Val Ala
                85                  90                  95

Glu Ile Ser Tyr Lys Lys Phe Arg Gln Leu Ile Gln Val Asn Pro Asp
            100                 105                 110

Ile Leu Met Arg Leu Ser Ala Gln Met Ala Arg Arg Leu Gln Val Thr
        115                 120                 125

Ser Glu Lys Val Gly Asn Leu Ala Phe Leu Asp Val Thr Gly Arg Ile
    130                 135                 140
```

Ala Gln Thr Leu Leu Asn Leu Ala Lys Gln Pro Asp Ala Met Thr His
145 150 155 160

Pro Asp Gly Met Gln Ile Lys Ile Thr Arg Gln Glu Ile Gly Gln Ile
165 170 175

Val Gly Cys Ser Arg Glu Thr Val Gly Arg Ile Leu Lys Met Leu Glu
180 185 190

Asp Gln Asn Leu Ile Ser Ala His Gly Lys Thr Ile Val Val Tyr Gly
195 200 205

Thr Arg
210

<210> 30
<211> 210
<212> PRT
<213> Escherichia coli

<220>
<221> misc_feature
<223> mutated crp

<400> 30

Met Val Leu Gly Lys Pro Gln Thr Asp Pro Thr Leu Glu Trp Phe Leu
1 5 10 15

Ser His Cys His Ile His Lys Tyr Pro Ser Lys Ser Thr Leu Ile His
20 25 30

Gln Gly Glu Lys Ala Glu Thr Leu Tyr Tyr Ile Val Lys Gly Ser Val
35 40 45

Ala Val Leu Ile Lys Asp Glu Glu Gly Lys Glu Met Ile Leu Ser Tyr
50 55 60

Leu Asn Gln Gly Asp Phe Ile Gly Glu Leu Gly Leu Phe Glu Glu Gly
65 70 75 80

Gln Glu Arg Ser Ala Trp Val Arg Ala Lys Thr Ala Cys Glu Val Ala
85 90 95

Glu Ile Ser Tyr Lys Lys Phe Arg Gln Leu Ile Gln Val Asn Pro Asp
100 105 110

Ile Leu Met Arg Leu Ser Ala Gln Met Ala Arg Arg Leu Gln Val Thr

```
            115                 120                 125

Ser Glu Lys Val Gly Asn Leu Ala Phe Leu Asp Val Thr Asp Arg Ile
    130                 135                 140

Ala Gln Thr Leu Leu Asn Leu Ala Lys Gln Pro Asp Ala Met Thr His
145                 150                 155                 160

Pro Asp Gly Met Gln Ile Lys Ile Thr Arg Gln Glu Ile Gly Gln Ile
                165                 170                 175

Val Gly Cys Ser Arg Glu Thr Val Gly Arg Ile Leu Lys Met Leu Glu
            180                 185                 190

Asp Gln Asn Leu Ile Ser Ala His Gly Lys Thr Ile Val Val Tyr Gly
        195                 200                 205

Thr Arg
    210
```

<210> 31
<211> 210
<212> PRT
<213> Escherichia coli

<220>
<221> misc_feature
<223> mutated crp

<400> 31

```
Met Val Leu Gly Lys Pro Gln Thr Asp Pro Thr Leu Glu Trp Phe Leu
1               5                   10                  15

Ser His Cys His Ile His Lys Tyr Pro Ser Lys Ser Thr Leu Ile His
            20                  25                  30

Gln Gly Glu Lys Ala Glu Thr Leu Tyr Tyr Ile Val Lys Gly Ser Val
        35                  40                  45

Ala Val Leu Ile Lys Asp Glu Glu Gly Lys Glu Met Ile Leu Ser Tyr
    50                  55                  60

Leu Asn Gln Gly Asp Phe Ile Gly Glu Leu Gly Leu Phe Glu Glu Gly
65                  70                  75                  80

Gln Glu Arg Ser Ala Trp Val Arg Ala Lys Thr Ala Cys Glu Val Ala
                85                  90                  95
```

Glu Ile Ser Tyr Lys Lys Phe Arg Gln Leu Ile Gln Val Asn Pro Asp
100 105 110

Ile Leu Met Arg Leu Ser Ala Gln Met Ala Arg Arg Leu Gln Val Thr
115 120 125

Ser Glu Lys Val Gly Asn Leu Ala Phe Leu Asp Val Thr Gly Asp Ile
130 135 140

Ala Gln Thr Leu Leu Asn Leu Ala Lys Gln Pro Asp Ala Met Thr His
145 150 155 160

Pro Asp Gly Met Gln Ile Lys Ile Thr Arg Gln Glu Ile Gly Gln Ile
165 170 175

Val Gly Cys Ser Arg Glu Thr Val Gly Arg Ile Leu Lys Met Leu Glu
180 185 190

Asp Gln Asn Leu Ile Ser Ala His Gly Lys Thr Ile Val Val Tyr Gly
195 200 205

Thr Arg
210

<210> 32
<211> 210
<212> PRT
<213> Escherichia coli

<220>
<221> misc_feature
<223> mutated crp

<400> 32

Met Val Leu Gly Lys Pro Gln Thr Asp Pro Thr Leu Glu Trp Phe Leu
1 5 10 15

Ser His Cys His Ile His Lys Tyr Pro Ser Lys Ser Thr Leu Ile His
20 25 30

Gln Gly Glu Lys Ala Glu Thr Leu Tyr Tyr Ile Val Lys Gly Ser Val
35 40 45

Ala Val Leu Ile Lys Asp Glu Glu Gly Lys Glu Met Ile Leu Ser Tyr
50 55 60

Leu Asn Gln Gly Asp Phe Ile Gly Glu Leu Gly Leu Phe Glu Glu Gly
65 70 75 80

```
Gln Glu Arg Ser Ala Trp Val Arg Ala Lys Thr Ala Cys Glu Val Ala
                85                  90                  95

Glu Ile Ser Tyr Lys Lys Phe Arg Gln Leu Ile Gln Val Asn Pro Asp
            100                 105                 110

Ile Leu Met Arg Leu Ser Ala Gln Met Ala Arg Arg Leu Gln Val Thr
        115                 120                 125

Ser Glu Lys Val Gly Asn Leu Ala Phe Leu Asp Val Thr Gly Arg Ile
    130                 135                 140

Ala Gln Thr Leu Arg Asn Leu Ala Lys Gln Pro Asp Ala Met Thr His
145                 150                 155                 160

Pro Asp Gly Met Gln Ile Lys Ile Thr Arg Gln Glu Ile Gly Gln Ile
                165                 170                 175

Val Gly Cys Ser Arg Glu Thr Val Gly Arg Ile Leu Lys Met Leu Glu
            180                 185                 190

Asp Gln Asn Leu Ile Ser Ala His Gly Lys Thr Ile Val Val Tyr Gly
        195                 200                 205

Thr Arg
    210
```

<210> 33
<211> 1845
<212> DNA
<213> Escherichia coli

<220>
<221> misc_feature
<223> btuB

<400> 33

```
atgattaaaa aagcttcgct gctgacggcg tgttccgtca cggcattttc cgcttgggca     60
caggatacca gcccggatac tctcgtcgtt actgctaacc gttttgaaca gccgcgcagc    120
actgtgcttg caccaaccac cgttgtgacc cgtcaggata tcgaccgctg gcagtcgacc    180
tcggtcaatg atgtgctgcg ccgtcttccg ggcgtcgata tcacccaaaa cggcggttca    240
ggtcagctct catctatttt tattcgcggt acaaatgcca gtcatgtgtt ggtgttaatt    300
gatggcgtac gcctgaatct ggcgggggtg agtggttctg ccgaccttag ccagttccct    360
attgcgcttg tccagcgtgt tgaatatatc cgtgggccgc gctccgctgt ttatggttcc    420
gatgcaatag gcggggtggt gaatatcatc acgacgcgcg atgaacccgg aacggaaatt    480
```

```
tcagcagggt ggggaagcaa tagttatcag aactatgatg tctctacgca gcaacaactg      540
ggggataaga cacgggtaac gctgttgggc gattatgccc atactcatgg ttatgatgtt      600
gttgcctatg gtaataccgg aacgcaagcg cagacagata acgatggttt tttaagtaaa      660
acgctttatg gcgcgctgga gcataacttt actgatgcct ggagcggctt tgtgcgcggc      720
tatggctatg ataaccgtac caattatgac gcgtattatt ctcccggttc accgttgctc      780
gatacccgta aactctatag ccaaagttgg gacgccgggc tgcgctataa cggcgaactg      840
attaaatcac aactcattac cagctatagc catagcaaag attacaacta cgatccccat      900
tatggtcgtt atgattcgtc ggcgacgctc gatgagatga agcaatacac cgtccagtgg      960
gcaaacaatg tcatcgttgg tcacggtagt attggtgcgg gtgtcgactg gcagaaacag     1020
actacgacgc cgggtacagg ttatgttgag gatggatatg atcaacgtaa taccggcatc     1080
tatctgaccg ggctgcaaca agtcggcgat tttacctttg aaggcgcagc acgcagtgac     1140
gataactcac agtttggtcg tcatggaacc tggcaaacca gcgccggttg ggaattcatc     1200
gaaggttatc gcttcattgc ttcctacggg acatcttata aggcaccaaa tctggggcaa     1260
ctgtatggct tctacggaaa tccgaatctg gacccggaga aaagcaaaca gtgggaaggc     1320
gcgtttgaag gcttaaccgc tggggtgaac tggcgtattt ccggatatcg taacgatgtc     1380
agtgacttga tcgattatga tgatcacacc ctgaaatatt acaacgaagg gaaagcgcgg     1440
attaagggcg tcgaggcgac cgccaatttt gataccggac cactgacgca tactgtgagt     1500
tatgattatg tcgatgcgcg caatgcgatt accgacacgc cgttgttacg ccgtgctaaa     1560
cagcaggtga aataccagct cgactggcag ttgtatgact tcgactgggg tattacttat     1620
cagtatttag gcactcgcta tgataaggat tactcatctt atccttatca aaccgttaaa     1680
atgggcggtg tgagcttgtg ggatcttgcg gttgcgtatc cggtcacctc tcacctgaca     1740
gttcgtggta aaatagccaa cctgttcgac aaagattatg agacagtcta tggctaccaa     1800
actgcaggac gggaatacac cttgtctggc agctacacct tctga                     1845
```

<210> 34
<211> 981
<212> DNA
<213> Escherichia coli

<220>
<221> misc_feature
<223> btuC

<400> 34

```
atgctgacac ttgcccgcca acaacagcga caaaatattc gctggttatt atgcctgtca        60
gttttgatgc tgctggcgct tctcttaagc ctttgcgccg gtgaacaatg gatctcgcca       120
ggtgactggt ttactcctcg tggcgaactg ttcgtctggc aaattcgcct gccacgtacg       180
ctggctgtat tgctggttgg tgcggcgctg gctatatccg gcgctgtaat gcaggcgttg       240
tttgaaaatc ctctggcaga acctggacta cttggcgtct ctaacggcgc aggcgtgggg       300
cttatcgccg cggtattgct tgggcaaggg caactcccca actgggcgct agggctgtgt       360
gcgattgctg gcgcgcttat catcacttta atactcttac gtttcgcccg tcgtcatctt       420
tcgaccagtc ggttattgct ggctggcgtt gcattaggga ttatctgtag cgcactaatg       480
acgtgggcta tctacttttc cacctcagtt gatttgcgtc agctgatgta ctggatgatg       540
ggcggttttg gcggcgtaga ctggcggcaa agctggctga tgctggcatt gatccccgtg       600
ttgttgtgga tctgttgtca gtccaggccg atgaatatgt tagcacttgg cgagatctcg       660
gcgcggcaac tgggtttacc cctgtggttc tggcgcaatg tgctggtggc agcgaccggc       720
tggatggttg gcgtcagtgt ggcgctggcg ggtgctatcg gctttattgg tctggtgatc       780
ccccatattc tccggttgtg tggtttaacc gatcatcgcg tattacttcc cggctgcgcg       840
ctggcagggg cgagcgcatt gctgctggcc gatattgtag cgcgcctggc attagctgcc       900
gcagagctgc ctattggcgt ggtcaccgca acgttaggtg cgccggtgtt tatctggtta       960
ttgttaaaag caggacgtta g                                                 981
```

<210> 35
<211> 750
<212> DNA
<213> Escherichia coli

<220>
<221> misc_feature
<223> btuD

<400> 35

```
atgtctattg tgatgcagtt acaagatgtt gcggaatcta cccgcctggg gccgctttct       60
ggcgaggttc gggctgggga gatcctgcac ctggtggggc cgaatggcgc gggtaagagt      120
accttactgg cgcgaatggc cggaatgacc agcggtaagg gaagcattca gttcgcgggg      180
caaccactgg aagcatggtc cgcaacaaaa ctcgcgctgc atcgcgccta tctttcacaa      240
cagcagacgc cgccgtttgc aacgccggtc tggcactacc tgacactgca tcagcacgat      300
aaaacgcgta ccgaactact gaatgatgtc gcaggggcgc tggctcttga tgacaaactc      360
ggacgtagca ccaatcaact ttccggcggt gaatggcaac gcgtacgtct tgctgcggtg      420
gtgttgcaaa tcacaccaca agccaatccc gcaggccaat tgctgcttct tgatgagccg      480
atgaacagtc ttgatgttgc gcaacaaagt gcgttagaca aaattctgag cgcgctgtgt      540
cagcaaggac tggcgattgt gatgagcagt cacgatctca accacacatt gcgtcatgcg      600
catcgggcgt ggttgctaaa aggtggaaaa atgctggcca gtggacgcag ggaagaggtg      660

ctcacgccgc caaatctggc gcaggcctat gggatgaatt ttcgccgtct ggatatcgaa      720
ggtcacagaa tgctgatttc gaccatctga                                      750
```

<210> 36
<211> 801
<212> DNA
<213> Escherichia coli

<220>
<221> misc_feature
<223> btuF

<400> 36

```
atggctaagt cactgttcag ggcgctggtc gccctgtctt ttcttgcgcc actgtggctc      60
aacgccgcgc cgcgcgtcat cacgctttct cccgccaaca ctgaacttgc ctttgccgcc     120
gggatcacgc cggttggggt cagcagctat tccgactatc ctccacaagc gcaaaagatt     180
gagcaggttt ccacctggca ggggatgaat ctggaacgca ttgtcgcgct gaaacccgat     240
ctggtgattg cctggcgtgg aggtaatgcc gagcggcagg ttgaccagct ggcttcgctg     300
ggaataaaag tgatgtgggt cgatgcgaca agcattgaac aaattgccaa tgcgttacgt     360
caactggccc cctggagtcc gcaaccagac aaggccgaac aagccgcgca atccctgctg     420
gatcagtacg cgcaattgaa agcgcaatat gctgataaac ctaaaaaacg tgtttttctg     480
caattcggca ttaatccgcc atttaccagt ggaaaagagt cgattcagaa ccaggtactc     540
gaagtttgtg gcggagaaaa catctttaaa gacagccggg ttccctggcc gcaagttagc     600
cgcgaacagg tgttagcacg ctcgccacag gcgattgtca ttacaggcgg accggaccaa     660
attcctaaaa tcaaacaata ctggggtgaa cagctcaaaa ttcccgttat tcctctcacg     720
agtgactggt ttgaacgtgc aagcccacgt attatcctcg ctgcacaaca gctctgtaat     780
gcgctttcac aggtagatta g                                                801
```

<210> 37
<211> 591
<212> DNA
<213> Escherichia coli

<220>
<221> misc_feature
<223> btuR

<400> 37

```
atgagtgatg aacgctacca acagcgtcag cagcgagtga aagaaaaagt agatgctcgt      60
gtggcccagg cccaggatga acgcggtatt atcatcgtct ttaccggcaa tggaaaaggc     120
aaaaccaccg cggcatttgg tacggcaaca cgcgcagttg gtcacggaaa aaaagtaggc     180
gtcgtgcagt ttattaaagg cacctggcct aatggcgaac gcaatctgct ggagccacat     240
ggcgttgagt ttcaggtgat ggcaacgggc tttacctggg atacacaaaa ccgcgagtct     300
gataccgccg cctgccgcga agtctggcaa catgcaaagc ggatgcttgc tgattcctca     360
ctggatatgg ttttgcttga tgaactgacg tatatggtgg cgtatgacta tttgccactg     420
gaagaagtgg tgcaggcgtt aaatgaacgt ccacatcaac agacggtgat tatcacgggt     480
cgtggttgtc atcgggatat tcttgaactg gcagatacgg taagtgaatt acgccccgtc     540
aaacatgcgt ttgatgccgg tgtaaaagcg cagatagga tcgattatta a              591
```

<210> 38
<211> 1824
<212> DNA
<213> Klebsiella pneumoniae

<220>
<221> misc_feature
<223> dhaB4

<400> 38

```
atgccgttaa tagccgggat tgatatcggc aacgccacca ccgaggtggc gctggcgtcc      60
gactacccgc aggcgagggc gtttgttgcc agcgggatcg tcgcgacgac gggcatgaaa     120
gggacgcggg acaatatcgc cgggaccctc gccgcgctgg agcaggccct ggcgaaaaca     180
ccgtggtcga tgagcgatgt ctctcgcatc tatcttaacg aagccgcgcc ggtgattggc     240
gatgtggcga tggagaccat caccgagacc attatcaccg aatcgaccat gatcggtcat     300
aacccgcaga cgccgggcgg ggtgggcgtt ggcgtgggga cgactatcgc cctcgggcgg     360
ctggcgacgc tgccggcggc gcagtatgcc gaggggtgga tcgtactgat tgacgacgcc     420
gtcgatttcc ttgacgccgt gtggtggctc aatgaggcgc tcgaccgggg gatcaacgtg     480
gtggcggcga tcctcaaaaa ggacgacggc gtgctggtga acaaccgcct gcgtaaaacc     540
ctgccggtgg tggatgaagt gacgctgctg gagcaggtcc ccgagggggt aatggcggcg     600
gtggaagtgg ccgcgccggg ccaggtggtg cggatcctgt cgaatcccta cgggatcgcc     660
accttcttcg ggctaagccc ggaagagacc caggccatcg tccccatcgc ccgcgccctg     720
attggcaacc gttccgcggt ggtgctcaag accccgcagg gggatgtgca gtcgcgggtg     780
atcccggcgg gcaacctcta cattagcggc gaaaagcgcc gcggagaggc cgatgtcgcc     840
gagggcgcgg aagccatcat gcaggcgatg agcgcctgcg ctccggtacg cgacatccgc     900
ggcgaaccgg gcacccacgc cggcggcatg cttgagcggg tgcgcaaggt aatggcgtcc     960
ctgaccggcc atgagatgag cgcgatatac atccaggatc tgctggcggt ggatacgttt    1020
attccgcgca aggtgcaggg cgggatggcc ggcgagtgcg ccatggagaa tgccgtcggg    1080
atggcggcga tggtgaaagc ggatcgtctg caaatgcagg ttatcgcccg cgaactgagc    1140
```

```
gcccgactgc agaccgaggt ggtggtgggc ggcgtggagg ccaacatggc catcgccggg     1200
gcgttaacca ctcccggctg tgcggcgccg ctggcgatcc tcgacctcgg cgccggctcg     1260
acggatgcgg cgatcgtcaa cgcggagggg cagataacgg cggtccatct cgccggggcg     1320
gggaatatgg tcagcctgtt gattaaaacc gagctgggcc tcgaggatct ttcgctggcg     1380
gaagcgataa aaaaataccc gctggccaaa gtggaaagcc tgttcagtat tcgtcacgag     1440
aatggcgcgg tggagttctt tcgggaagcc ctcagcccgg cggtgttcgc caaagtggtg     1500
tacatcaagg agggcgaact ggtgccgatc gataacgcca gcccgctgga aaaaattcgt     1560
ctcgtgcgcc ggcaggcgaa agagaaagtg tttgtcacca actgcctgcg cgcgctgcgc     1620
caggtctcac ccggcggttc cattcgcgat atcgcctttg tggtgctggt gggcggctca     1680
tcgctggact ttgagatccc gcagcttatc acggaagcct tgtcgcacta tggcgtggtc     1740
gccgggcagg gcaatattcg gggaacagaa gggccgcgca atgcggtcgc caccgggctg     1800
ctactggccg gtcaggcgaa ttaa                                            1824
```

<210> 39
<211> 354
<212> DNA
<213> Klebsiella pneumoniae

<220>
<221> misc_feature
<223> orf2b

<400> 39

```
atgtcgcttt caccgccagg cgtacgcctg ttttacgatc cgcgcgggca ccatgccggc       60
gccatcaatg agctgtgctg ggggctggag gagcaggggg tcccctgcca gaccataacc      120
tatgacggag gcggtgacgc cgctgcgctg ggcgccctgg cggccagaag ctcgcccctg      180
cgggtgggta tcgggctcag cgcgtccggc gagatagccc tcactcatgc ccagctgccg      240
gcggacgcgc cgctggctac cggacacgtc accgatagcg acgatcaact gcgtacgctc      300
ggcgccaacg ccgggcagct ggttaaagtc ctgccgttaa gtgagagaaa ctga            354
```

<210> 40
<211> 432
<212> DNA
<213> Klebsiella pneumoniae

<220>
<221> misc_feature
<223> orf2a

<400> 40

atgatgaaca agagccaaca agttcagaca atcaccctgg ccgccgccca gcaaatggcg 60

gcggcggtgg aaaaaaaagc cactgagatc aacgtggcgg tggtgttttc cgtagttgac 120

cgcggaggca acacgctgct tatccagcgg atggacgagg ccttcgtctc cagctgcgat 180

atttccctga ataaagcctg gagcgcctgc agcctgaagc aaggtaccca tgaaattacg 240

tcagcggtcc agccaggaca atctctgtac ggtctgcagc taaccaacca acagcgaatt 300

attatttttg gcggcggcct gccagttatt tttaatgagc aggtaattgg cgccgtcggc 360

gttagcggcg gtacggtcga gcaggatcaa ttattagccc agtgcgccct ggattgtttt 420

tccgcattat aa 432

<210> 41
<211> 2932
<212> DNA
<213> Escherichia coli

<220>
<221> misc_feature
<223> pntAB

<400> 41

```
atgcgaattg gcataccaag agaacggtta accaatgaaa cccgtgttgc agcaacgcca      60
aaaacagtgg aacagctgct gaaactgggt tttaccgtcg cggtagagag cggcgcgggt     120
caactggcaa gttttgacga taaagcgttt gtgcaagcgg gcgctgaaat tgtagaaggg     180
aatagcgtct ggcagtcaga gatcattctg aaggtcaatg cgccgttaga tgatgaaatt     240
gcgttactga atcctgggac aacgctggtg agttttatct ggcctgcgca gaatccggaa     300
ttaatgcaaa aacttgcgga acgtaacgtg accgtgatgg cgatggactc tgtgccgcgt     360
atctcacgcg cacaatcgct ggacgcacta agctcgatgg cgaacatcgc cggttatcgc     420
gccattgttg aagcggcaca tgaatttggg cgcttcttta ccgggcaaat tactgcggcc     480
gggaaagtgc caccggcaaa agtgatggtg attggtgcgg gtgttgcagg tctggccgcc     540
attggcgcag caaacagtct cggcgcgatt gtgcgtgcat tcgacacccg cccggaagtg     600
aaagaacaag ttcaaagtat gggcgcggaa ttcctcgagc tggattttaa agaggaagct     660
ggcagcggcg atggctatgc caaagtgatg tcggacgcgt tcatcaaagc ggaaatggaa     720
ctctttgccg cccaggcaaa agaggtcgat atcattgtca ccaccgcgct tattccaggc     780
aaaccagcgc cgaagctaat tacccgtgaa atggttgact ccatgaaggc gggcagtgtg     840
attgtcgacc tggcagccca aaacggcggc aactgtgaat acaccgtgcc gggtgaaatc     900
ttcactacgg aaaatggtgt caaagtgatt ggttataccg atcttccggg ccgtctgccg     960
acgcaatcct cacagcttta cggcacaaac ctcgttaatc tgctgaaact gttgtgcaaa    1020
gagaaagacg gcaatatcac tgttgatttt gatgatgtgg tgattcgcgg cgtgaccgtg    1080
atccgtgcgg gcgaaattac ctggccggca ccgccgattc aggtatcagc tcagccgcag    1140
```

gcggcacaaa aagcggcacc ggaagtgaaa actgaggaaa aatgtacctg ctcaccgtgg 1200
cgtaaatacg cgttgatggc gctggcaatc attctttttg gctggatggc aagcgttgcg 1260
ccgaaagaat tccttgggca cttcaccgtt ttcgcgctgg cctgcgttgt cggttattac 1320
gtggtgtgga atgtatcgca cgcgctgcat acaccgttga tgtcggtcac caacgcgatt 1380
tcagggatta ttgttgtcgg agcactgttg cagattggcc agggcggctg ggttagcttc 1440
cttagtttta tcgcggtgct tatagccagc attaatattt tcggtggctt caccgtgact 1500
cagcgcatgc tgaaaatgtt ccgcaaaaat taaggggtaa catatgtctg gaggattagt 1560
tacagctgca tacattgttg ccgcgatcct gtttatcttc agtctggccg gtctttcgaa 1620
acatgaaacg tctcgccagg gtaacaactt cggtatcgcc gggatggcga ttgcgttaat 1680
cgcaaccatt tttggaccgg atacgggtaa tgttggctgg atcttgctgg cgatggtcat 1740
tggtggggca attggtatcc gtctggcgaa gaaagttgaa atgaccgaaa tgccagaact 1800
ggtggcgatc ctgcatagct tcgtgggtct ggcggcagtg ctggttggct ttaacagcta 1860
tctgcatcat gacgcgggaa tggcaccgat tctggtcaat attcacctga cggaagtgtt 1920
cctcggtatc ttcatcgggg cggtaacgtt cacgggttcg gtggtggcgt tcggcaaact 1980
gtgtggcaag atttcgtcta aaccattgat gctgccaaac cgtcacaaaa tgaacctggc 2040
ggctctggtc gtttccttcc tgctgctgat tgtatttgtt cgcacggaca gcgtcggcct 2100
gcaagtgctg gcattgctga taatgaccgc aattgcgctg gtattcggct ggcatttagt 2160
cgcctccatc ggtggtgcag atatgccagt ggtggtgtcg atgctgaact cgtactccgg 2220
ctgggcggct gcggctgcgg gctttatgct cagcaacgac ctgctgattg tgaccggtgc 2280
gctggtcggt tcttcggggg ctatcctttc ttacattatg tgtaaggcga tgaaccgttc 2340
ctttatcagc gttattgcgg gtggtttcgg caccgacggc tcttctactg gcgatgatca 2400
ggaagtgggt gagcaccgcg aaatcaccgc agaagagaca gcggaactgc tgaaaaactc 2460
ccattcagtg atcattactc cggggtacgg catggcagtc gcgcaggcgc aatatcctgt 2520
cgctgaaatt actgagaaat tgcgcgctcg tggtattaat gtgcgtttcg gtatccaccc 2580
ggtcgcgggg cgtttgcctg gacatatgaa cgtattgctg gctgaagcaa aagtaccgta 2640
tgacatcgtg ctggaaatgg acgagatcaa tgatgacttt gctgataccg ataccgtact 2700
ggtgattggt gctaacgata cggttaaccc ggcggcgcag gatgatccga agagtccgat 2760
tgctggtatg cctgtgctgg aagtgtggaa agcgcagaac gtgattgtct ttaaacgttc 2820
gatgaacact ggctatgctg gtgtgcaaaa cccgctgttc ttcaaggaaa acacccacat 2880
gctgtttggt gacgccaaag ccagcgtgga tgcaatcctg aaagctctgt aa 2932

<210> 42

<211> 1401
<212> DNA
<213> Escherichia coli

<220>
<221> misc_feature
<223> sthA (udhA)

<400> 42

```
atgccacatt cctacgatta cgatgccata gtaataggtt ccggccccgg cggcgaaggc      60
gctgcaatgg gcctggttaa gcaaggtgcg cgcgtcgcag ttatcgagcg ttatcaaaat     120
gttggcggcg gttgcaccca ctggggcacc atcccgtcga aagctctccg tcacgccgtc     180
agccgcatta tagaattcaa tcaaaaccca ctttacagcg accattcccg actgctccgc     240
tcttcttttg ccgatatcct taaccatgcc gataacgtga ttaatcaaca aacgcgcatg     300
cgtcagggat tttacgaacg taatcactgt gaaatattgc agggaaacgc tcgctttgtt     360
gacgagcata cgttggcgct ggattgcccg gacggcagcg ttgaaacact aaccgctgaa     420
aaatttgtta ttgcctgcgg ctctcgtcca tatcatccaa cagatgttga tttcacccat     480
ccacgcattt acgacagcga ctcaattctc agcatgcacc acgaaccgcg ccatgtactt     540
atctatggtg ctggagtgat cggctgtgaa tatgcgtcga tcttccgcgg tatggatgta     600
aaagtggatc tgatcaacac ccgcgatcgc ctgctggcat ttctcgatca agagatgtca     660
gattctctct cctatcactt ctggaacagt ggcgtagtga ttcgtcacaa cgaagagtac     720
gagaagatcg aaggctgtga cgatggtgtg atcatgcatc tgaagtcggg taaaaaactg     780
aaagctgact gcctgctcta tgccaacggt cgcaccggta ataccgattc gctggcgtta     840
cagaacattg ggctagaaac tgacagccgc ggacagctga aggtcaacag catgtatcag     900
accgcacagc cacacgttta cgcggtgggc gacgtgattg gttatccgag cctggcgtcg     960
gcggcctatg accaggggcg cattgccgcg caggcgctgg taaaaggcga agccaccgca    1020
catctgattg aagatatccc taccggtatt tacaccatcc cggaaatcag ctctgtgggc    1080
aaaaccgaac agcagctgac cgcaatgaaa gtgccatatg aagtgggccg cgcccagttt    1140
aaacatctgg cacgcgcaca aatcgtcggc atgaacgtgg gcacgctgaa aattttgttc    1200
catcgggaaa caaaagagat tctgggtatt cactgctttg gcgagcgcgc tgccgaaatt    1260
attcatatcg gtcaggcgat tatggaacag aaaggtggcg gcaacactat tgagtacttc    1320
gtcaacacca cctttaacta cccgacgatg gcggaagcct atcgggtagc tgcgttaaac    1380
ggtttaaacc gcctgtttta a                                              1401
```

<210> 43
<211> 1434

<212> DNA
<213> Streptococcus mutans

<220>
<221> misc_feature
<223> gapN

<400> 43

```
atggcaatga caaaacaata taaaaattat gtcaatggcg agtggaagct ttcagaaaat      60
gaaattaaaa tctacgaacc agccagtgga gctgaattgg gttcagttcc agcaatgagt     120
actgaagaag tagattatgt ttatgcttca gccaagaaag ctcaaccagc ttggcgagca     180
ctttcataca tagaacgtgc tgcctacctt cataaggtag cagatatttt gatgcgtgat     240
aaagaaaaaa taggtgctat tctttccaaa gaggttgcta aaggttataa atcagcagtc     300
agcgaagttg ttcgtactgc agaaatcatt aattatgcag ctgaagaagg tcttcgtatg     360
gaaggtgaag tccttgaagg cggcagtttt gaagcagcca gcaagaaaaa aattgccgtt     420
gttcgtcgtg aaccagtagg tcttgtatta gctatttcac catttaacta ccctgttaac     480
ttggcaggtt cgaaaattgc accggctctt attgcgggaa atgttattgc ttttaaacca     540
ccgacgcaag gatcaatctc agggctctta cttgctgaag catttgctga agctggactt     600
cctgcaggtg tctttaatac cattacaggt cgtggttctg aaattggaga ctatattgta     660
gaacatcaag ccgttaactt tatcaatttc actggttcaa caggaattgg cgaacgtatt     720
ggcaaaatgg ctggtatgcg tccgattatg cttgaactcg gtggaaaaga ttcagccatc     780
gttcttgaag atgcggacct tgaattgact gctaaaaata ttattgcagg tgcttttggt     840
tattcaggtc aacgctgtac agcagttaaa cgtgttcttg tgatggaaag tgttgctgat     900
gaactggtcg aaaaaatccg tgaaaaagtt cttgcattaa caattggtaa tccagaagac     960
gatgcagata ttacaccgtt gattgataca aaatcagctg attatgtaga aggtcttatt    1020
aatgatgcca atgataaagg agccactgcc cttactgaaa tcaaacgtga aggtaatctt    1080
atctgtccaa tcctctttga taaggtaacg acagatatgc gtcttgcttg ggaagaacca    1140
tttggtcctg ttcttccgat cattcgtgtg acatctgtag aagaagccat tgaaatttct    1200
aacaaatcgg aatatggact tcaggcttct atctttacaa atgatttccc acgcgctttt    1260
ggtattgctg agcagcttga agttggtaca gttcatatca ataataagac acagcgcggc    1320
acggacaact tcccattctt aggggctaaa aaatcaggtg caggtattca aggggtaaaa    1380
tattctattg aagctatgac aactgttaaa tccgtcgtat ttgatatcaa ataa          1434
```

<210> 44
<211> 996
<212> DNA

<213> Escherichia coli

<220>
<221> misc_feature
<223> gapA

<400> 44

```
atgactatca aagtaggtat caacggtttt ggccgtatcg gtcgcattgt tttccgtgct      60
gctcagaaac gttctgacat cgagatcgtt gcaatcaacg acctgttaga cgctgattac     120
atggcataca tgctgaaata tgactccact cacggccgtt tcgacggtac cgttgaagtg     180
aaagacggtc atctgatcgt taacggtaaa aaaatccgtg ttaccgctga acgtgatccg     240
gctaacctga aatgggacga agttggtgtt gacgttgtcg ctgaagcaac tggtctgttc     300
ctgactgacg aaactgctcg taaacacatc accgctggtg cgaagaaagt ggttatgact     360
ggtccgtcta aagacaacac tccgatgttc gttaaaggcg ctaacttcga caaatatgct     420
ggccaggaca tcgtttccaa cgcttcctgc accaccaact gcctggctcc gctggctaaa     480
gttatcaacg ataacttcgg catcatcgaa ggtctgatga ccaccgttca cgctactacc     540
gctactcaga aaaccgttga tggcccgtct cacaaagact ggcgcggcgg ccgcggcgct     600
tcccagaaca tcatcccgtc ctctaccggt gctgctaaag ctgtaggtaa agtactgcca     660
gaactgaatg gcaaactgac tggtatggcg ttccgcgttc cgaccccgaa cgtatctgta     720
gttgacctga ccgttcgtct ggaaaaagct gcaacttacg agcagatcaa agctgccgtt     780
aaagctgctg ctgaaggcga aatgaaaggc gttctgggct acaccgaaga tgacgtagta     840
tctaccgatt tcaacggcga agtttgcact tccgtgttcg atgctaaagc tggtatcgct     900
ctgaacgaca acttcgtgaa actggtatcc tggtacgaca acgaaaccgg ttactccaac     960
aaagttctgg acctgatcgc tcacatctcc aaataa                             996
```

<210> 45
<211> 1650
<212> DNA
<213> Escherichia coli

<220>
<221> misc_feature
<223> pgi

<400> 45

```
atgaaaaaca tcaatccaac gcagaccgct gcctggcagg cactacagaa acacttcgat      60
gaaatgaaag acgttacgat cgccgatctt tttgctaaag acggcgatcg tttttctaag     120
ttctccgcaa ccttcgacga tcagatgctg gtggattact ccaaaaaccg catcactgaa     180
gagacgctgg cgaaattaca ggatctggcg aaagagtgcg atctggcggg cgcgattaag     240
tcgatgttct ctggcgagaa gatcaaccgc actgaaaacc gcgccgtgct gcacgtagcg     300
ctgcgtaacc gtagcaatac cccgattttg gttgatggca aagacgtaat gccggaagtc     360
aacgcggtgc tggagaagat gaaaaccttc tcagaagcga ttatttccgg tgagtggaaa     420

ggttataccg gcaaagcaat cactgacgta gtgaacatcg ggatcggcgg ttctgacctc     480
ggcccataca tggtgaccga agctctgcgt ccgtacaaaa accacctgaa catgcacttt     540
gtttctaacg tcgatgggac tcacatcgcg gaagtgctga aaaaagtaaa cccggaaacc     600
acgctgttct tggtagcatc taaaaccttc accactcagg aaactatgac caacgcccat     660
agcgcgcgtg actggttcct gaaagcggca ggtgatgaaa aacacgttgc aaaacacttt     720
gcggcgcttt ccaccaatgc caaagccgtt ggcgagtttg gtattgatac tgccaacatg     780
ttcgagttct gggactgggt tggcggccgt tactctttgt ggtcagcgat tggcctgtcg     840
attgttctct ccatcggctt tgataacttc gttgaactgc tttccggcgc acacgcgatg     900
gacaagcatt tctccaccac gcctgccgag aaaaacctgc ctgtactgct ggcgctgatt     960
ggcatctggt acaacaattt ctttggtgcg gaaactgaag cgattctgcc gtatgaccag    1020
tatatgcacc gtttcgcggc gtacttccag cagggcaata tggagtccaa cggtaagtat    1080
gttgaccgta acggtaacgt tgtggattac cagactggcc cgattatctg gggtgaacca    1140
ggcactaacg gtcagcacgc gttctaccag ctgatccacc agggaaccaa aatggtaccg    1200
tgcgatttca tcgctccggc tatcacccat aacccgctct ctgatcatca ccagaaactg    1260
ctgtctaact tcttcgccca gaccgaagcg ctggcgtttg gtaaatcccg cgaagtggtt    1320
gagcaggaat atcgtgatca gggtaaagat ccggcaacgc ttgactacgt ggtgccgttc    1380
aaagtattcg aaggtaaccg cccgaccaac tccatcctgc tgcgtgaaat cactccgttc    1440
agcctgggtg cgttgattgc gctgtatgag cacaaaatct ttactcaggg cgtgatcctg    1500
aacatcttca ccttcgacca gtggggcgtg gaactgggta aacagctggc gaaccgtatt    1560
ctgccagagc tgaaagatga taaagaaatc agcagccacg atagctcgac caatggtctg    1620
attaaccgct ataaagcgtg gcgcggttaa                                     1650
```

<210> 46
<211> 963
<212> DNA
<213> Escherichia coli

<220>
<221> misc_feature
<223> pfkA

<400> 46

```
atgattaaga aaatcggtgt gttgacaagc ggcggtgatg cgccaggcat gaacgccgca      60
attcgcgggg ttgttcgttc tgcgctgaca gaaggtctgg aagtaatggg tatttatgac     120
ggctatctgg gtctgtatga agaccgtatg gtacagctag accgttacag cgtgtctgac     180
atgatcaacc gtggcggtac gttcctcggt tctgcgcgtt tcccggaatt ccgcgacgag     240
aacatccgcg ccgtggctat cgaaaacctg aaaaaacgtg gtatcgacgc gctggtggtt     300
atcggcggtg acggttccta catgggtgca atgcgtctga ccgaaatggg cttcccgtgc     360
atcggtctgc cgggcactat cgacaacgac atcaaaggca ctgactacac tatcggtttc     420
ttcactgcgc tgagcaccgt tgtagaagcg atcgaccgtc tgcgtgacac ctcttcttct     480
caccagcgta tttccgtggt ggaagtgatg ggccgttatt gtggagatct gacgttggct     540
gcggccattg ccggtggctg tgaattcgtt gtggttccgg aagttgaatt cagccgtgaa     600
gacctggtaa acgaaatcaa agcgggtatc gcgaaaggta aaaaacacgc gatcgtggcg     660
attaccgaac atatgtgtga tgttgacgaa ctggcgcatt tcatcgagaa agaaaccggt     720
cgtgaaaccc gcgcaactgt gctgggccac atccagcgcg gtggttctcc ggtgccttac     780
gaccgtattc tggcttcccg tatgggcgct tacgctatcg atctgctgct ggcaggttac     840
ggcggtcgtt gtgtaggtat ccagaacgaa cagctggttc accacgacat catcgacgct     900
atcgaaaaca tgaagcgtcc gttcaaaggt gactggctgg actgcgcgaa aaaactgtat     960
taa                                                                   963
```

<210> 47
<211> 1476
<212> DNA
<213> Escherichia coli

<220>
<221> misc_feature
<223> zwf

<400> 47

```
atggcggtaa cgcaaacagc ccaggcctgt gacctggtca ttttcggcgc gaaaggcgac      60
cttgcgcgtc gtaaattgct gccttccctg tatcaactgg aaaaagccgg tcagctcaac     120
ccggacaccc ggattatcgg cgtagggcgt gctgactggg ataaagcggc atataccaaa     180
gttgtccgcg aggcgctcga aactttcatg aaagaaacca ttgatgaagg tttatgggac     240
accctgagtg cacgtctgga tttttgtaat ctcgatgtca atgacactgc tgcattcagc     300
cgtctcggcg cgatgctgga tcaaaaaaat cgtatcacca ttaactactt tgccatgccg     360
cccagcactt ttggcgcaat ttgcaaaggg cttggcgagg caaaactgaa tgctaaaccg     420
gcacgcgtag tcatggagaa accgctgggg acgtcgctgg cgacctcgca ggaaatcaat     480
gatcaggttg gcgaatactt cgaggagtgc caggtttacc gtatcgacca ctatcttggt     540
aaagaaacgg tgctgaacct gttggcgctg cgttttgcta actccctgtt tgtgaataac     600
tgggacaatc gcaccattga tcatgttgag attaccgtgg cagaagaagt ggggatcgaa     660
gggcgctggg gctattttga taaagccggt cagatgcgcg acatgatcca gaaccacctg     720
ctgcaaattc tttgcatgat tgcgatgtct ccgccgtctg acctgagcgc agacagcatc     780
cgcgatgaaa aagtgaaagt actgaagtct ctgcgccgca tcgaccgctc caacgtacgc     840
gaaaaaaccg tacgcgggca atatactgcg ggcttcgccc agggcaaaaa agtgccggga     900
tatctggaag aagagggcgc gaacaagagc agcaatacag aaactttcgt ggcgatccgc     960
gtcgacattg ataactggcg ctgggccggt gtgccattct acctgcgtac tggtaaacgt    1020
ctgccgacca aatgttctga agtcgtggtc tatttcaaaa cacctgaact gaatctgttt    1080
aaagaatcgt ggcaggatct gccgcagaat aaactgacta tccgtctgca acctgatgaa    1140
ggcgtggata tccaggtact gaataaagtt cctggccttg accacaaaca taacctgcaa    1200
atcaccaagc tggatctgag ctattcagaa acctttaatc agacgcatct ggcggatgcc    1260
tatgaacgtt tgctgctgga aaccatgcgt ggtattcagg cactgtttgt acgtcgcgac    1320
gaagtggaag aagcctggaa atgggtagac tccattactg aggcgtgggc gatggacaat    1380
gatgcgccga aaccgtatca ggccggaacc tggggacccg ttgcctcggt ggcgatgatt    1440
acccgtgatg gtcgttcctg gaatgagttt gagtaa                              1476
```

<210> 48
<211> 474
<212> PRT
<213> Escherichia coli

<220>
<221> misc_feature
<223> lpd

<400> 48

```
Met Ser Thr Glu Ile Lys Thr Gln Val Val Val Leu Gly Ala Gly Pro
1               5                   10                  15

Ala Gly Tyr Ser Ala Ala Phe Arg Cys Ala Asp Leu Gly Leu Glu Thr
            20                  25                  30

Val Ile Val Glu Arg Tyr Asn Thr Leu Gly Gly Val Cys Leu Asn Val
        35                  40                  45

Gly Cys Ile Pro Ser Lys Ala Leu Leu His Val Ala Lys Val Ile Glu
    50                  55                  60

Glu Ala Lys Ala Leu Ala Glu His Gly Ile Val Phe Gly Glu Pro Lys
65                  70                  75                  80

Thr Asp Ile Asp Lys Ile Arg Thr Trp Lys Glu Lys Val Ile Asn Gln
                85                  90                  95

Leu Thr Gly Gly Leu Ala Gly Met Ala Lys Gly Arg Lys Val Lys Val
```

```
            100                 105                 110

Val Asn Gly Leu Gly Lys Phe Thr Gly Ala Asn Thr Leu Glu Val Glu
        115                 120                 125

Gly Glu Asn Gly Lys Thr Val Ile Asn Phe Asp Asn Ala Ile Ile Ala
    130                 135                 140

Ala Gly Ser Arg Pro Ile Gln Leu Pro Phe Ile Pro His Glu Asp Pro
145                 150                 155                 160

Arg Ile Trp Asp Ser Thr Asp Ala Leu Glu Leu Lys Glu Val Pro Glu
                165                 170                 175

Arg Leu Leu Val Met Gly Gly Gly Ile Ile Gly Leu Glu Met Gly Thr
            180                 185                 190

Val Tyr His Ala Leu Gly Ser Gln Ile Asp Val Val Glu Met Phe Asp
        195                 200                 205

Gln Val Ile Pro Ala Ala Asp Lys Asp Ile Val Lys Val Phe Thr Lys
    210                 215                 220

Arg Ile Ser Lys Lys Phe Asn Leu Met Leu Glu Thr Lys Val Thr Ala
225                 230                 235                 240

Val Glu Ala Lys Glu Asp Gly Ile Tyr Val Thr Met Glu Gly Lys Lys
                245                 250                 255

Ala Pro Ala Glu Pro Gln Arg Tyr Asp Ala Val Leu Val Ala Ile Gly
            260                 265                 270

Arg Val Pro Asn Gly Lys Asn Leu Asp Ala Gly Lys Ala Gly Val Glu
        275                 280                 285

Val Asp Asp Arg Gly Phe Ile Arg Val Asp Lys Gln Leu Arg Thr Asn
    290                 295                 300

Val Pro His Ile Phe Ala Ile Gly Asp Ile Val Gly Gln Pro Met Leu
305                 310                 315                 320

Ala His Lys Gly Val His Glu Gly His Val Ala Ala Glu Val Ile Ala
                325                 330                 335

Gly Lys Lys His Tyr Phe Asp Pro Lys Val Ile Pro Ser Ile Ala Tyr
            340                 345                 350
```

Thr Glu Pro Glu Val Ala Trp Val Gly Leu Thr Glu Lys Glu Ala Lys
355 360 365

Glu Lys Gly Ile Ser Tyr Glu Thr Ala Thr Phe Pro Trp Ala Ala Ser
370 375 380

Gly Arg Ala Ile Ala Ser Asp Cys Ala Asp Gly Met Thr Lys Leu Ile
385 390 395 400

Phe Asp Lys Glu Ser His Arg Val Ile Gly Gly Ala Ile Val Gly Thr
405 410 415

Asn Gly Gly Glu Leu Leu Gly Glu Ile Gly Leu Ala Ile Glu Met Gly
420 425 430

Cys Asp Ala Glu Asp Ile Ala Leu Thr Ile His Ala His Pro Thr Leu
435 440 445

His Glu Ser Val Gly Leu Ala Ala Glu Val Phe Glu Gly Ser Ile Thr
450 455 460

Asp Leu Pro Asn Pro Lys Ala Lys Lys Lys
465 470

<210> 49
<211> 474
<212> PRT
<213> Escherichia coli

<220>
<221> misc_feature
<223> mutated lpd

<400> 49

Met Ser Thr Glu Ile Lys Thr Gln Val Val Val Leu Gly Ala Gly Pro
1 5 10 15

Ala Gly Tyr Ser Ala Ala Phe Arg Cys Ala Asp Leu Gly Leu Glu Thr
20 25 30

Val Ile Val Glu Arg Tyr Asn Thr Leu Gly Gly Val Cys Leu Asn Val
35 40 45

Gly Cys Ile Pro Ser Lys Ala Leu Leu His Val Ala Lys Val Ile Glu
50 55 60

Glu Ala Lys Ala Leu Ala Glu His Gly Ile Val Phe Gly Glu Pro Lys
65 70 75 80

```
Thr Asp Ile Asp Lys Ile Arg Thr Trp Lys Glu Lys Val Ile Asn Gln
                85                  90                  95

Leu Thr Gly Gly Leu Ala Gly Met Ala Lys Gly Arg Lys Val Lys Val
            100                 105                 110

Val Asn Gly Leu Gly Lys Phe Thr Gly Ala Asn Thr Leu Glu Val Glu
        115                 120                 125

Gly Glu Asn Gly Lys Thr Val Ile Asn Phe Asp Asn Ala Ile Ile Ala
    130                 135                 140

Ala Gly Ser Arg Pro Ile Gln Leu Pro Phe Ile Pro His Glu Asp Pro
145                 150                 155                 160

Arg Ile Trp Asp Ser Thr Asp Ala Leu Glu Leu Lys Glu Val Pro Glu
                165                 170                 175

Arg Leu Leu Val Met Gly Gly Gly Ile Ile Ala Leu Glu Met Ala Thr
            180                 185                 190

Val Tyr His Ala Leu Gly Ser Gln Ile Asp Val Val Val Arg Lys His
        195                 200                 205

Gln Val Ile Arg Ala Ala Asp Lys Asp Ile Val Lys Val Phe Thr Lys
    210                 215                 220

Arg Ile Ser Lys Lys Phe Asn Leu Met Leu Glu Thr Lys Val Thr Ala
225                 230                 235                 240

Val Glu Ala Lys Glu Asp Gly Ile Tyr Val Thr Met Glu Gly Lys Lys
                245                 250                 255

Ala Pro Ala Glu Pro Gln Arg Tyr Asp Ala Val Leu Val Ala Ile Gly
            260                 265                 270

Arg Val Pro Asn Gly Lys Asn Leu Asp Ala Gly Lys Ala Gly Val Glu
        275                 280                 285

Val Asp Asp Arg Gly Phe Ile Arg Val Asp Lys Gln Leu Arg Thr Asn
    290                 295                 300

Val Pro His Ile Phe Ala Ile Gly Asp Ile Val Gly Gln Pro Met Leu
305                 310                 315                 320

Ala His Lys Gly Val His Glu Gly His Val Ala Ala Glu Val Ile Ala
                325                 330                 335
```

Gly Lys Lys His Tyr Phe Asp Pro Lys Val Ile Pro Ser Ile Ala Tyr
340 345 350

Thr Glu Pro Glu Val Ala Trp Val Gly Leu Thr Glu Lys Glu Ala Lys
355 360 365

Glu Lys Gly Ile Ser Tyr Glu Thr Ala Thr Phe Pro Trp Ala Ala Ser
370 375 380

Gly Arg Ala Ile Ala Ser Asp Cys Ala Asp Gly Met Thr Lys Leu Ile
385 390 395 400

Phe Asp Lys Glu Ser His Arg Val Ile Gly Gly Ala Ile Val Gly Thr
405 410 415

Asn Gly Gly Glu Leu Leu Gly Glu Ile Gly Leu Ala Ile Glu Met Gly
420 425 430

Cys Asp Ala Glu Asp Ile Ala Leu Thr Ile His Ala His Pro Thr Leu
435 440 445

His Glu Ser Val Gly Leu Ala Ala Glu Val Phe Glu Gly Ser Ile Thr
450 455 460

Asp Leu Pro Asn Pro Lys Ala Lys Lys Lys
465 470

<210> 50
<211> 1548
<212> DNA
<213> Escherichia coli

<220>
<221> misc_feature
<223> yjeF (nrr)

<400> 50

atgacggacc atacaatgaa gaaaaacccc gtaagtatac cacacaccgt ctggtacgcc 60

gacgatatcc gccgcggaga acgcgaggcg gcagatgtgc tggggctcac actctatgag 120

ctgatgcttc gcgctggcga ggccgcattc caggtgtgtc gttcggcgta tcctgacgcc 180

cgccactggc tggtgctgtg cggtcatggt aataacggcg gcgatggcta cgtggtcgcg 240

cgactggcca aagcggtcgg cattgaggtc acgttgttgg cccaggagag cgacaaaccg 300

ttgccggaag aggccgcgct ggcacgcgaa gcatggttaa acgcgggtgg cgagatccat 360

gcttcgaata ttgtctggcc cgaatcggta gatctgattg ttgatgcgct gctcggtacc 420

ggtttgcggc aagcgccccg cgaatccatt agccagttaa tcgaccacgc taattcccat 480

cctgcgccga ttgtggcggt tgatatccct tccggcctgc tggctgaaac tggcgctacg 540

ccaggcgcgg tgatcaacgc cgatcacacc atcactttta ttgcgctgaa accaggcttg 600

ctcactggaa aagcgcggga tgttaccgga caactgcatt ttgactcact ggggctggat 660

agttggctgg caggtcagga gacgaaaatt cagcggtttt cagcagaaca actttctcac 720

tggctaaaac cgcgtcgccc gacttcgcat aaaggcgatc acgggcggct ggtaattatc 780

ggtggcgatc acggcacggc gggggctatt cgtatgacgg gggaagcggc gctgcgtgct 840

ggtgctggtt tagtccgagt actgacccgc agtgaaaaca ttgcgccgct gctgactgca 900

cgaccggaat tgatggtgca tgaactgacg atggactctc ttaccgaaag cctggaatgg 960

gccgatgtgg tggtgattgg tcccggtctg ggccagcaag agtgggggaa aaaagcactg 1020

caaaaagttg agaattttcg caaaccgatg ttgtgggatg ccgatgcatt gaacctgctg 1080

gcaatcaatc ccgataagcg tcacaatcgc gtgatcacgc cgcatcctgg cgaggccgca 1140

cggttgttag gctgttccgt cgctgaaatt gaaagtgacc gcttacattg cgccaaacgt 1200

ctggtacaac gttatggcgg cgtagcggtg ctgaaaggtg ccggaaccgt ggtcgccgcc 1260

catcctgacg ctttaggcat tattgatgcc ggaaatgcag gcatggcgag cggcggcatg 1320

ggcgatgtgc tctctggtat tattggcgca ttgcttgggc aaaaactgtc gccgtatgat 1380

gcagcctgtg caggctgtgt cgcgcacggt gcggcagctg acgtactggc ggcgcgtttt 1440

ggaacgcgcg ggatgctggc aaccgatctc ttttccacgc tacagcgtat tgttaacccg 1500

gaagtgactg ataaaaacca tgatgaatcg agtaattccg ctccctga 1548

<210> 51
<211> 50
<212> DNA
<213> Artificial

<220>
<223> DxylAB 1

<220>
<221> misc_feature
<223> DxylAB 1

<400> 51
acgacatcat ccatcacccg cggcattacc tgattatgga gttcaatatg 50

<210> 52
<211> 50
<212> DNA
<213> Artificial

<220>
<223> DxylAB 2

<400> 52
cccccacccg gtcaggcagg ggataacgtt tacgccatta atggcagaag 50

<210> 53
<211> 975
<212> DNA
<213> Escherichia coli

<220>
<221> misc_feature
<223> yiaE

<400> 53

```
atgaagccgt ccgttatcct ctacaaagcc ttacctgatg atttactgca acgcctgcaa      60
gagcatttca ccgttcacca ggtggcaaac ctcagcccac aaaccgtcga acaaaatgca     120
gcaatttttg ccgaagctga aggtttactg ggttcaaacg agaatgtaaa tgccgcattg     180
ctggaaaaaa tgccgaaact gcgtgccaca tcaacgatct ccgtcggcta tgacaatttt     240
gatgtcgatg cgcttaccgc ccgaaaaatt ctgctgatgc acacgccaac cgtattaaca     300
gaaaccgtcg ccgatacgct gatggcgctg gtgttgtcta ccgctcgtcg ggttgtggag     360
gtagcagaac gggtaaaagc aggcgaatgg accgcgagca taggcccgga ctggtacggc     420
actgacgttc accataaaac actgggcatt gtcgggatgg gacggatcgg catggcgctg     480
gcacaacgtg cgcactttgg cttcaacatg cccatcctct ataacgcgcg ccgccaccat     540
aaagaagcag aagaacgctt caacgcccgc tactgcgatt tggatactct gttacaagag     600
tcagatttcg tttgcctgat cctgccgtta actgatgaga cgcatcatct gtttggcgca     660
gaacaattcg ccaaaatgaa atcctccgcc attttcatta atgccggacg tggcccggtg     720
gttgacgaaa atgcactgat cgcagcattg cagaaaggcg aaattcacgc tgccgggctg     780
gatgtcttcg aacaagagcc actgtccgta gattcgccgt tgctctcaat ggccaacgtc     840
gtcgcagtac cgcatattgg atctgccacc catgagacgc gttatggcat ggccgcctgt     900
gccgtggata atttgattga tgcgttacaa ggaaaggttg agaagaactg tgtgaatccg     960
cacgtcgcgg actaa                                                      975
```

<210> 54
<211> 939
<212> DNA
<213> Escherichia coli

<220>
<221> misc_feature
<223> ycdW

<400> 54

```
atggatatca tcttttatca cccaacgttc gatacccaat ggtggattga ggcactgcgc        60

aaagctattc ctcaggcaag agtcagagca tggaaaagcg gagataatga ctctgctgat       120
tatgctttag tctggcatcc tcctgttgaa atgctggcag ggcgcgatct taaagcggtg       180
ttcgcactcg ggccggtgt tgattctatt ttgagcaagc tacaggcaca ccctgaaatg        240
ctgaaccctt ctgttccact ttttcgcctg gaagataccg gtatgggcga gcaaatgcag       300
gaatatgctg tcagtcaggt gctgcattgg tttcgacgtt ttgacgatta tcgcatccag       360
caaaatagtt cgcattggca accgctgcct gaatatcatc gggaagattt taccatcggc       420
attttgggcg caggcgtact gggcagtaaa gttgctcaga gtctgcaaac ctggcgcttt       480
ccgctgcgtt gctggagtcg aacccgtaaa tcgtggcctg gcgtgcaaag ctttgccgga       540
cgggaagaac tgtctgcatt tctgagccaa tgtcgggtat tgattaattt gttaccgaat       600
acccctgaaa ccgtcggcat tattaatcaa caattactcg aaaaattacc ggatggcgcg       660
tatctcctca acctggcgcg tggtgttcat gttgtggaag atgacctgct cgcggcgctg       720
gatagcggca aagttaaagg cgcaatgttg gatgttttta atcgtgaacc cttaccgcct       780
gaaagtccgc tctggcaaca tccacgcgtg acgataacac cacatgtcgc cgcgattacc       840
cgtcccgctg aagctgtgga gtacatttct cgcaccattg cccagctcga aaaaggggag       900
agggtctgcg ggcaagtcga ccgcgcacgc ggctactaa                              939
```

<210> 55
<211> 50
<212> DNA
<213> Artificial

<220>
<223> DyjhH 1

<400> 55
aatgcgcgaa gttgccgact tcctgattaa taaaggggtc gacgggctgt 50

<210> 56
<211> 50
<212> DNA
<213> Artificial

<220>
<223> DyjhH 2

<400> 56
gtaccgactt aactgtgttg atcatcgtac gcaagtgacc aacgctgtcg 50

<210> 57
<211> 50
<212> DNA
<213> Artificial

<220>
<223> DyagE 1

<400> 57
ggcggcacca acgcccggga aaccatcgaa ctcagccagc acgcgcagca 50

<210> 58
<211> 50
<212> DNA
<213> Artificial

<220>
<223> DyagE 2

<400> 58
agcacggtga agtgcggatg ggcacctttg acggtatgga tcatgctgcg 50

<210> 59
<211> 50
<212> DNA
<213> Artificial

<220>
<223> DyiaE 1

<400> 59
catatttcag gctaaggtga tcgccttatc agtgaatgga gagaagcatg 50

<210> 60
<211> 50
<212> DNA
<213> Artificial

<220>
<223> DyiaE 2

<400> 60
tatcgggctt tactctacgc agtcgcggct tagtccgcga cgtgcggatt 50

<210> 61
<211> 50
<212> DNA
<213> Artificial

<220>
<223> DycdW 1

<400> 61
aacgataagt gcgaataaat ttcgcacaac gcttttcggg agtcagtatg 50

<210> 62
<211> 50
<212> DNA
<213> Artificial

<220>
<223> DycdW 2

<400> 62
ccaaggatag caggaatcct gatgctttat tagtagccgc gtgcgcggtc 50

<210> 63
<211> 100
<212> DNA
<213> Artificial

<220>
<223> DptsG1

<400> 63

atgtttaaga atgcatttgc taacctgcaa aaggtcggta aatcgctgat gctgccggta 60

tccgtactgc ctatcgcagg tgtaggctgg agctgcttcg 100

<210> 64
<211> 100
<212> DNA
<213> Artificial

<220>
<223> DptsG2

<400> 64

ttagtggtta cggatgtact catccatctc ggttttcagg ttatcggatt tagtaccgaa 60

aatcgcctga acaccagaac catatgaata tcctccttag 100

<210> 65
<211> 759
<212> DNA
<213> Escherichia coli

<220>
<221> misc_feature
<223> yciK

<400> 65

```
atgcattacc agccaaaaca agatttactc aatgatcgca ttatcctggt gacgggagcc       60
agcgatggta ttggtcgtga agccgcgatg acgtatgcac gctatggtgc gacagtgatt      120
ctgttgggcc gtaatgaaga aaaattacgt caggtagcca gccacataaa cgaagaaact      180
gggcgtcagc cacagtggtt tattctcgat ttgctgacct gcacgtccga aaattgccaa      240
caactggcac agcgcattgc cgttaattat ccgcgtctgg atggtgtttt gcataatgcc      300
ggattgctcg gcgatgtttg cccaatgagc gaacaaaatc cgcaggtctg gcaggacgtc      360
atgcaggtca acgttaatgc cacctttatg ctcacccagg cactgcttcc tttattactc      420
aaatcggacg ccggttcact ggtctttact tcatcaagcg ttggacgtca gggacgagcc      480
aactggggtg catatgcagc gtcgaaattt gccaccgaag ggatgatgca ggtactggcc      540
gatgaatatc agcagcgcct gcgtgtcaac tgcattaacc caggcggtac gcgcaccgca      600
atgcgtgcca gcgccttccc gaccgaagat ccacagaaac ttaaaacacc cgctgatatc      660
atgccgctct acctctggct gatgggcgat gacagccgcc gtaaaaccgg catgaccttt      720
gacgcccaac cgggccgtaa accaggaatt tcccaatga                              759
```

<210> 66
<211> 43
<212> DNA
<213> Artificial

<220>
<223> Ptrc

<400> 66
gagctgttga caattaatca tccggctcgt ataatgtgtg gaa 43

<210> 67
<211> 50
<212> DNA
<213> Artificial

<220>
<223> Ptrc-yciK-btuR 1

<400> 67
ttctgtttca ggcataaagc cccaaagtca taaagtacac tggcagcgcg 50

<210> 68
<211> 50
<212> DNA
<213> Artificial

<220>
<223> Ptrc-yciK-btuR 2

<400> 68
atcttgtttt ggctggtaat gcatggctac tcctcaacga cgttgtctgt 50

<210> 69
<211> 21
<212> DNA
<213> Artificial

<220>
<223> RBS120

<400> 69
atccggtata ggaggtatag a 21

<210> 70
<211> 100
<212> DNA
<213> Artificial

<220>
<223> DudhA1

<400> 70

ggtgcgcgcg tcgcagttat cgagcgttat caaaatgttg gcggcggttg cacccactgg 60

ggcaccatcc cgtcgaaagc catatgaata tcctccttag 100

<210> 71
<211> 100
<212> DNA
<213> Artificial

<220>
<223> DudhA2

<400> 71

cccagaatct cttttgtttc ccgatggaac aaaattttca gcgtgcccac gttcatgccg 60

acgatttgtg cgcgtgccag tgtaggctgg agctgcttcg 100

<210> 72
<211> 101
<212> DNA
<213> Artificial

<220>
<223> DpfkA1

<400> 72

gttcctcggt tctgcgcgtt tcccggaatt ccgcgacgag aacatccgcg ccgtggctat 60

cgaaaacctg aaaaaacgtg gtgtaggctg gagctgcttc g 101

<210> 73

<211> 102
<212> DNA
<213> Artificial

<220>
<223> DpfkA2

<400> 73

```
ggcctgataa gcgaagcgca tcaggcattt ttgcttctgt catcggtttc agggtaaagg       60

aatctgcctt tttccgaaat cacatatgaa tatcctcctt ag                         102
```

<210> 74
<211> 83
<212> DNA
<213> Phage lambda

<220>
<221> misc_feature
<223> PR01

<400> 74

```
acgttaaatc tatcaccgca agggataaat atctaacacc gtgcgtgttg acaattttac       60

ctctggcggt gataatggtt gca                                               83
```

<210> 75
<211> 714
<212> DNA
<213> Phage lambda

<220>
<221> misc_feature
<223> CI857

<400> 75

```
atgagcacaa aaaagaaacc attaacacaa gagcagcttg aggacgcacg tcgccttaaa        60
gcaatttatg aaaaaaagaa aaatgaactt ggcttatccc aggaatctgt cgcagacaag       120
atggggatgg ggcagtcagg cgttggtgct ttatttaatg gcatcaatgc attaaatgct       180
tataacgccg cattgcttac aaaaattctc aaagttagcg ttgaagaatt tagcccttca       240
atcgccagag aaatctacga gatgtatgaa gcggttagta tgcagccgtc acttagaagt       300
gagtatgagt accctgtttt ttctcatgtt caggcaggga tgttctcacc taagcttaga       360
acctttacca aaggtgatgc ggagagatgg gtaagcacaa ccaaaaaagc cagtgattct       420
gcattctggc ttgaggttga aggtaattcc atgaccgcac caacaggctc caagccaagc       480
tttcctgacg gaatgttaat tctcgttgac cctgagcagg ctgttgagcc aggtgatttc       540
tgcatagcca gacttggggg tgatgagttt accttcaaga aactgatcag ggatagcggt       600
caggtgtttt tacaaccact aaacccacag tacccaatga tcccatgcaa tgagagttgt       660
tccgttgtgg ggaaagttat cgctagtcag tggcctgaag agacgtttgg ctga             714
```

<210> 76
<211> 34
<212> DNA
<213> Artificial

<220>
<223> RBS150

<400> 76
taccaactaa cgcacgttta agtaggaacc gtat 34

<210> 77
<211> 50
<212> DNA
<213> Artificial

<220>
<223> DgapA::RN/TTadcca-CI857-PR01/RBS150-gapN::K7 1

<400> 77
acgtgactga ttctaacaaa acattaacac caactggcaa aattttgtcc 50

<210> 78
<211> 50
<212> DNA
<213> Artificial

<220>
<223> DgapA::RN/TTadcca-CI857-PR01/RBS150-gapN::K7 2

<400> 78
aaaaaagagc gaccgaagtc gctcttttta gatcacagtg tcatctcaac 50

<210> 79
<211> 50

<212> DNA
<213> Artificial

<220>
<223> Ptrc-xylFGH::K7 1

<400> 79
ctaaaaattg gttacgttta tcgcggtgat tgttacttat taaaactgtc 50

<210> 80
<211> 50
<212> DNA
<213> Artificial

<220>
<223> Ptrc-xylFGH::K7 2

<400> 80
tgagtagaat gttctttatt ttcatggtgt agggccttct gtagttagag 50